# EUROPEAN PATENT APPLICATION

(11) **EP 1 712 631 A2**
(43) Date of publication of application: **18.10.2006**
(21) Application number: 06010773.7
(22) Date of filing: 20.01.1998
(51) Int. Cl.: C12N 15/57, C12N 9/64, C12N 1/21, C12N 1/19, C12N 5/10

(54) **Fc receptors and polypeptides**

(30) Priority: 21.01.1997 US 34205 P; 18.06.1997 US 49872 P
(62) Divisional of application: 98902673.7
(71) Applicant: HUMAN GENOME SCIENCES, INC., Rockville, MD 20850 (US)
(72) Inventor: Olsen, Henrik, Gaithersburg, MD 20878 (US); Ni, Jian, Germantown, MD 20874 (US); Murphy, Marianne, London NW8 9QX (GB); Ruben, Steven, Brookeville, MD 20833 (US); Gentz, Reiner, 82131 Gauting (DE)
(74) Representative: Vossius & Partner

(57) **Abstract**

The present invention relates to five novel Fc receptor-like proteins which are members of the Fc Receptor family. In particular, isolated nucleic acid molecules are provided encoding the human FcR-I, FcR-II, FcR-III, FcR-IV and FcR-V proteins. FcR-I, FcR-II, FcR-III, FcR-IV and FcR-V polypeptides are also provided as are vectors, host cells and recombinant methods for producing the same. The invention further relates to screening methods for identifying agonists and antagonists of FcR-I, FcR-II, FcR-III, FcR-IV and FcR-V activities. Also provided are diagnostic methods for detecting immune system-related disorders and therapeutic methods for treating immune system-related disorders.

## Description

### Field of the Invention

The present invention relates to several novel human genes encoding polypeptides which are members of the Fc Receptor family. More specifically, isolated nucleic acid molecules are provided encoding human polypeptides named Fc Receptor-like I, Fc Receptor-like II, Fc Receptor-like III, Fc Receptor-like IV, and Fc Receptor-like V, hereinafter referred to as FcR-I, FcR-II, FcR-III, FcR-IV, and FcR-V respectively. FcR-I, FcR-II, FcR-III, FcR-IV, and FcR-V polypeptides are also provided, as are vectors, host cells and recombinant methods for producing the same. Also provided are diagnostic methods for detecting disorders related to the immune and hematopoietic systems, and therapeutic methods for treating such disorders.

The invention further relates to screening methods for identifying agonists and antagonists of FcR-I, FcR-II, FcR-III. FcR-IV, and FcR-V activity.

### Background of the Invention

Fc receptors (FcR) are a major group of cell membrane glycoproteins involved in homeostasis of the immune system. Specific receptors for all immunoglobulin (Ig) classes have been defined and are found on a wide variety of immune cells including B-cells and some T-cells as well as myeloid cells and other non-haemopoietic cells (Raghavan, M. and Bjorkman, P. J. (1996) Annu. Rev. Cell Dev. Biol. 12: 181-220; Dickler, (1976) Adv. Immun., 24:167-215; U. S. Patent No. 5,451.669). The principle role of these receptors is to bind Ig molecules via the Fc region of the Ig molecule. It is through this interaction that a wide range of biological effects are initiated including phagocytosis of immune complexes by macrophages and neutrophils (Capron, M., et al., (1984) J. Immunol., 132:462-468) and direct or indirect regulation of antibody production by membrane-bound or soluble Fc receptor (Fridman, W. H., et al, (1981) Immunol. Rev., 56:51-58; U. S. Patent 5,451,669).

There are at least six Fc receptors thus far identified: the high affinity FcγRI which recognizes monomeric IgG, two low affinity Fc receptors which recognize immune complexes of IgG (FcγRII, FcγRIII), FcαR which recognizes secretory IgA, and a high (FcεRI) and low (FcεRII, CD23) affinity receptor for IgE. In addition, the expression of these receptors, while confined to particular effector cell populations during the resting stage, may be induced by T-cell-derived cytokines such as interferon-γ to be expressed on multiple cell types during an active immune response (Ravetch, J. V. and Kinet, J. P., (1991) Annu. Rev. Immunol. 9:457-492).

FcR are heterodimeric cell surface molecules of α and β chains. Both chains consist of an extracellular region containing repeated Ig-like domains, a transmembrane region, and varingly sized cytoplasmic domains. In addition, at least three FcR (FeγRI, III and FceRI) associate with an intracellular γ chain subunit which is necessary for assembly and signaling through the FcR (Ravetch, J. V. and Kinet, J. P., (1991) Annu. Rev. Immunol. 9:457-492).

One broad disease area in which Fc receptor function has been implicated is immune-complex related inflammatory diseases such as rheumatoid arthritis, systemic lupus erythromatosis, autoimmune hemolytic anemia, thrombocytopenia and IgG- or IgE-mediated inflammation or anaphlylaxis (allergy). This important role is evidenced most clearly by mice rendered deficient in the common FcR-γ chain by gene targeting. These mice were found to be more resistant to disease in experimental models of autoimmune hemolytic anemia, thrombocytopenia and the cutaneous Arthus reaction (Clynes, R. and Ravetch, J. V., (1995) Immunity 3:21-26; Sylvestre, D. L. and Ravetch, J. V., (1994) Science 265:1095-1098; and Sylvestre, D. L. and Ravetch, J. V., (1996) Immunity 5:387-390).

The main functional role of the FcRs,is thought to be a mechanism to elicit an activation signal for effector cells. This signal can be measured experimentally by examining the phosphorylation of the common γ chain or downstream signaling molecules (Salcedo, T. W., et al., (1993) J. Exp. Med 177:1475-1480), or by the antibody-dependent cell-mediated cytotoxicity (ADCC) assay in which effector cells recognize and are induced to kill the P815 tumor target cell which is coated with IgG (Trinchieri, G., et al., (1984) J. Immunol. 133:1869-1877). In addition to the activating FcR, non-specific effector cells, such as NK, have recently been shown to possess receptors which inhibit their activation. These receptors are part of a growing family of cell surface molecules referred to as killer cell inhibitory receptors (KIR; Lanier, L. L., et al.,(1997) lmmunol. Rev. 155:145-154.; Selvakuman, A., et al., (1997) Immunol. Rev. 155:183-196; Renard, V., et al., (1997) Immunol. Rev. 155:205-221). These receptors do not recognize Fc regions of Ig, but rather, polymorphic molecules which are present on almost all cell types in the body and are encoded by the class I major histocompatibility complex (Class I MHC). In contrast to FcR. most KIR deliver suppressive signals to the effector cells, controlling their activation and preventing autoimmune reactivity.

Like FcR KIR are also members of the Ig superfamily and possess one or more Ig-like domains in their extracellular region. The structures and functions of KIRs, and related molecules, has only recently been described (Lanier, L. L., et al., (1997) Immunol. Rev. 155:145-154.; Selvakuman, A., et al., (1997) Immunol. Rev. 155:183-196; Renard, V., et al., (1997) Immunol. Rev. 155:205-221). However, more than thirty such molecules have already been identified. Studies with these molecules have made it clear that they are a multigene family and are likely to play an important role in modulating the activities of inflammatory effector cells. The fact that they share structural motifs in common with FcR, including some signaling motifs within the cytoplasmic domains (Renard, V., et al., (1997) Immunol. Rev. 155:205-221), suggest that the two receptor types act in concert to regulate the functions of a wide variety of effector cell types. However, unlike FcRs, KIRs generally function in an inhibitory capacity, effectively slowing or even stopping NK cell activity. As a result, the use of specific KIR antagonists, such as antibodies or soluble receptors, may be useful to alleviate their suppressive activity and allow macrophages. NK cells, and other effector cells to recognize and destroy infectious agents or malignant cells.

Thus, there is a need for polypeptides that function as regulators of the immune and hematopoietic systems, since disturbances of such regulation may be involved in disorders relating to inflammation, hemostasis, arthritis, immunodeficiency, and other immune and hematopoietic system anomalies. Therefore, there is a need for identification and characterization of such human polypeptides which can play a role in detecting, preventing, ameliorating or correcting such disorders.

### Summary of the Invention

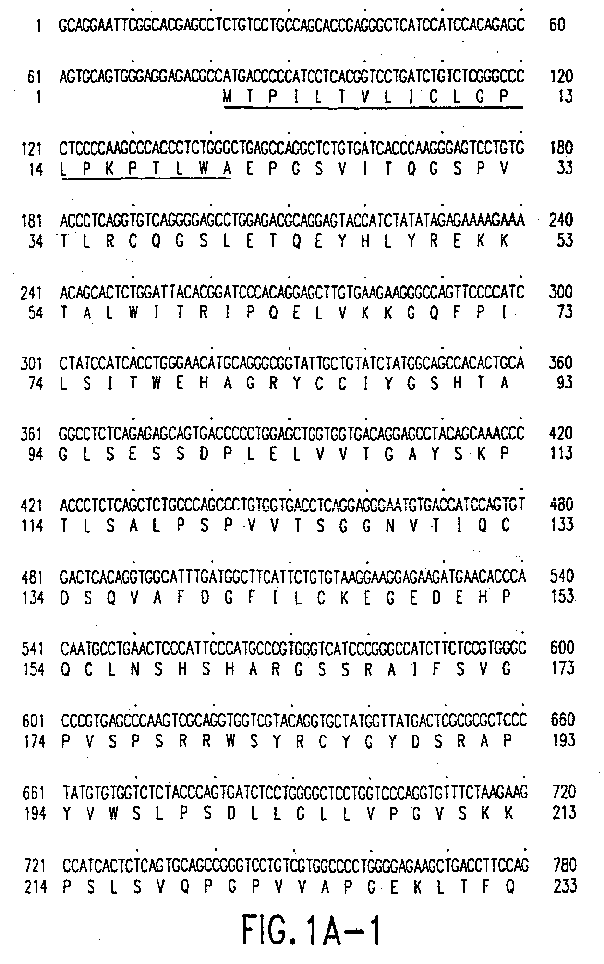

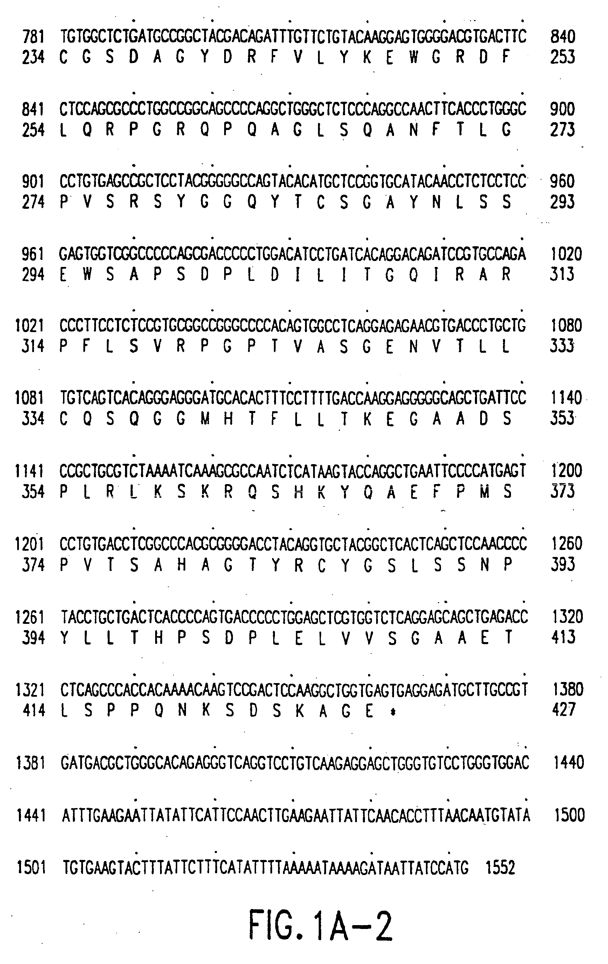

The present invention provides isolated nucleic acid molecules comprising polynucleotides encoding at least a portion of each of the five FcR polypeptides having the complete amino acid sequences shown in SEQ ID NO:2, SEQ ID NO:4, SEQ ID NO:6, or SEQ ID NO:8 or the complete amino acid sequences encoded by the cDNA clones deposited as pooled plasmid DNA in ATCC Deposit Number 97891 on February 21, 1997. The present invention also provides an isolated nucleic acid molecule comprising a polynucleotide encoding at least a portion of the FcR polypeptide having the complete amino acid sequence shown in SEQ ID NO:10 or the complete amino acid sequence encoded by the cDNA clone deposited as plasmid DNA in ATCC Deposit Number 209100 on June 6, 1997 The nucleotide sequences determined by sequencing the deposited FcR-I. FcR-II, FcR-III, FcR-IV. and FcR-V clones, which are shown in Figures 1 A (SEQ ID NO: 1), 2A (SEQ ID NO:3), 3A (SEQ ID NO:5), 4A (SEQ ID NO:7), and 5A (SEQ ID NO:9), contain open reading frames encoding complete polypeptides of 427, 263, 623, 472, and 514 amino acid residues, respectively, including initiation codons encoding an N-terminal methionine at nucleotide positions 82-84, 37-39, 73-75, 22-24, and 46-48, and predicted molecular weights of about 46.2,28.8, 68.5, 51.9, and 56.3 kDa. Nucleic acid molecules of the invention include those encoding the complete amino acid sequence excepting the N-terminal methionine shown in SEQ ID NO:2, SEQ ID NO:4, SEQ ID NO:6. SEQ ID NO:8, and SEQ ID NO:10, or the complete amino acid sequence excepting the N-terminal methionine encoded by the cDNA clones in pooled ATCC Deposit Number 97891. or by the cDNA clone in ATCC Deposit Number 209100, which molecules also can encode additional amino acids fused to the N-terminus of the FcR-I, FcR-II, FcR-III, FcR-IV, and FcR-V amino acid sequences, including an N-terminal methionine.

The FcR-I, FcR-II, FcR-III, FcR-IV, and FcR-V proteins of the present invention share sequence homology with many FcRs and KIRs, but especially with the translation product of the bovine mRNA for Fc-γ2 Receptor (SEQ ID NO:11), including the following conserved domains: (a) the predicted extracellular domains which consist of two or more IgG-like domain repeats of about 90 amino acids; (b) the predicted transmembrane domains of about 21 amino acids, and (c) the intracytoplasmic domains of about 15 to 150 amino acids. The Fc-γ2 receptor is thought to be important in regulation of the immune and hematopoietic systems. The homology between the Fc-γ2 receptor and the novel FcR-I, FcR-II, FcR-III, FcR-IV, and FcR-V molecules indicates that FcR-I, FcR-II, FcR-III, FcR-IV, and FcR-V may also be involved in regulation of the immune and hematopoietic systems.

Each of the encoded polypeptides. FcR-I, FcR-II, FcR-III, FcR-IV, and FcR-V, appear to have a predicted leader sequence of 21, 18, 16, 16, and 16 amino acids, respectively. The amino acid sequence of the predicted mature FcR-I, FcR-II, FcR-III, FcR-IV, and FcR-V proteins are shown in Figures 1A, 2A, 3A, 4A, and 5A as amino acid residues 22-427, 19-263, 17-623, 17-472, and 17-514 respectively, and as residues 1-406, 1-245, 1-607, 1-456, and 1-498 in SEQ ID NO:2, SEQ ID NO:4, SEQ ID NO:6, SEQ ID NO:8, and SEQ ID NO:10, respectively.

Thus, one aspect of the invention provides an isolated nucleic acid molecule comprising a polynucleotide having a nucleotide sequence at least 95% identical to a sequence selected from the group consisting of: (a) a nucleotide sequence encoding the FcR-I polypeptide having the amino acid sequence at positions -21 to 406 of SEQ ID NO:2 or the complete amino acid sequence encoded by the FcR-I cDNA clone contained in ATCC Deposit No. 97891; (b) a nucleotide sequence encoding the FcR-II polypeptide having the amino acid sequence at positions -18 to 245 of SEQ ID NO:4 or the complete amino acid sequence encoded by the FcR-II cDNA clone contained in ATCC Deposit No. 97891; (c) a nucleotide sequence encoding the FcR-III polypeptide having the amino acid sequence at positions -16 to 607 of SEQ ID NO:6 or the complete amino acid sequence encoded by the FcR-III cDNA clone contained in ATCC Deposit No. 97891; (d) a nucleotide sequence encoding the FcR-IV polypeptide having the amino acid sequence at positions -16 to 456 of SEQ ID NO:8 or the complete amino acid sequence encoded by the FcR-IV cDNA clone contained in ATCC Deposit No. 97891; (e) a nucleotide sequence encoding the FcR-V polypeptide having the amino acid sequence at positions -16 to 498 of SEQ ID NO:10 or the complete amino acid sequence encoded by the FcR-V cDNA clone contained in ATCC Deposit No. 209100; (f) a nucleotide sequence encoding the FcR-I polypeptide having the amino acid sequence at positions -20 to 406 of SEQ ID NO:2 or the complete amino acid sequence excepting the N-terminal methionine encoded by the FcR-I cDNA clone contained in ATCC Deposit No. 97891; (g) a nucleotide sequence encoding the FcR-II polypeptide having the amino acid sequence at positions -17 to 245 of SEQ ID NO:4 or the complete amino acid sequence excepting the N-terminal methionine encoded by the FcR-II cDNA clone contained in ATCC Deposit No. 97891; (h) a nucleotide sequence encoding the FcR-III polypeptide having the amino acid sequence at positions -15 to 607 of SEQ ID NO:6 or the complete amino acid sequence excepting the N-terminal methionine encoded by the FcR-III cDNA clone contained in ATCC Deposit No. 97891; (i) a nucleotide sequence encoding the FcR-IV polypeptide having the amino acid sequence at positions -15 to 456 of SEQ ID NO:8 or the complete amino acid sequence excepting the N-terminal methionine encoded by the FcR-IV cDNA clone contained in ATCC Deposit No. 97891; (j) a nucleotide sequence encoding the FcR-V polypeptide having the amino acid sequence at positions -15 to 498 of SEQ ID NO:10 or the complete amino acid sequence excepting the N-terminal methionine encoded by the FcR-V cDNA clone contained in ATCC Deposit No. 209100; (k) a nucleotide sequence encoding the mature form of the FcR-I polypeptide having the amino acid sequence at positions 1 to 406 in SEQ ID NO:2, or as encoded by the FcR-I cDNA clone contained in ATCC Deposit No. 97891; (l) a nucleotide sequence encoding the mature form of the FcR-II polypeptide having the amino acid sequence at positions 1 to 245 in SEQ ID NO:4, or as encoded by the FcR-II cDNA clone contained in ATCC Deposit No. 97891; (m) a nucleotide sequence encoding the mature form of the FcR-III polypeptide having the amino acid sequence at positions 1 to 607 in SEQ ID NO:6, or as encoded by the FcR-III cDNA clone contained in ATCC Deposit No. 97891; (n) a nucleotide sequence encoding the mature form of the FcR-IV polypeptide having the amino acid sequence at positions 1 to 456 in SEQ ID NO:8, or as encoded by the FcR-IV cDNA clone contained in ATCC Deposit No. 97891; (o) a nucleotide sequence encoding the mature form of the FcR-V polypeptide having the amino acid sequence at positions 1 to 498 in SEQ ID NO:10, or as encoded by the FcR-V cDNA clone contained in ATCC Deposit No. 209100; (p) a nucleotide sequence encoding a polypeptide comprising the extracellular domain of the FcR-I polypeptide having the amino acid sequence at positions 1 to 289 in SEQ ID NO:2 or as encoded by the FcR-I cDNA clone contained in ATCC Deposit No. 97891; (q) a nucleotide sequence encoding a polypeptide comprising the extracellular domain of the FcR-II polypeptide having the amino acid sequence at positions 1 to 211 in SEQ ID NO:4 or as encoded by the FcR-II cDNA clone contained in ATCC Deposit No. 97891; (r) a nucleotide sequence encoding a polypeptide comprising the extracellular domain of the FcR-III polypeptide having the amino acid sequence at positions I to 421 in SEQ ID NO:6 or as encoded by the FcR-III cDNA clone contained in ATCC Deposit No. 97891; (s) a nucleotide sequence encoding a polypeptide comprising the extracellular domain of the FcR-IV polypeptide having the amino acid sequence at positions 1 to 243 in SEQ ID NO: 8 or as encoded by the FcR-IV cDNA clone contained in ATCC Deposit No. 97891; (t) a nucleotide sequence encoding a polypeptide comprising the extracellular domain of the FcR-V polypeptide having the amino acid sequence at positions 1 to 343 in SEQ ID NO: 10 or as encoded by the FcR-V cDNA clone contained in ATCC Deposit No. 209100; (u) a nucleotide sequence encoding a polypeptide comprising the transmembrane domain of the FcR-I polypeptide having the amino acid sequence at positions 290 to 312 in SEQ ID NO:2 or as encoded by the FcR-I cDNA clone contained in ATCC Deposit No. 97891; (v) a nucleotide sequence encoding a polypeptide comprising the transmembrane domain of the FcR-II polypeptide having the amino acid sequence at positions 212 to 229 in SEQ ID NO:4 or as encoded by the FcR-II cDNA clone contained in ATCC Deposit No. 97891; (w) a nucleotide sequence encoding a polypeptide comprising the transmembrane domain of the FcR-III polypeptide having the amino acid sequence at positions 422 to 448 in SEQ ID NO:6 or as encoded by the FcR-III cDNA clone contained in ATCC Deposit No. 97891; (x) a nucleotide sequence encoding a polypeptide comprising the transmembrane domain of the FcR-IV polypeptide having the amino acid sequence at positions 244 to 264 in SEQ ID NO:8 or as encoded by the FcR-IV cDNA clone contained in ATCC Deposit No. 97891; (y) a nucleotide sequence encoding a polypeptide comprising the transmembrane domain of the FcR-V polypeptide having the amino acid sequence at positions 344 to 364 in SEQ ID NO:10 or as encoded by the FcR-V cDNA clone contained in ATCC Deposit No. 209100; (z) a nucleotide sequence encoding a polypeptide comprising the intracellular domain of the FcR-I polypeptide having the amino acid sequence at positions 313 to 406 in SEQ ID NO:2 or as encoded by the FcR-I cDNA clone contained in ATCC Deposit No. 97891; (aa) a nucleotide sequence encoding a polypeptide comprising the intracellular domain of the FcR-II polypeptide having the amino acid sequence at positions 230 to 245 in SEQ ID NO:4 or as encoded by the FcR-II cDNA clone contained in ATCC Deposit No. 97891; (ab) a nucleotide sequence encoding a polypeptide comprising the intracellular domain of the FcR-III polypeptide having the amino acid sequence at positions 449 to 607 in SEQ ID NO:6 or as encoded by the FcR-III cDNA clone contained in ATCC Deposit No. 97891; (ac) a nucleotide sequence encoding a polypeptide comprising the intracellular domain of the FcR-IV polypeptide having the amino acid sequence at positions 265 to 456 in SEQ ID NO:8 or as encoded by the FcR-IV cDNA clone contained in ATCC Deposit No. 97891; (ad) a nucleotide sequence encoding a polypeptide comprising the intracellular domain of the FcR-V polypeptide having the amino acid sequence at positions 365 to 498 in SEQ ID NO:10 or as encoded by the FcR-V cDNA clone contained in ATCC Deposit No. 97891; (ae) a nucleotide sequence encoding a soluble FcR-I polypeptide having the extracellular and intracellular domains but lacking the transmembrane domain; (af) a nucleotide sequence encoding a soluble FcR-II polypeptide having the extracellular and intracellular domains but lacking the transmembrane domain: (ag) a nucleotide sequence encoding a soluble FcR-III polypeptide having the extracellular and intracellular domains but lacking the transmembrane domain; (ah) a nucleotide sequence encoding a soluble FcR-IV polypeptide having the extracellular and intracellular domains but lacking the transmembrane domain; (ai) a nucleotide sequence encoding a soluble FcR-V polypeptide having the extracellular and intracellular domains but lacking the transmembrane domain; and; (aj) a nucleotide sequence complementary to any of the nucleotide sequences in (a) through (ai) above.

Further embodiments of the invention include isolated nucleic acid molecules that comprise a polynucleotide having a nucleotide sequence at least 90% identical, and more preferably at least 95%, 96%, 97%, 98% or 99% identical, to any of the nucleotide sequences in (a) through (aj) above, or a polynucleotide which hybridizes under stringent hybridization conditions to a polynucleotide in (a) through (aj) above. This polynucleotide which hybridizes does not hybridize under stringent hybridization conditions to a polynucleotide having a nucleotide sequence consisting of only A residues or of only T residues. An additional nucleic acid embodiment of the invention relates to an isolated nucleic acid molecule comprising a polynucleotide which encodes the amino acid sequence of an epitope-bearing portion of the FcR-I, FcR-II, FcR-III, FcR-IV, and FcR-V polypeptides having an amino acid sequence in (a) through (ai) above.

The present invention also relates to recombinant vectors, which include the isolated nucleic acid molecules of the present invention, and to host cells containing the recombinant vectors, as well as to methods of making such vectors and host cells and for using them for production of FcR-I, FcR-II, FcR-III, FcR-IV, and FcR-V polypeptides or peptides by recombinant techniques.

The invention further provides isolated FcR-1, FcR-II, FcR-III, FcR-IV, and FcR-V polypeptides comprising an amino acid sequences selected from the group consisting of: (a) the amino acid sequence of the complete FcR-I polypeptide having the amino acid sequence at positions -21 to 406 of SEQ ID NO:2 or the complete FcR-I amino acid sequence encoded by the FcR-I cDNA clone contained in ATCC Deposit No. 97891; (b) the amino acid sequence of the complete FcR-I polypeptide having the amino acid sequence at positions -20 to 406 of SEQ ID NO:2 or the complete FcR-I amino acid sequence excepting the N-terminal methionine encoded by the FcR-I cDNA clone contained in ATCC Deposit No. 97891; (c) the amino acid sequence of the mature FcR-I polypeptide having the amino acid sequence at positions 1 to 406 in SEQ ID NO:2, or as encoded by the FcR-I cDNA clone contained in ATCC Deposit No. 97891; (d) the amino acid sequence of the extracellular domain of the FcR-I polypeptide having the amino acid sequence at positions 1-289 in SEQ ID NO:2, or as encoded by the FcR-I cDNA clone contained in ATCC Deposit No. 97891; (e) the amino acid sequence of the transmembrane domain of the FcR-I polypeptide having the amino acid sequence at positions 290-312 in SEQ ID NO:2, or as encoded by the FcR-I cDNA clone contained in ATCC Deposit No. 97891; (f) the amino acid sequence of the intracellular domain of the FcR-I polypeptide having the amino acid sequence at positions 313-406 in SEQ ID NO:2, or as encoded by the FcR-I cDNA clone contained in ATCC Deposit No. 97891; (g) the amino acid sequence of a soluble FcR-I polypeptide comprising the extracellular and intracelluar domains, but lacking the transmembrane domain; (h) the amino acid sequence of the complete FcR-II polypeptide having the amino acid sequence at positions -18 to 245 of SEQ ID NO:4 or the complete FcR-II amino acid sequence encoded by the FcR-II cDNA clone contained in ATCC Deposit No. 97891; (i) the amino acid sequence of the complete FcR-II polypeptide having the amino acid sequence at positions -17 to 245 of SEQ ID NO:4 or the complete FcR-II amino acid sequence excepting the N-terminal methionine encoded by the FcR-11 cDNA clone contained in ATCC Deposit No. 97891; (j) the amino acid sequence of the mature FcR-II polypeptide having the amino acid sequence at positions 1 to 245 in SEQ ID NO:4, or as encoded by the FcR-II cDNA clone contained in ATCC Deposit No. 97891; (k) the amino acid sequence of the extracellular domain of the FcR-II polypeptide having the amino acid sequence at positions 1-211 in SEQ ID NO:4, or as encoded by the FcR-II cDNA clone contained in ATCC Deposit No. 97891; (1) the amino acid sequence of the transmembrane domain of the FcR-II polypeptide having the amino acid sequence at positions 212-229 in SEQ ID NO:4, or as encoded by the FcR-II cDNA clone contained in ATCC Deposit No. 97891; (m) the amino acid sequence of the intracellular domain of the FcR-II polypeptide having the amino acid sequence at positions 230-245 in SEQ ID NO:4, or as encoded by the FcR-II cDNA clone contained in ATCC Deposit No. 97891; (n) the amino acid sequence of a soluble FcR-II polypeptide comprising the extracellular and intracelluar domains, but lacking the transmembrane domain; (o) the amino acid sequence of the complete FcR-111 polypeptide having the amino acid sequence at positions -16 to 607 of SEQ ID NO:6 or the complete FcR-III amino acid sequence encoded by the FcR-III cDNA clone contained in ATCC Deposit No. 97891; (p) the amino acid sequence of the complete FcR-III polypeptide having the amino acid sequence at positions -15 to 607 of SEQ ID NO:6 or the complete FcR-111 amino acid sequence excepting the N-terminal methionine encoded by the FcR-III cDNA clone contained in ATCC Deposit No. 97891; (q) the amino acid sequence of the mature FcR-111 polypeptide having the amino acid sequence at positions 1 to 607 in SEQ ID NO:6. or as encoded by the FcR-111 cDNA clone contained in ATCC Deposit No. 9789i; (r) the amino acid sequence of the extracellular domain of the FcR-111 polypeptide having the amino acid sequence at positions 1-421 in SEQ ID NO:6, or as encoded by the FcR-III cDNA clone contained in ATCC Deposit No. 97891; (s) the amino acid sequence of the transmembrane domain of the FcR-111 polypeptide having the amino acid sequence at positions 422-448 in SEQ ID NO:6. or as encoded by the FcR-111 cDNA clone contained in ATCC Deposit No. 97891; (t) the amino acid sequence of the intracellular domain of the FcR-III polypeptide having the amino acid sequence at positions 449-607 in SEQ ID NO:6, or as encoded by the FcR-111 cDNA clone contained in ATCC Deposit No. 97891; (u) the amino acid sequence of a soluble FcR-111 polypeptide comprising the extracellular and intracelluar domains, but lacking the transmembrane domain; (v) the amino acid sequence of the complete FcR-IV polypeptide having the amino acid sequence positions -16 to 456 of SEQ ID NO:8 or the complete FcR-IV amino acid sequence encoded by the FcR-IV cDNA clone contained in ATCC Deposit No. 97891; (w) the amino acid sequence of the complete FcR-IV polypeptide having the amino acid sequence positions -15 to 456 of SEQ ID NO:8 or the complete FcR-IV amino acid sequence excepting the N-terminal methionine encoded by the FcR-IV cDNA clone contained in ATCC Deposit No. 97891; (x) the amino acid sequence of the mature FcR-IV polypeptide having the amino acid sequence at positions 1 to 456 in SEQ ID NO:8, or as encoded by the FcR-IV cDNA clone contained in ATCC Deposit No. 97891; (y) the amino acid sequence of the extracellular domain of the FcR-IV polypeptide having the amino acid sequence at positions 1-243 in SEQ ID NO:8, or as encoded by the FcR-IV cDNA clone contained in ATCC Deposit No. 97891; (z) the amino acid sequence of the transmembrane domain of the FcR-IV polypeptide having the amino acid sequence at positions 244-264 in SEQ ID NO:8. or as encoded by the FcR-IV cDNA clone contained in ATCC Deposit No. 97891; (aa) the amino acid sequence of the intracellular domain of the FcR-IV polypeptide having the amino acid sequence at positions 265-456 in SEQ ID NO:8, or as encoded by the FcR-IV cDNA clone contained in ATCC Deposit No. 97891; (ab) the amino acid sequence of a soluble FcR-IV polypeptide comprising the extracellular and intracelluar domains, but lacking the transmembrane domain; (ac) the amino acid sequence of the complete FcR-V polypeptide having the amino acid sequence positions -16 to 498 of SEQ ID NO:10 or the complete FcR-V amino acid sequence encoded by the FcR-V cDNA clone contained in ATCC Deposit No. 209100; (ad) the amino acid sequence of the complete FcR-V polypeptide having the amino acid sequence positions -15 to 498 of SEQ ID NO: 10 or the complete FcR-V amino acid sequence excepting the N-terminal methionine encoded by the FcR-V cDNA clone contained in ATCC Deposit No. 209100; (ae) the amino acid sequence of the mature FcR-V polypeptide having the amino acid sequence at positions 1 to 498 in SEQ ID NO:10, or as encoded by the FcR-V cDNA clone contained in ATCC Deposit No. 209100; (af) the amino acid sequence of the extracellular domain of the FcR-V polypeptide having the amino acid sequence at positions 1-343 in SEQ ID NO:10, or as encoded by the FcR-V cDNA clone contained in ATCC Deposit No. 209100; (ag) the amino acid sequence of the transmembrane domain of the FcR-V polypeptide having the amino acid sequence at positions 344-364 in SEQ ID NO:10, or as encoded by the FcR-V cDNA clone contained in ATCC Deposit No. 209100; (ah) the amino acid sequence of the intracellular domain of the FcR-V polypeptide having the amino acid sequence at positions 365-498 in SEQ ID NO:10, or as encoded by the FcR-V cDNA clone contained in ATCC Deposit No. 209100; (ai) the amino acid sequence of a soluble FcR-V polypeptide comprising the extracellular and intracelluar domains, but lacking the transmembrane domain. The polypeptides of the present invention also include polypeptides having an amino acid sequence at least 80% identical, more preferably at least 90% identical, and still more preferably 95%, 96%, 97%, 98% or 99% identical to those described in (a) through (ai) above, as well as polypeptides having an amino acid sequence with at least 90% similarity, and more preferably at least 95% similarity, to those above.

An additional embodiment of this aspect of the invention relates to a peptide or polypeptide which comprises the amino acid sequence of an epitope-bearing portion of an FcR-I, FcR-II, FcR-III, FcR-IV, or FcR-V polypeptide having an amino acid sequence described in (a) through (ai) above. Peptides or polypeptides having the amino acid sequence of an epitope-bearing portion of a FcR-I, FcR-II, FcR-III, FcR-IV, or FcR-V polypeptide of the invention include portions of such polypeptides with at least six or seven, preferably at least nine, and more preferably at least about 30 amino acids to about 50 amino acids, although epitope-bearing polypeptides of any length up to and including the entire amino acid sequence of a polypeptide of the invention described above also are included in the invention.

In another embodiment, the invention provides an isolated antibody that binds specifically to an FcR-I, FcR-II, FcR-III, FcR-IV, or FcR-V polypeptide having an amino acid sequence described in (a) through (ai) above. The invention further provides methods for isolating antibodies that bind specifically to an FcR-I, FcR-II, FcR-III, FcR-IV, or FcR-V polypeptide having an amino acid sequence as described herein. Such antibodies are useful diagnostically or therapeutically as described below.

The invention also provides for pharmaceutical compositions comprising FcR-I, FcR-II, FcR-III, FcR-IV, or FcR-V polypeptides, particularly human FcR-I, FcR-II, FcR-III, FcR-IV, or FcR-V polypeptides, which may be employed, for instance, to treat immune-complex related inflammatory diseases such as rheumatoid arthritis, systemic lupus erythematosis, autoimmune hemolytic anemia, thrombocytopenia and IgG- or IgE-mediated inflammation, anaphylaxis or allergy. Methods of treating individuals in need of FcR-I, FcR-II, FcR-III, FcR-IV, or FcR-V polypeptides are also provided.

The invention further provides compositions comprising an FcR-I, FcR-II, FcR-III, FcR-IV, or FcR-V polynucleotide or an FcR-I, FcR-II, FcR-III, FcR-IV, or FcR-V polypeptide for administration to cells *in vitro,* to cells *ex vivo* and to cells *in vivo,* or to a multicellular organism. In certain particularly preferred embodiments of this aspect of the invention, the compositions comprise an FcR-I, FcR-II, FcR-III, FcR-IV, or FcR-V polynucleotide for expression of an FcR-I, FcR-II, FcR-III, FcR-IV, or FcR-V polypeptide in a host organism for treatment of disease. Particularly preferred in this regard is expression in a human patient for treatment of a dysfunction associated with aberrant endogenous activity of FcR-I, FcR-II, FcR-III, FcR-IV, or FcR-V.

The present invention also provides a screening method for identifying compounds capable of enhancing or inhibiting a biological activity of the FcR-I, FcR-II, FcR-III, FcR-IV, or FcR-V polypeptides, which involves contacting a molecule (or molecules) which binds to the encoded proteins and modulates activity, which is inhibited or enhanced by the FcR-I, FcR-II, FcR-III, FcR-IV, or FcR-V polypeptides with the candidate compound in the presence of a FcR-I, FcR-II, FcR-III. FcR-IV. or FcR-V polypeptide, assaying the phosphorylation of the common γ chain or downstream signaling molecules or by using the ADCC assay to measure specfic effector-mediated killing target cells in the presence of the candidate compound and of FcR-I, FcR-II, FcR-III, FcR-IV, or FcR-V polypeptides, and comparing the ligand activity to a standard level of activity, the standard being assayed when contact is made between the ligand and in the presence of an FcR-I, FcR-II, FcR-III, FcR-IV, or FcR-V polypeptide and the absence of the candidate compound In this assay, an increase in ADCC killing activity over the standard indicates that the candidate compound is an agonist of FcR-I, FcR-II, FcR-III, FcR-IV, or FcR-V activity and a decrease in ADCC killing activity compared to the standard indicates that the compound is an antagonist of FcR-I, FcR-II, FcR-III, FcR-IV, or FcR-V activity.

In another aspect, a screening assay for agonists and antagonists is provided which involves determining the effect a candidate compound has on FcR-I, FcR-II, FcR-III, FcR-IV, or FcR-V binding to a ligand. In particular, the method involves contacting the ligand with an FcR-I, FcR-II, FcR-III, FcR-IV, or FcR-V polypeptide and a candidate compound and determining whether FcR-I, FcR-II, FcR-III, FcR-IV, or FcR-V polypeptide binding to the ligand is increased or decreased due to the presence of the candidate compound. In this assay, an increase in binding of FcR-I, FcR-II, FcR-III, FcR-IV, or FcR-V over the standard binding indicates that the candidate compound is an agonist of FcR-I, FcR-II, FcR-III, FcR-IV, or FcR-V binding activity and a decrease in FcR-I, FcR-II, FcR-III, FcR-IV, or FcR-V binding compared to the standard indicates that the compound is an antagonist of FcR-I, FcR-II, FcR-III, FcR-IV, or FcR-V binding activity.

It has been discovered that Fclt-I, FcR-II, FcR-111, FcR-IV, or FcR-V are expressed not only in activated monocytes, primary dendritic cells, macrophages, and macrophages, respectively, but also in several additional cell and tissue types. For example, in addition to activated monocytes, expression of FcR-1 has also been detected in primary dendritic cells, GM-CSF-treated macrophages, macrophages, bone marrow, poly-[I-C]-stimulated pBMCs, and activated neutrophils. FcR-II clones were detected not only in primary dendritic cells, but also in cDNA libraries constructed from GM-CSF-treated macrophages, bone marrow, activated monocytes, activated neutrophils, hemangiopericytoma, colon cancer cells, kidney cortex, and whole, human 8 week old embryo. Message encoding FcR-III can be found not only in macrophages, but also in GM-CSF-treated macrophages, primary dendritic cells, activated monocytes, and activated neutrophils. In addition to macrophages, FcR-IV can be detected in primary dendritic cells, spleen, activated macrophages, adult pulmonary tissue, and activated monocytes. Finally, in addition to GM-CSF-treated macrophages, FcR-V can be detected in activated monocytes, monocytes, primary dendritic cells, bone marrow, CD34-depleted leukocytes, and activated neutrophils. Therefore, nucleic acids of the invention are useful as hybridization probes for differential identification of the tissue(s) or cell type(s) present in a biological sample. Similarly, polypeptides and antibodies directed to those polypeptides are useful to provide immunological probes for differential identification of the tissue(s) or cell type(s) In addition, for a number of disorders of the above tissues or cells, particularly of the immune system, significantly higher or lower levels of FcR-I, FcR-II, FcR-III, FcR-IV, or FcR-V gene expression may be detected in certain tissues (e.g., cancerous and wounded tissues) or bodily fluids (e.g., serum, plasma, urine, synovial fluid or spinal fluid) taken from an individual having such a disorder, relative to a "standard" FcR-1, FcR-II, FcR-III, FcR-IV, or FcR-V gene expression level, i.e., the FcR-1. FcR-11, FcR-III. FcR-IV, or FcR-V expression level in healthy tissue from an individual not having the immune system disorder. Thus, the invention provides a diagnostic method useful during diagnosis of such a disorder, which involves: (a) assaying FcR-I. FcR-II, FcR-III, FcR-IV, or FcR-V gene expression level in cells or body fluid of an individual; (b) comparing the FcR-I, FcR-II, FcR-III, FcR-IV, or FcR-V gene expression level with a standard FcR-I, FcR-II, FcR-III, FcR-IV, or FcR-V gene expression level, whereby an increase or decrease in the assayed FcR-I, FcR-II, FcR-III, FcR-IV, or FcR-V gene expression level compared to the standard expression level is indicative of disorder in the immune system.

An additional aspect of the invention is related to a method for treating an individual in need of an increased level of FcR-I, FcR-II, FcR-III, FcR-IV, or FcR-V activity in the body comprising administering to such an individual a composition comprising a therapeutically effective amount of an isolated FcR-I, FcR-II, FcR-III, FcR-IV, or FcR-V polypeptide of the invention or an agonist thereof.

A still further aspect of the invention is related to a method for treating an individual in need of a decreased level of FcR-I, FcR-II, FcR-III, FcR-IV, or FcR-V activity in the body comprising, administering to such an individual a composition comprising a therapeutically effective amount of an FcR-I, FcR-II, FcR-III, FcR-IV, or FcR-V antagonist. Preferred antagonists for use in the present invention are FcR-I, FcR-II, FcR-III, FcR-IV, or FcR-V-specific antibodies.

### Brief Description of the Figures

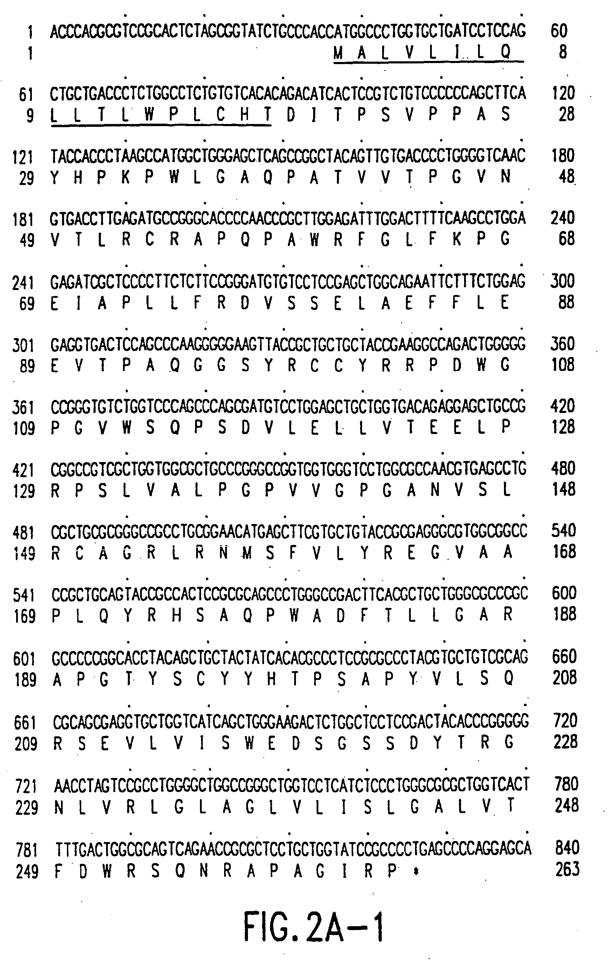

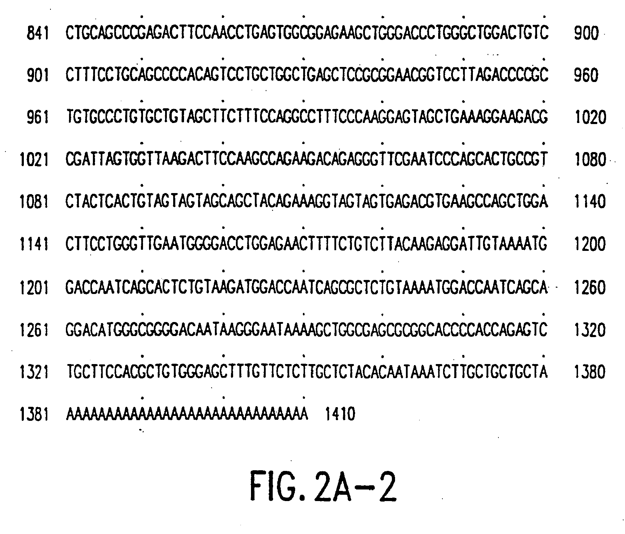

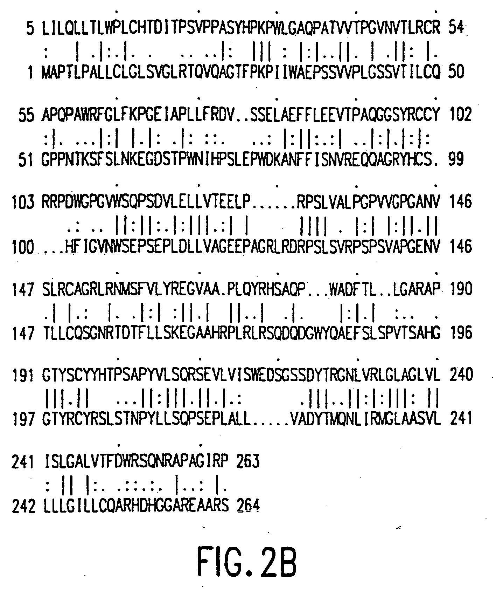

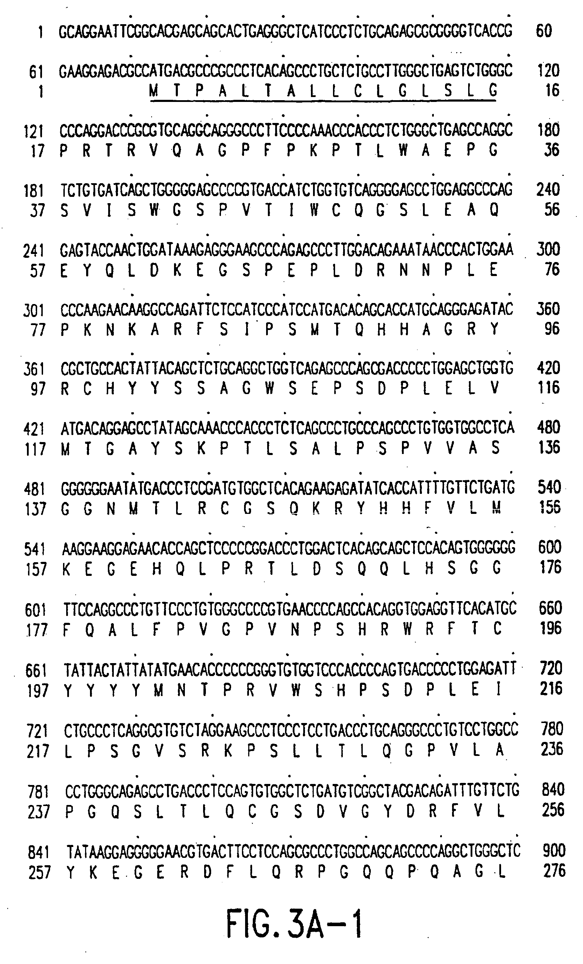

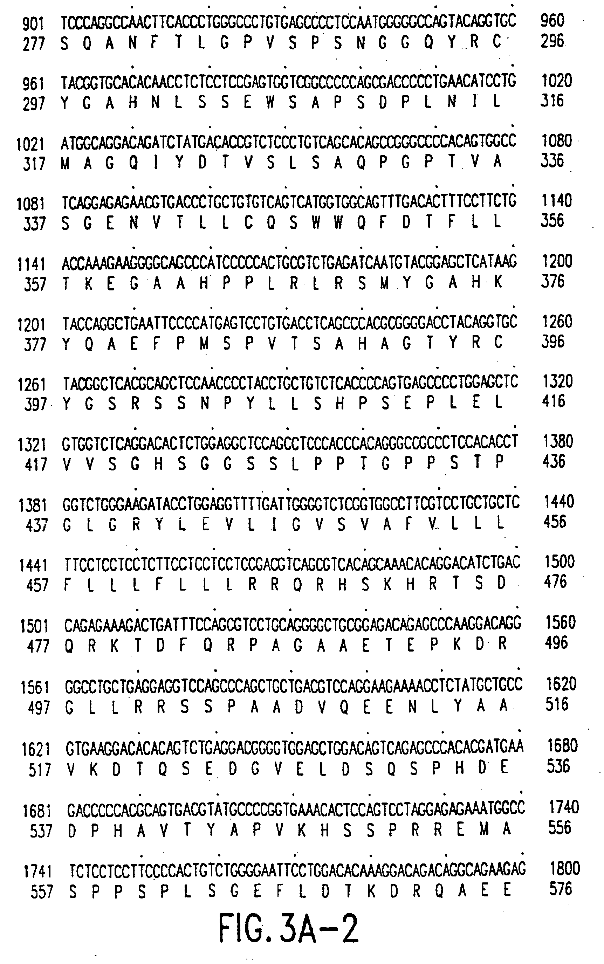

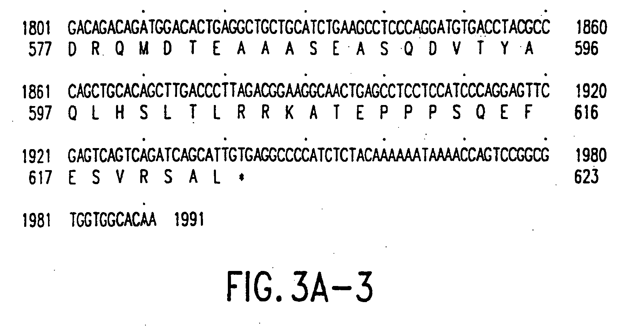

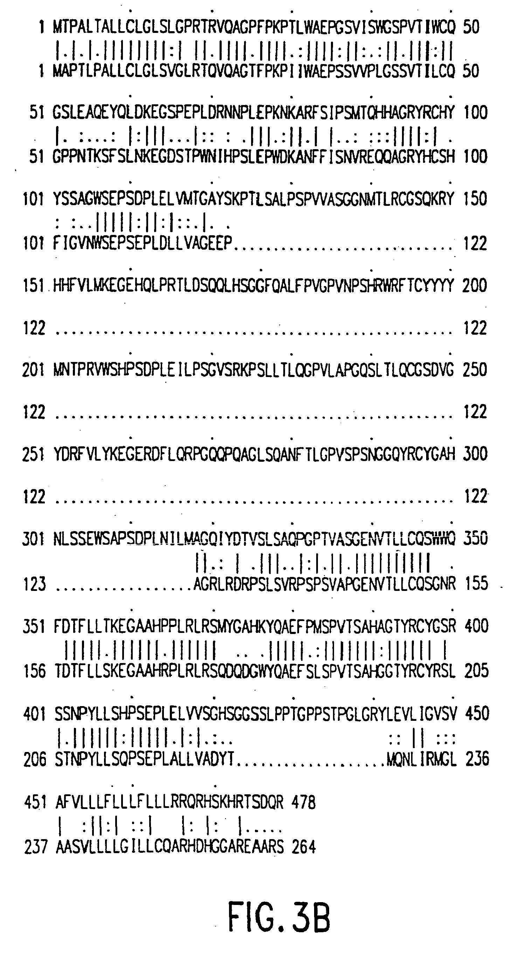

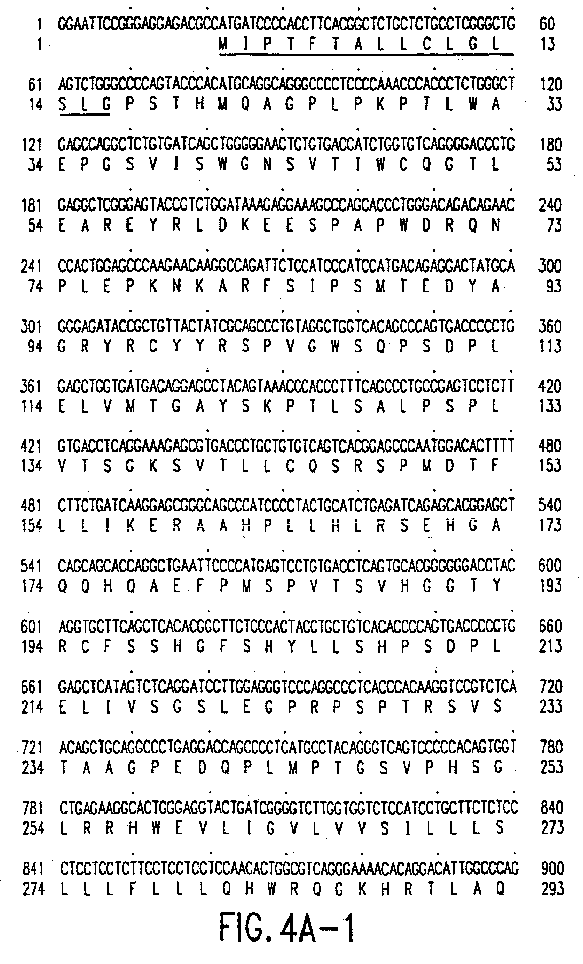

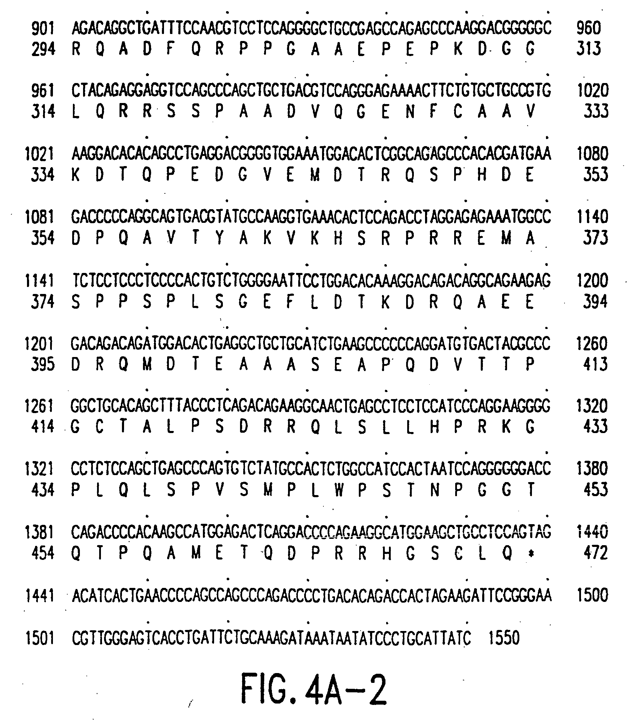

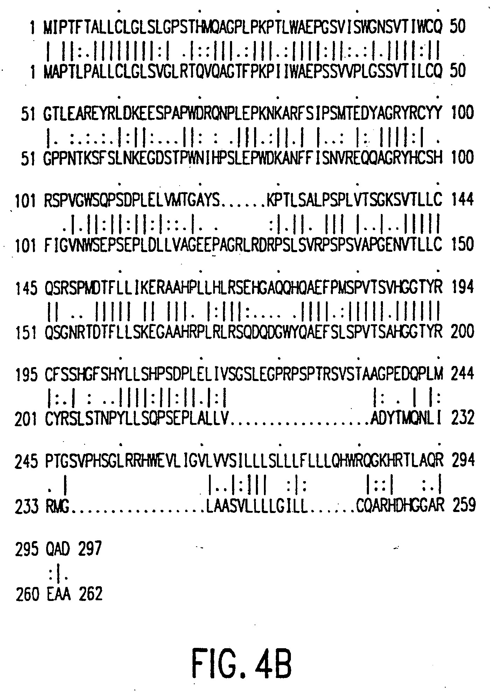

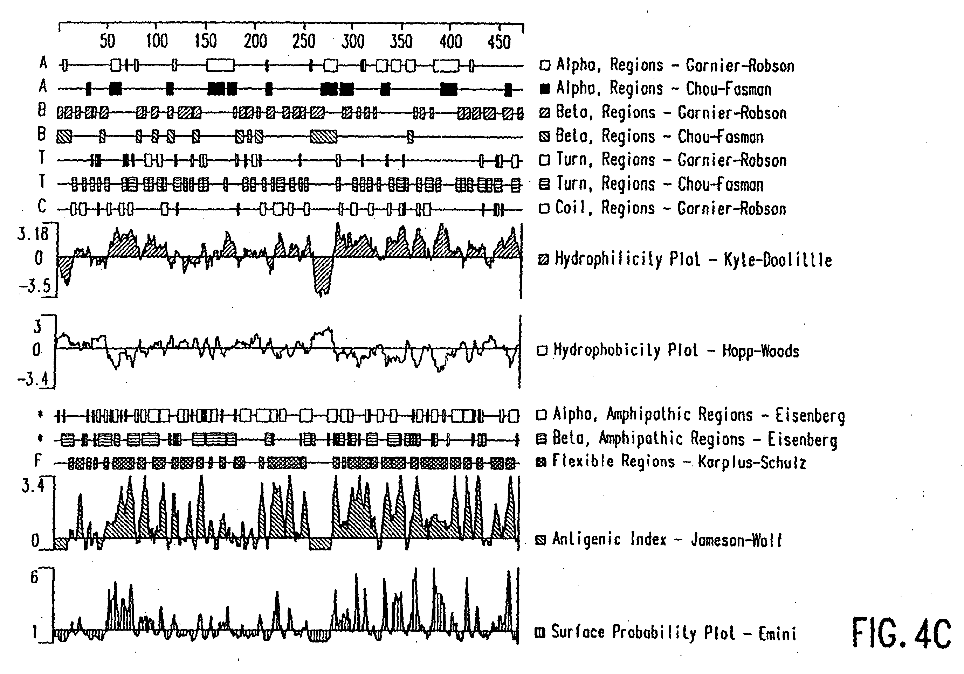

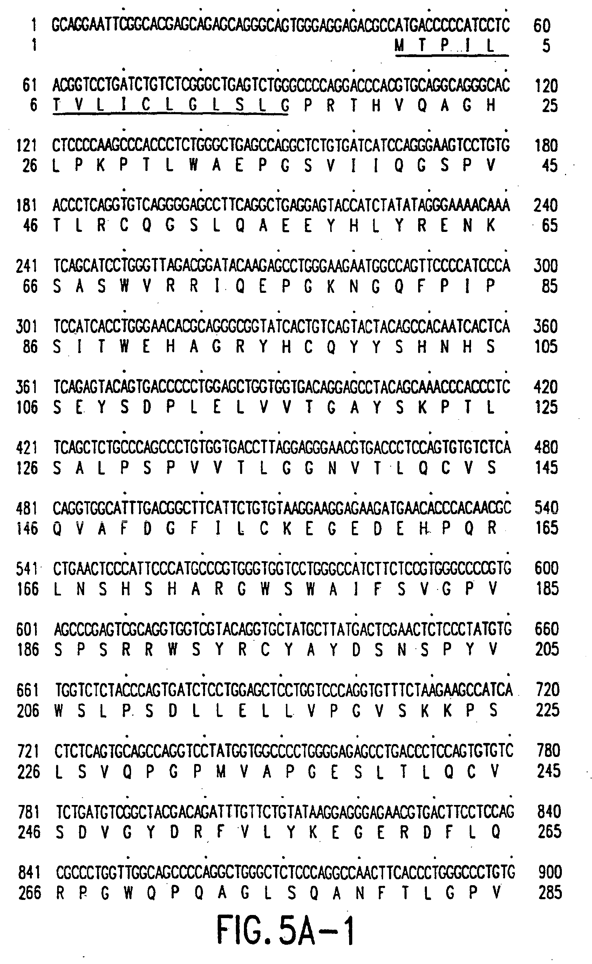

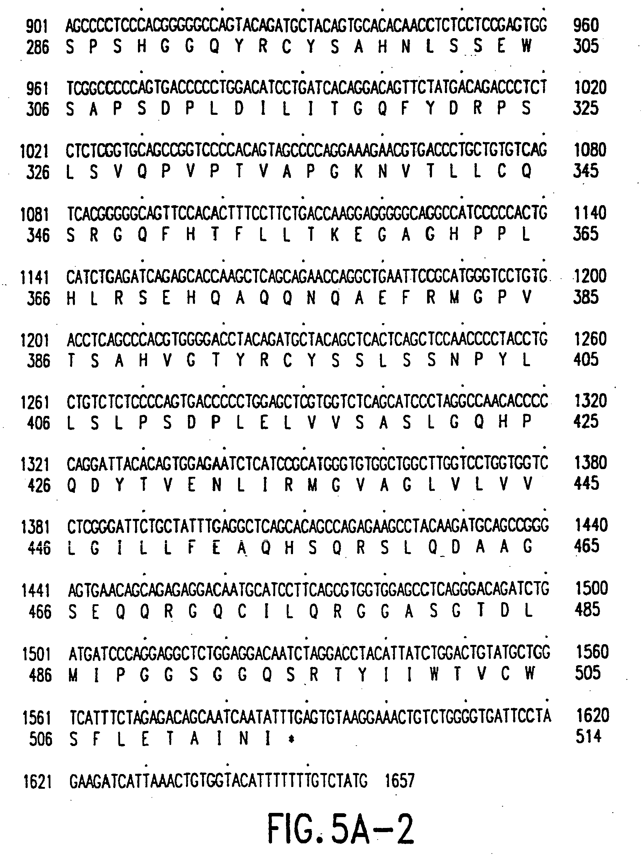

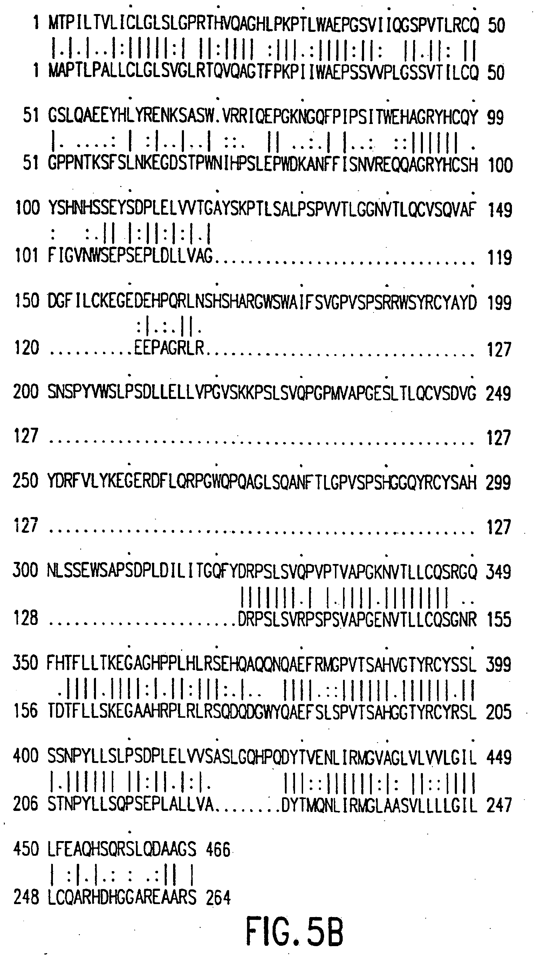

Figure 1A shows the nucleotide sequence (SEQ ID NO:1) and deduced amino acid sequence (SEQ ID NO:2) of FcR-I. Figure 2A shows the nucleotide sequence (SEQ ID NO:3) and deduced amino acid sequence (SEQ ID NO:4) of FcR-II. Figure 3A shows the nucleotide sequence (SEQ ID NO:5) and deduced amino acid sequence (SEQ ID NO:6) of FcR-III. Figure 4A shows the nucleotide sequence (SEQ ID NO:7) and deduced amino acid sequence (SEQ ID NO:8) of FcR-IV. Figure 5A shows the nucleotide sequence (SEQ ID NO:9) and deduced amino acid sequence (SEQ ID NO:10) of FcR-V.

The predicted leader sequences of about 21, 18, 16, 16, or 16 amino acids in SEQ ID NO:2, SEQ ID NO:4, SEQ ID NO:6, SEQ ID NO:8, or SEQ ID NO:10, respectively, are underlined. Note that the methionine residue at the beginning of the leader sequence in Figures 1A, 2A, 3A, 4A, and 5A are shown in position number (positive) 1, whereas the leader positions in the corresponding sequences of SEQ ID NO:2, SEQ ID NO:4, SEQ ID NO:6, SEQ ID NO:8, and SEQ ID NO:10, respectively, are designated with negative position numbers. Thus, the leader sequence positions 1 to 21 in Figure 1A correspond to positions -21 to -1 in SEQ ID NO:2. Likewise, the leader sequence positions 1 to 18 in Figure 2A correspond to positions -18 to -1 in SEQ ID NO:4. Also, the leader sequence positions 1 to 16 in Figure 3A correspond to positions -16 to -1 in SEQ ID NO:6. The leader sequence positions 1 to 16 in Figure 4A correspond to positions -16 to -1 in SEQ ID NO:8. And, finally, the leader sequence positions 1 to 16 in Figure 5A correspond to positions -16 to -1 in SEQ ID NO:10.

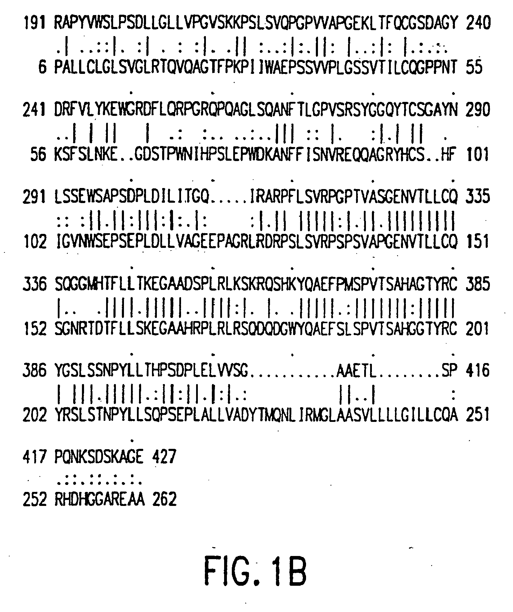

Figures 1B, 2B, 3B, 4B, and 5B show the regions of identity between the amino acid sequences of the FcR-I, FcR-II, FcR-III, FcR-IV, and FcR-V proteins and the translation product of the bovine mRNA for Fc-γ2 Receptor (SEQ ID NO:11), respectively, as determined by the computer program Bestfit (Wisconsin Sequence Analysis Package, Version 8 for Unix, Genetics Computer Group, University Research Park, 575 Science Drive. Madison, WI 53711) using the default parameters."

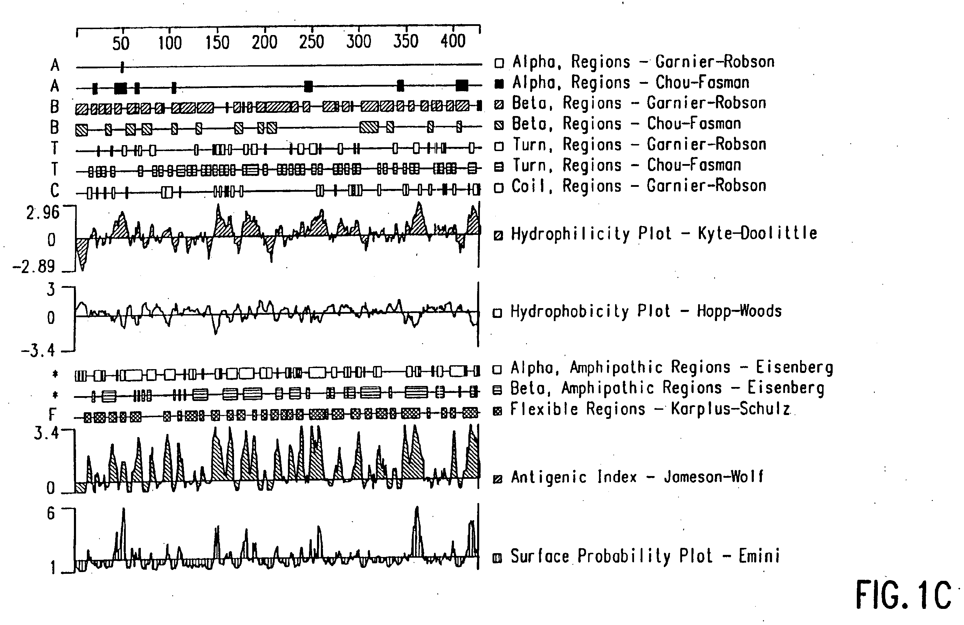

Figures 1C, 2C, 3C. 4C, and 5C show DNASTAR computer analyses of the FcR-1. FcR-11, FcR-111, FcR-IV, and FcR-V amino acid sequences (DNASTAR, Inc., Madison. WI). Alpha, beta, turn and coil regions; hydrophilicity and hydrophobicity; amphipathic regions; flexible regions; antigenic index and surface probability are shown. In the "Antigenic Index-Jameson-Wolf" graph, the positive peaks indicate locations of the highly antigenic regions of the FcR-1, FcR-II, FcR-III, FcR-IV, and FcR-V proteins. i.e., regions from which epitope-bearing peptides of the invention can be obtained.

### Detailed Description

The present invention provides isolated nucleic acid molecules comprising a polynucleotide encoding an FcR-I, FcR-II, FcR-III, FcR-IV, and FcR-V polypeptide having the amino acid sequence shown in SEQ ID NO:2. SEQ ID NO:4, SEQ ID NO:6. SEQ ID NO:8. SEQ ID NO:10, respectively, which were determined by sequencing cloned cDNAs. The nucleotide sequences shown in Figures 1A, 2A. 3A. and 4A (SEQ ID NO:1. SEQ ID NO:3, SEQ ID NO:5, and SEQ ID NO:7, respectively) were obtained by sequencing the cDNA clones designated 733890 (HMQDO20), 1629586 (HDPMK33), 197745 (HMPAP73), and 1446672 (HMSHH46) respectively, which were pooled and deposited on February 21, 1997 at the American Type Culture Collection, 12301 Park Lawn Drive, Rockville, Maryland 20852, and given accession number ATCC 97891. The nucleotide sequence shown in Figure 5A (SEQ ID NO:9) was obtained by sequencing the cDNA clone designated 709035 (HMAAB68), which was deposited on June 6, 1997 at the American Type Culture Collection, 12301 Park Lawn Drive, Rockville, Maryland 20852, and given accession number ATCC 209100. The deposited clones are contained in the pBluescript SK(-) plasmid (Stratagene, La Jolla, CA), except for HDMPK33, which is contained in the pCMVSport 3.0 plasmid vector (Life Technologies, Gaithersburg, MD).

The FcR-I, FcR-II, FcR-III, FcR-IV, and FcR-V proteins of the present invention share sequence homology with the translation product of the bovine mRNA for Fc-γ2 receptor (SEQ ID NO: 11). Fc-γ2 receptor is thought to function as an important trigger of complex immune defense responses including phagocytosis, antibody-dependent cellular cytotoxicity, and release of inflammatory mediators (Clynes, R. and Ravetch, J. V., (1995) Immunity 3:21-26; Miller, K. L, et al., (1996) Exp. Med 183:2227-2233). Such processes can ultimately lead to cellular destruction and the amplification of the inflammatory response (Gallin, J. I. (1993) Inflammation. In: Fundamental Immunology, Third Edition, W. Paul, ed.; New York: Raven Press; pp. 1015-1032). Furthermore, FcRs appear to play a dominant role in an early step in type II hypersensitivity reactions.

### Nucleic Acid Molecules

Unless otherwise indicated, all nucleotide sequences determined by sequencing a DNA molecule herein were determined using an automated DNA sequencer (such as the Model 373 from Applied Biosystems, Inc., Foster City, CA), and all amino acid sequences of polypeptides encoded by DNA molecules determined herein were predicted by translation of a DNA sequence determined as above. Therefore, as is known in the art for any DNA sequence determined by this automated approach, any nucleotide sequence determined herein may contain some errors. Nucleotide sequences determined by automation are typically at least about 90% identical, more typically at least about 95% to at least about 99.9% identical to the actual nucleotide sequence of the sequenced DNA molecule. The actual sequence can be more precisely determined by other approaches including manual DNA sequencing methods well known in the art. As is also known in the art, a single insertion or deletion in a determined nucleotide sequence compared to the actual sequence will cause a frame shift in translation of the nucleotide sequence such that the predicted amino acid sequence encoded by a determined nucleotide sequence will be completely different from the amino acid sequence actually encoded by the sequenced DNA molecule, beginning at the point of such an insertion or deletion.

By "nucleotide sequence" of a nucleic acid molecule or polynucleotide is intended, for a DNA molecule or polynucleotide, a sequence of deoxyribonucleotides, and for an RNA molecule or polynucleotide, the corresponding sequence of ribonucleotides (A, G, C and U), where each thymidine deoxyribonucleotide (T) in the specified deoxyribonucleotide sequence is replaced by the ribonucleotide uridine (U).

Using the information provided herein, such as the nucleotide sequences in Figures 1A, 2A, 3A, 4A, and 5A (SEQ ID NO:1, SEQ ID NO:3, SEQ ID NO:5, SEQ ID NO: 7. and SEQ ID NO:9, respectively), nucleic acid molecules of the present invention encoding five novel Fc-R-like polypeptides may be obtained using standard cloning and screening procedures, such as those for cloning cDNAs using mRNA as starting material. Illustrative of the invention, the nucleic acid molecule described in Figure 1A (SEQ ID NO:1) was discovered in a cDNA library derived from activated monocytes. The nucleic acid molecule described in Figure 2A (SEQ ID NO:3) was discovered in a cDNA library derived from primary dendritic cells. The nucleic acid molecules described in Figures 3A (SEQ ID NO:5) and 4A (SEQ ID NO:7) were discovered in cDNA libraries derived from macrophages. The nucleic acid molecules described in Figure 5A (SEQ ID NO:9) was discovered in a cDNA library derived from GM-CSF-treated macrophages.

Additional clones of FcR-I (SEQ ID NO:1) were also identified in cDNA libraries from the following cells and/or tissues: primary dendritic cells. GM-CSF-treated macrophages, macrophages, bone marrow, poly-[I-C]-stimulated pBMCs, and activated neutrophils. Additional clones of FcR-II (SEQ ID NO:3) were also identified in cDNA libraries from the following cells and/or tissues: GM-CSF-treated macrophages, bone marrow, activated monocytes, activated neutrophils, hemangiopericytoma, colon cancer, kidney cortex, and whole 8 week old embryo. Additional clones of FcR-III (SEQ ID NO:5) were also identified in cDNA libraries from the following cells and/or tissues: GM-CSF-treated macrophages, primary dendritic cells, activated monocytes, and activated neutrophils. Additional clones of FcR-IV (SEQ ID NO:7) were also identified in cDNA libraries from the following cells and/or tissues: primary dendritic cells, spleen, activated macrophages, adult pulmonary tissue, and activated monocytes. Additional clones of FcR-V (SEQ ID NO:9) were also identified in cDNA libraries from the following cells and/or tissues: activated monocytes, monocytes, primary dendritic cells, bone marrow, CD34-depleted leukocytes, and activated neutrophils.

The determined nucleotide sequences of the FcR-I. FcR-II, FcR-III, FcR-IV. and FcR-V cDNAs of Figures 1A, 2A, 3A, 4A, and 5A (SEQ ID NO:1, SEQ ID NO:3. SEQ ID NO:5, SEQ ID NO:7, and SEQ ID NO:9, respectively) which contain open reading frames encoding proteins of 427 263, 623, 472, and 514 amino acid residues, respectively, with initiation codons at nucleotide positions 82-84, 37-39, 135-137, 22-24, and 46-48. respectively, of the nucleotide sequences in Figures 1A, 2A, 3A, 4A, and 5A (SEQ ID NO:1, SEQ ID NO:3, SEQ ID NO:5, SEQ ID NO:7, and SEQ ID NO:9, respectively), and deduced molecular weights of about 46.2, 28.8, 53.7, 47.2, and 56.3 kDa, respectively. The amino acid sequences of the FcR-I, FcR-II, FcR-III, FcR-IV, and FcR-V proteins shown in SEQ ID NO:2, SEQ ID NO:4, SEQ ID N0:6, SEQ ID N0:8, and SEQ ID NO: 10. respectively, are about 45.1, 37.6. 53.8, 68.0, and 55.5% identical to the mRNA encoding the bovine Fc-γ2 receptor (Figures 1B, 2B, 3B, 4B, and 5B, respectively). The bovine Fc-γ2 receptor (Zhang, G., et al., J. Immunol. 155:1534-1541; 1995) can be accessed through the GenBank database using accession number Z37506.

The open reading frames of the FcR-I, FcR-II, FcR-III, FcR-IV, and FcR-V genes share sequence homology with the translation product of the bovine mRNA for the Fc-γ2 receptor (SEQ ID NO:9; see also Figures 1B, 2B, 3B, 4B, and 5B), including the following conserved domains: (a) the predicted extracellular domains of about 288, 210, 421, 243. and 343 amino acids, respectively; (b) the predicted transmembrane domain of about 22. 17. 26. 20. and 20 amino acids, respectively, and (c) the intracytoplasmic domain of about 93, 15, 158. 191. and 133 amino acids, respectively.

The amino acid sequences of the novel FcR-I, FcR-II. FcR-III, FcR-IV, and FcR-V molecules and the Fc-γ2 receptor are also especially related in the Ig-like repeat elements of the extracellular domain. In general, the conservation of such Ig-like domains is a hallmark of FcRs. The FcR Ig-like domain is characterized by two main conserved structural amino acid sequences, each of which is centered around a cysteine residue (Raghavan, M. and Bjorkman, P. J. Annu. Rev. Cell Dev. 12:181-220; 1996). The first of these is a β-turn connecting strand identifiable at the primary sequence level by the sequence Gxx*x*xC, where x is any amino acid and * is any hydrophobic amino acid (particularly Leucine, isoleucine, or valine). The second conserved sequence component of the FcR Ig-like domain has been designated the tyrosine corner and comprises the sequence Lx*xx*xxxDx#xYxC, where, as above, x is any amino acid and * is any hydrophobic amino acid (particularly Leucine, isoleucine, or valine), and # is any small or acid amino acid (particularly glycine, alanine, or aspartic acid). Each of the novel FcR molecules of the present invention characteristically contains either two or three of the above-mentioned repeat sequence pairs. For example, FcR-I contains three pairs of the Ig-like domains in its extracellular domain located around the three pairs cysteine residues located at positions 16 and 65, 112 and 164, and 213 and 264 of SEQ ID NO:2. FcR-II, however, contains only two pairs of the Ig-like domains in its extracellular domain located around the two pairs cysteine residues located at positions 35 and 82 and 132 and 177 of SEQ ID NO:4. Similarly to FcR-1, FcR-III also contains three pairs of the Ig-like domains in its extracellular domain located around the three pairs cysteine residues located at positions 33 and 82, 128 and 180, and 239 and (339 or 390) of SEQ ID NO:6. FcR-IV, like FcR-II, again contains only two pairs of the Ig-like domains in its extracellular domain located around the two pairs cysteine residues located at positions 33 and 82 and 128 and 179 of SEQ ID NO:8. Finally, FcR-V contains three pairs of the Ig-like domains in its extracellular domain located around the three pairs cysteine residues located at positions 33 and 81, 139 and 179, and 228 and 279 of SEQ ID NO:10. The Fc-γ2 receptor is thought to be important in modulation of the immune and hematopoietic systems. The homology between the Fc-γ2 receptor and FcR-I, FcR-II, FcR-III, FcR-IV, and FcR-V indicates that FcR-I, FcR-II, FcR-III, FcR-IV, and FcR-V may also be involved in modulation of the immune and hematopoietic systems.

As one of ordinary skill would appreciate, due to the possibilities of sequencing errors discussed above, the actual complete FcR-I, FcR-II, FcR-III, FcR-IV, and FcR-V polypeptides encoded by the deposited cDNAs, which comprises about 427. 263. 623, 472, and 514 amino acids, respectively, may be somewhat longer or shorter. In fact, the actual open reading frames may be anywhere in the range of ±20 amino acids, more likely in the range of ±10 amino acids, of that predicted from the methionine codon from the N-terminus shown in Figures 1A, 2A, 3A, 4A, and 5A (SEQ ID NO:1. SEQ ID NO:3, SEQ ID NO:5. SEQ ID NO:7, SEQ ID NO:9, respectively). It will further be appreciated that, depending on the analytical criteria used for identifying various functional domains, the exact "address" of the extracellular, transmembrane, and intracytoplasmic domains of the FcR-I, FcR-II: FcR-III. FcR-IV, and FcR-V polypeptides may differ slightly from the predicted positions above. For example, the exact location of the FcR-I, FcR-II, FcR-III, FcR-IV, and FcR-V extracellular domains in SEQ ID NO:2, SEQ ID NO:4, SEQ ID NO:6, SEQ ID NO:8, and SEQ ID NO:10, respectively, may vary slightly (e.g., the address may "shift" by about 1 to about 20 residues, more likely about 1 to about 5 residues) depending on the criteria used to define the domain. In this case, the ends of the transmembrane domains and the beginning of the extracellular domains were predicted on the basis of the identification of the hydrophobic amino acid sequence in the above indicated positions, as shown in Figures 1C, 2C, 3C, 4C, and 5C. In any event, as discussed further below, the invention further provides polypeptides having various residues deleted from the N-terminus of the complete polypeptide, including polypeptides lacking one or more amino acids from the N-terminus of the extracellular domain described herein, which constitute soluble forms of the extracellular domains of the FcR-I, FcR-II, FcR-III, FcR-IV, and FcR-V proteins.

### Leader and Mature Sequences

The amino acid sequences of the complete FcR-I, FcR-II, FcR-III, FcR-IV, and FcR-V proteins include a leader sequence and a mature protein, as shown in SEQ ID NO:2, SEQ ID NO:4, SEQ ID NO:6, SEQ ID NO:8, and SEQ ID NO:10, respectively. More in particular, the present invention provides nucleic acid molecules encoding a mature form of the FcR-I, FcR-II, FcR-III, FcR-IV, and FcR-V proteins. Thus, according to the signal hypothesis, once export of the growing protein chain across the rough endoplasmic reticulum has been initiated, proteins secreted by mammalian cells have a signal or secretory leader sequence which is cleaved from the complete polypeptide to produce a secreted "mature" form of the protein. Most mammalian cells and even insect cells cleave secreted proteins with the same specificity. However, in some cases, cleavage of a secreted protein is not entirely uniform, which results in two or more mature species of the protein. Further, it has long been known that the cleavage specificity of a secreted protein is ultimately determined by the primary structure of the complete protein, that is, it is inherent in the amino acid sequence of the polypeptide. Therefore, the present invention provides a nucleotide sequence encoding the mature FcR-I, FcR-II, FcR-III, FcR-IV, and FcR-V polypeptides having the amino acid sequences encoded by the FcR-I, FcR-II, FcR-III, and FcR-IV cDNA clones contained in ATCC Deposit No. 97891 and having the amino acid sequence encoded by the FcR-V cDNA clone contained in ATCC Deposit No. 209100. By the "mature FcR-I, FcR-II, FcR-III, FcR-IV, and FcR-V polypeptides having the amino acid sequences encoded by the FcR-I, FcR-II, FcR-III, and FcR-IV cDNA clones contained in ATCC Deposit No. 97891 and having the amino acid sequence encoded by the FcR-V cDNA clone contained in ATCC Deposit No. 209100" is meant the mature forms of the FcR-I, FcR-II, FcR-III, FcR-IV, and FcR-V proteins produced by expression in a mammalian cell (e.g., COS cells, as described below) of the complete open reading frame encoded by the human DNA sequence of the clone contained in the vector in the deposited host.

In addition, methods for predicting whether a protein has a secretory leader as well as the cleavage point for that leader sequence are available. For instance, the method of McGeoch (*Virus Res.* **3:**271-286; 1985) uses the information from a short N-terminal charged region and a subsequent uncharged region of the complete (uncleaved) protein. The method of von Heinje (Nucleic Acids Res. 14:4683-4690; 1986) uses the information from the residues surrounding the cleavage site, typically residues -13 to +2 where +1 indicates the amino terminus of the mature protein. The accuracy of predicting the cleavage points of known mammalian secretory proteins for each of these methods is in the range of 75-80% (von Heinje, *supra*). However, the two methods do not always produce the same predicted cleavage point(s) for a given protein.

In the present case, the deduced amino acid sequence of the complete FcR-I, FcR-II, FcR-III, FcR-IV, and 1 cR-V polypeptides were analyzed by the computer program designated PSORT. The program is an expert system for predicting the cellular location of a protein based on the amino acid sequence and is available from The Institute for Chemical Research. Kyoto University (see Nakai, K. and Kanehisa, M. Genomics 14:897-911; 1992). As part of this computational prediction of localization, the methods of McGeoch and von Heinje are incorporated. The analysis of the FcR-I, FcR-II, FcR-III, FcR-IV, and FcR-V amino acid sequences by this program predicted a single N-terminal signal sequence within the complete amino acid sequences shown in SEQ ID NO:2, SEQ ID NO:4, SEQ ID NO:6, SEQ ID NO:8. and SEQ ID NO:10.

As one of ordinary skill would appreciate from the above discussions, due to the possibilities of sequencing errors as well as the variability of cleavage sites in different known proteins, the mature FcR-I, FcR-II, FcR-III, FcR-IV, and FcR-V polypeptides encoded by the deposited cDNAs are expected to consist of about 406, 245, 607, 456, and 498 amino acids, respectively (presumably residues 1 to 406, 1 to 245, 1 to 607, 1 to 456, 1 to 498, respectively, of SEQ ID NO:2, SEQ ID NO:4, SEQ ID NO:6, SEQ ID NO:8, and SEQ ID NO:10, respectively, but may consist of any number of amino acids in the range of about 1 to 386-426, 1 to 225-265, 1 to 587-627, 1 to 436-476, 1 to 478-528 amino acids, respectively; and the actual leader sequence(s) of this protein is expected to be 21, 18, 16, 16, and 16 amino acids (presumably residues -21 through -1, -18 through -1, -16 through -1, -16 through -1, and -16 through -1 of SEQ ID NO:2, SEQ ID NO:4, SEQ ID NO:6, SEQ ID NO:8, and SEQ ID NO:10, respectively, but may consist of any number of amino acids in the range of 10-31. 10-28, 10-26, 10-26, and 10-26 amino acids.

As indicated, nucleic acid molecules of the present invention may be in the form of RNA, such as mRNA, or in the form of DNA, including, for instance, cDNA and genomic DNA obtained by cloning or produced synthetically. The DNA may be double-stranded or single-stranded. Single-stranded DNA or RNA may be the coding strand, also known as the sense strand, or it may be the non-coding strand, also referred to as the anti-sense strand.

By "isolated" nucleic acid molecule(s) is intended a nucleic acid molecule, DNA or RNA, which has been removed from its native environment For example, recombinant DNA molecules contained in a vector are considered isolated for the purposes of the present invention. Further examples of isolated DNA molecules include recombinant DNA molecules maintained in heterologous host cells or purified (partially or substantially) DNA molecules in solution. Isolated RNA molecules include *in vivo* or *in vitro* RNA transcripts of the DNA molecules of the present invention. Isolated nucleic acid molecules according to the present invention further include such molecules produced synthetically.

Isolated nucleic acid molecules of the present invention include DNA molecules comprising an open reading frame (ORF) with an initiation codon at nucleotide positions 82-84, 37-39,135-137,22-24, and 46-48 of the nucleotide sequences shown in Figures 1A, 2A, 3A, 4A, and 5A, respectively, (SEQ ID NO:1, SEQ ID NO:3, SEQ ID NO:5, SEQ ID NO:7, and SEQ ID NO:10. respectively).

Also included are DNA molecules comprising the coding sequence for the predicted mature FcR-I, FcR-II, FcR-III, FcR-IV, and FcR-V proteins shown at positions 1-406, 1-245, 1-607, 1-456, and 1-498 of SEQ ID NO:2. SEQ ID NO:4. SEQ ID NO:6. SEQ ID NO:8, and SEQ ID NO:10, respectively.

In addition, isolated nucleic acid molecules of the invention include DNA molecules which comprise a sequence substantially different from those described above but which, due to the degeneracy of the genetic code, still encode the FcR-I, FcR-II, FcR-III, FcR-IV, and FcR-V proteins. Of course, the genetic code and species-specific codon preferences are well known in the art. Thus, it would be routine for one skilled in the art to generate the degenerate variants described above, for instance, to optimize codon expression for a particular host (e.g., change codons in the human mRNA to those preferred by a bacterial host such as *E*. *coli*).

In another aspect, the invention provides isolated nucleic acid molecules encoding the FcR-I, FcR-II, FcR-III, FcR-IV, and FcR-V polypeptides having amino acid sequences encoded by the FcR-I, FcR-II, FcR-III, and FcR-IV cDNA clones contained in the plasmids deposited as ATCC Deposit No. 97891 on February 21, 1997 and having the amino acid sequences encoded by the FcR-V cDNA clone contained in the plasmid deposited as ATCC Deposit No. 209100 on June 6, 1997. Preferably, this nucleic acid molecule will encode the mature polypeptides encoded by the above-described deposited cDNA clones.

The invention further provides isolated nucleic acid molecules having the nucleotide sequences shown in Figures 1A, 2A, 3A, 4A, and 5A (SEQ ID NO:1, SEQ ID NO:3, SEQ ID NO:5, SEQ ID NO:7, and SEQ ID NO:9, respectively), or the nucleotide sequence of the FcR-I, FcR-II, FcR-III, FcR-IV, or FcR-V cDNAs contained in the above-described deposited clones, or a nucleic acid molecule having a sequence complementary to one of the above sequences. Such isolated molecules, particularly DNA molecules, are useful as probes for gene mapping, by *in situ* hybridization with chromosomes, and for detecting expression of the FcR-I, FcR-II, FcR-III, FcR-IV, or FcR-V genes in human tissue, for instance, by Northern blot analysis.

The present invention is further directed to nucleic acid molecules encoding portions of the nucleotide sequences described herein as well as to fragments of the isolated nucleic acid molecules described herein. In particular, the invention provides polynucleotides having a nucleotide sequence representing the portion of SEQ ID NO:1, SEQ ID NO:3, SEQ ID NO:5, SEQ ID NO:7, and SEQ ID NO:9 which consists of positions 82-1362, 37-825. 73-1939, 22-1437.46-1587, respectively.

In addition to the above-described nucleic acid molecules, the invention provides nucleic acid molecules having nucleotide sequences related to extensive portions of SEQ ID NO: 1 which have been determined from the following related cDNA clones: HMQDO20R (SEQ ID NO:12), HMQDP62R (SEQ ID NO:13), HNFDF57R (SEQ ID NO:14), and HBMTQ47R (SEQ ID NO:15). The invention also provides a nucleic acid molecule having a nucleotide sequence related to a portion of SEQ ID NO:3 which has been determined from the related cDNA clone HCQBI83RP (SEQ ID NO: 16). Furthermore, the invention provides nucleic acid molecules having nucleotide sequences related to two portions of SEQ ID NO:5 which has been determined from the related cDNA clones HMOAC87R (SEQ ID NO:17), HMSCX46R (SEQ ID NO:18), HFTAM84R (SEQ ID NO:19), and HMPAP73R (SEQ ID NO:20). The present invention also provides nucleic acid molecules having nucleotide sequences related to extensive portions of SEQ ID NO:7 which have been determined from the following related cDNA clones: HLHSM30R (SEQ ID NO:19), HAGAX06R (SEQ ID NO:20), HMSDO34R (SEQ ID NO:21), and HMSCX46R (SEQ ID NO:22). Finally, the invention provides nucleic acid molecules having nucleotide sequences related to a portion of SEQ ID NO:9 which has been determined from the related cDNA clone HNFDF57R (SEQ ID NO:24).

Further, the invention includes a polynucleotide comprising any portion of at least about 30 nucleotides, preferably at least about 50 nucleotides, of SEQ ID NO:1 from residue 340 to 400, 750 to 860, 1480 to 1552. More preferably, the invention includes a polynucleotide comprising nucleotide residues 100-1500, 250-1250, 500-1000, 600-800,250-1500, 500-1500, 750-1500, 1000-1500, 1250-1500, 100-1250, 100-1000, 100-750, 100-500, I-250, I-650, 100-500, 200-400, 300-500, 200-400, 1320-1552, and 1400-1500.

Further, the invention includes a polynucleotide comprising any portion of at least about 30 nucleotides, preferably at least about 50 nucleotides, of SEQ ID NO:3 from residue 1 to residue 1070. More preferably, the invention includes a polynucleotide comprising nucleotide residues 100-1070, 250-1070, 500-1070, 750-1070, 100-750, 100-500, 100-250, 250-750, 250-500, 500-750, 1-1100, 500-1100, 1000-1100, 1-1200, 500-1200, 1000-1200, 1-1300, 500-1300, 1000-1300, 1-1400, 500-1400, and 1000-1400.

Further, the invention includes a polynucleotide comprising any portion of at least about 30 nucleotides, preferably at least about 50 nucleotides, of SEQ ID NO:5 from residue 1-650 and from 1350-1650. More preferably, the invention includes a polynucleotide comprising nucleotide residues 100-1900, 250-1900, 500-1900, 750-1900, 1000-1900, 1250-1900, 1500-1900,100-1600,250-1600, 500-1600, 750-1600,1000-1600,1250-1600, 100-1250, 250-1250, 500-1250, 750-1250, 1000-1250, 100-1000, 250-1000, 500-1000, 750-1000, 100-750, 250-750, 500-750, 100-500, and 250-500.

Further, the invention includes a polynucleotide comprising any portion of at least about 30 nucleotides, preferably at least about 50 nucleotides, of SEQ ID NO:7 from residue 1 to residue 1000. More preferably, the invention includes a polynucleotide comprising nucleotide residues 100-1400, 250-1400, 500-1400, 750-1400, 1000-1400. 100-1000, 250-1000, 500-1000, 750-1000. 100-750, 250-750, 500-750, 100-500, 250-500,. and 100-250.

Further, the invention includes a polynucleotide comprising any portion of at least about 30 nucleotides, preferably at least about 50 nucleotides, of SEQ ID NO:9 from residue 1 to residue 650. More preferably, the invention includes a polynucleotide comprising nucleotide residues 100-1650, 250-1650, 500-1650, 750-1650, 1000-1650, 1250-1650, 250-1000, 500-1000, 750-1000, 100-750, 250-750, 500-750, 100-500, 250-500, and 1-250.

More generally, by a fragment of an isolated nucleic acid molecule having the nucleotide sequence of the deposited cDNAs or the nucleotide sequences shown in Figures 1A, 2A, 3A, 4A, and 5A (SEQ ID NO:1, SEQ ID NO:3, SEQ ID NO:5, SEQ ID NO:7, and SEQ ID NO:9, respectively) is intended fragments at least about 15 nt, and more preferably at least about 20 nt, still more preferably at least about 30 nt, and even more preferably, at least about 40 nt in length which are useful as diagnostic probes and primers as discussed herein. Of course, larger fragments 50-300 nt in length are also useful according to the present invention as are fragments corresponding to most, if not all, of the nucleotide sequence of the deposited cDNAs or as shown in Figures 1A, 2A, 3A, 4A, and 5A (SEQ ID NO:1, SEQ ID NO:3, SEQ ID NO:5, SEQ ID NO:7, and SEQ ID NO:9, respectively). By a fragment at least 20 nt in length, for example, is intended fragments which include 20 or more contiguous bases from the nucleotide sequence of the deposited cDNAs or the nucleotide sequences as shown in Figures 1A, 2A, 3A, 4A, and 5A (SEQ ID NO:1, SEQ ID NO:3, SEQ ID NO:5, SEQ ID NO:7, and SEQ ID NO:9, respectively). Preferred nucleic acid fragments of the present invention include nucleic acid molecules encoding epitope-bearing portions of the FcR-I, FcR-II, FcR-III, FcR-IV, and FcR-V polypeptide as identified in Figures 1C, 2C, 3C, 4C, and 5C, and described in more detail below.

In another aspect, the invention provides an isolated nucleic acid molecule comprising a polynucleotide which hybridizes under stringent hybridization conditions to a portion of the polynucleotide in a nucleic acid molecule of the invention described above, for instance, the cDNA clone contained in ATCC Deposit No. 97891. By "stringent hybridization conditions" is intended overnight incubation at 42° C in a solution comprising: 50% formamide. 5x SSC (150 mM NaCI, 15 mM trisodium citrate), 50 mM sodium phosphate (pH 7.6), 5x Denhardt's solution, 10% dextran sulfate, and 20 µg/ml denatured, sheared salmon sperm DNA. followed by washing the filters in 0.1 x SSC at about 65° C.

By a polynucleotide which hybridizes to a "portion" of a polynucleotide is intended a polynucleotide (either DNA or RNA) hybridizing to at least about 15 nucleotides (nt), and more preferably at least about 20 nt, still more preferably at least about 30 nt, and even more preferably about 30-70 (e.g., 50) nt of the reference polynucleotide. These are useful as diagnostic probes and primers as discussed above and in more detail below.

By a portion of a polynucleotide of "at least 20 nt in length," for example, is intended 20 or more contiguous nucleotides from the nucleotide sequence of the reference polynucleotides (e.g., the deposited cDNAs or the nucleotide sequences as shown in Figures 1A, 2A, 3A, 4A, and 5A (SEQ ID NO:1, SEQ ID NO:3, SEQ ID NO:5, SEQ ID NO:7, and SEQ ID NO:9, respectively)). Of course, a polynucleotide which hybridizes only to a poly A sequence (such as the 3' terminal poly(A) tract of the FcR-I, FcR-II, FcR-III, FcR-IV, and FcR-V cDNAs shown in Figures 1A, 2A, 3A, 4A, and 5A (SEQ ID NO:1, SEQ ID NO:3, SEQ ID NO:5, SEQ ID NO:7, and SEQ ID NO:9, respectively)), or to a complementary stretch of T (or U) residues, would not be included in a polynucleotide of the invention used to hybridize to a portion of a nucleic acid of the invention, since such a polynucleotide would hybridize to any nucleic acid molecule containing a poly (A) stretch or the complement thereof (e.g., practically any double-stranded cDNA clone).

As indicated, nucleic acid molecules of the present invention which encode an FcR-I, FcR-II, FcR-III, FcR-IV, or FcR-V polypeptides may include, but are not limited to those encoding the amino acid sequence of the mature polypeptides, by themselves; and the coding sequences for the mature polypeptides and additional sequences, such as those encoding the about 21, 18, 16, 16, and 16 amino acid leader or secretory sequence, such as a pre-, or pro- or prepro- protein sequence: the coding sequence of the mature polypeptide, with or without the aforementioned additional coding sequences.

Also encoded by nucleic acids of the invention are the above protein sequences together with additional, non-coding sequences, including for example, but not limited to introns and non-coding 5' and 3' sequences, such as the transcribed, non-translated sequences that play a role in transcription, mRNA processing, including splicing and polyadenylation signals, for example - ribosome binding and stability of mRNA; an additional coding sequence which codes for additional amino acids, such as those which provide additional functionalities.

Thus, the sequence encoding the polypeptide may be fused to a marker sequence, such as a sequence encoding a peptide which facilitates purification of the fused polypeptide. In certain preferred embodiments of this aspect of the invention, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described by Gentz and colleagues (Proc. Natl. Acad Sci. LSA 86:821-824; 1989), for instance, hexa-histidine provides for convenient purification of the fusion protein. The "HA-tag" is another peptide useful for purification which corresponds to an epitope derived from the influenza hemagglutinin protein (Wilson et al., (1984) Cell 37:767). As discussed below, other such fusion proteins include the FcR-1. FcR-II. FcR-111, FcR-IV, and FcR-V polypeptides fused to Fc at the N- or C-terminus.

### Variant and Mutant Polynucleotides

The present invention further relates to variants of the nucleic acid molecules of the present invention, which encode portions, analogs or derivatives of the FcR-I, FcR-II, FcR-III, FcR-IV, and FcR-V proteins. Variants may occur naturally, such as a natural allelic variant. By an "allelic variant" is intended one of several alternate forms of a gene occupying a given locus on a chromosome of an organism (Genes II, Lewin, B., ed.. John Wiley & Sons, New York; 1985). Non-naturally occurring variants may be produced using art-known mutagenesis techniques.

Such variants include those produced by nucleotide substitutions, deletions or additions. The substitutions, deletions or additions may involve one or more nucleotides. The variants may be altered in coding regions, non-coding regions, or both. Alterations in the coding regions may produce conservative or non-conservative amino acid substitutions, deletions or additions. Especially preferred among these are silent substitutions, additions and deletions, which do not alter the properties and activities of the FcR-I, FcR-II, FcR-III, FcR-IV, and FcR-V proteins or portions thereof. Also especially preferred in this regard are conservative substitutions.

Most highly preferred are nucleic acid molecules encoding the mature protein having the amino acid sequence shown in SEQ ID NO:2. SEQ ID NO:4, SEQ ID NO:6, SEQ ID NO:8, and SEQ ID NO:10, or the mature FcR-I, FcR-II, FcR-III, FcR-IV, and FcR-V amino acid sequences encoded by a deposited cDNA clone.

Most highly preferred are nucleic acid molecules encoding the extracellular domain of the proteins having the amino acid sequence shown in SEQ ID NO:2, SEQ ID NO:4, SEQ ID NO:6, SEQ ID NO:8, or SEQ ID NO:10 or the extracellular domain of the FcR-I, FcR-II, FcR-III, FcR-IV, or FcR-V amino acid sequences encoded by a deposited cDNA clone.

Thus, one aspect of the invention provides an isolated nucleic acid molecule comprising a polynucleotide having a nucleotide sequence at least 95% identical to a sequence selected from the group consisting of: (a) a nucleotide sequence encoding the FcR-I polypeptide having the amino acid sequence at positions -21 to 406 of SEQ ID NO:2 or the complete amino acid sequence encoded by the FcR-I cDNA clone contained in ATCC Deposit No. 97891; (b) a nucleotide sequence encoding the FcR-II polypeptide having the amino acid sequence at positions -18 to 245 of SEQ ID NO:4 or the complete amino acid sequence encoded by the FcR-II cDNA clone contained in ATCC Deposit No. 97891; (c) a nucleotide sequence encoding the FcR-III polypeptide having the amino acid sequence at positions -16 to 607 of SEQ ID NO:6 or the complete amino acid sequence encoded by the FcR-III cDNA clone contained in ATCC Deposit No. 97891; (d) a nucleotide sequence encoding the FcR-IV polypeptide having the amino acid sequence at positions -16 to 456 of SEQ ID NO:8 or the complete amino acid sequence encoded by the FcR-IV cDNA clone contained in ATCC Deposit No. 97891; (e) a nucleotide sequence encoding the FcR-V polypeptide having the amino acid sequence at positions -16 to 498 of SEQ ID NO:10 or the complete amino acid sequence encoded by the FcR-V cDNA clone contained in ATCC Deposit No. 209100; (f) a nucleotide sequence encoding the FcR-I polypeptide having the amino acid sequence at positions -20 to 406 of SEQ ID NO:2 or the complete amino acid sequence excepting the N-terminal methionine encoded by the FcR-I cDNA clone contained in ATCC Deposit No. 97891; (g) a nucleotide sequence encoding the FcR-II polypeptide having the amino acid sequence at positions -17 to 245 of SEQ ID NO:4 or the complete amino acid sequence excepting the N-terminal methionine encoded by the FcR-II cDNA clone contained in ATCC Deposit No. 97891; (h) a nucleotide sequence encoding the FcR-III polypeptide having the amino acid sequence at positions -15 to 607 of SEQ ID NO:6 or the complete amino acid sequence excepting the N-terminal methionine encoded by the FcR-III cDNA clone contained in ATCC Deposit No. 97891; (i) a nucleotide sequence encoding the FcR-IV polypeptide having the amino acid sequence at positions -15 to 456 of SEQ ID NO:8 or the complete amino acid sequence excepting the N-terminal methionine encoded by the FcR-IV cDNA clone contained in ATCC Deposit No. 97891; (j) a nucleotide sequence encoding the FcR-V polypeptide having the amino acid sequence at positions -15 to 498 of SEQ ID NO:10 or the complete amino acid sequence excepting the N-terminal methionine encoded by the FcR-V cDNA clone contained in ATCC Deposit No. 209100; (k) a nucleotide sequence encoding the mature form of the FcR-I polypeptide having the amino acid sequence at positions 1 to 406 in SEQ ID NO:2, or as encoded by the FcR-I cDNA clone contained in ATCC Deposit No. 97891; (L) a nucleotide sequence encoding the mature form of the FcR-II polypeptide having the amino acid sequence at positions 1 to 245 in SEQ ID NO:4. or as encoded by the FcR-II cDNA clone contained in ATCC Deposit No. 97891; (m) a nucleotide sequence encoding the mature form of the FcR-III polypeptide having the amino acid sequence at positions 1 to 607 in SEQ ID NO:6, or as encoded by the FcR-III cDNA clone contained in ATCC Deposit No. 97891; (n) a nucleotide sequence encoding the mature form of the FcR-IV polypeptide having the amino acid sequence at positions 1 to 456 in SEQ ID NO:8, or as encoded by the FcR-IV cDNA clone contained in ATCC Deposit No. 97891; (o) a nucleotide sequence encoding the mature form of the FcR-V polypeptide having the amino acid sequence at positions 1 to 498 in SEQ ID NO:10, or as encoded by the FcR-V cDNA clone contained in ATCC Deposit No. 209100; (p) a nucleotide sequence encoding a polypeptide comprising the extracellular domain of the FcR-I polypeptide having the amino acid sequence at positions 1 to 289 in SEQ ID NO:2 or as encoded by the FcR-I cDNA clone contained in ATCC Deposit No. 97891; (q) a nucleotide sequence encoding a polypeptide comprising the extracellular domain of the FcR-II polypeptide having the amino acid sequence at positions 1 to 211 in SEQ ID NO:4 or as encoded by the FcR-II cDNA clone contained in ATCC Deposit No. 97891; (r) a nucleotide sequence encoding a polypeptide comprising the extracellular domain of the FcR-III polypeptide having the amino acid sequence at positions 1 to 421 in SEQ ID NO:6 or as encoded by the FcR-III cDNA clone contained in ATCC Deposit No. 97891; (s) a nucleotide sequence encoding a polypeptide comprising the extracellular domain of the FcR-IV polypeptide having the amino acid sequence at positions 1 to 243 in SEQ ID NO: 8 or as encoded by the FcR-IV cDNA clone contained in ATCC Deposit No. 97891; (t) a nucleotide sequence encoding a polypeptide comprising the extracellular domain of the FcR-V polypeptide having the amino acid sequence at positions 1 to 343 in SEQ ID NO:10 or as encoded by the FcR-V cDNA clone contained in ATCC Deposit No. 209100; (u) a nucleotide sequence encoding a polypeptide comprising the transmembrane domain of the FcR-I polypeptide having the amino acid sequence at positions 290 to 312 in SEQ ID NO:2 or as encoded by the FcR-I cDNA clone contained in ATCC Deposit No. 97891; (v) a nucleotide sequence encoding a polypeptide comprising the transmembrane domain of the FcR-II polypeptide having the amino acid sequence at positions 212 to 229 in SEQ ID NO:4 or as encoded by the FcR-II cDNA clone contained in ATCC Deposit No. 97891; (w) a nucleotide sequence encoding a polypeptide comprising the transmembrane domain of the FcR-III polypeptide having the amino acid sequence at positions 422 to 448 in SEQ ID NO:6 or as encoded by the FcR-III cDNA clone contained in ATCC Deposit No. 97891; (x) a nucleotide sequence encoding a polypeptide comprising the transmembrane domain of the FcR-IV polypeptide having the amino acid sequence at positions 244 to 264 in SEQ ID NO:8 or as encoded by the FcR-IV cDNA clone contained in ATCC Deposit No. 97891; (y) a nucleotide sequence encoding a polypeptide comprising the transmembrane domain of the FcR-V polypeptide having the amino acid sequence at positions 344 to 364 in SEQ ID NO:10 or as encoded by the FcR-V cDNA clone contained in ATCC Deposit No. 209100; (z) a nucleotide sequence encoding a polypeptide comprising the intracellular domain of the FcR-I polypeptide having the amino acid sequence at positions 313 to 406 in SEQ ID NO:2 or as encoded by the FcR-I cDNA clone contained in ATCC Deposit No. 97891; (aa) a nucleotide sequence encoding a polypeptide comprising the intracellular domain of the FcR-II polypeptide having the amino acid sequence at positions 230 to 245 in SEQ ID NO:4 or as encoded by the FcR-II cDNA clone contained in ATCC Deposit No. 97891; (ab) a nucleotide sequence encoding a polypeptide comprising the intracellular domain of the FcR-III polypeptide having the amino acid sequence at positions 449 to 607 in SEQ ID NO:6 or as encoded by the FcR-111 cDNA clone contained in ATCC Deposit No. 97891; (ac) a nucleotide sequence encoding a polypeptide comprising the intracellular domain of the FcR-IV polypeptide having the amino acid sequence at positions 265 to 456 in SEQ ID NO:8 or as encoded by the FcR-IV cDNA clone contained in ATCC Deposit No. 97891; (ad) a nucleotide sequence encoding a polypeptide comprising the intracellular domain of the FcR-V polypeptide having the amino acid sequence at positions 365 to 498 in SEQ ID NO:10 or as encoded by the FcR-V cDNA clone contained in ATCC Deposit No. 97891; (ae) a nucleotide sequence encoding a soluble FcR-I polypeptide having the extracellular and intracellular domains but lacking the transmembrane domain; (af) a nucleotide sequence encoding a soluble FcR-II polypeptide having the extracellular and intracellular domains but lacking the transmembrane domain; (ag) a nucleotide sequence encoding a soluble FcR-III polypeptide having the extracellular and intracellular domains but lacking the transmembrane domain; (ah) a nucleotide sequence encoding a soluble FcR-IV polypeptide having the extracellular and intracellular domains but lacking the transmembrane domain; (ai) a nucleotide sequence encoding a soluble FcR-V polypeptide having the extracellular and intracellular domains but lacking the transmembrane domain: and; (aj) a nucleotide sequence complementary to any of the nucleotide sequences in (a) through (ai) above.

Further embodiments of the invention include isolated nucleic acid molecules that comprise a polynucleotide having a nucleotide sequence at least 90% identical, and more preferably at least 95%, 96%, 97%, 98% or 99% identical, to any of the nucleotide sequences in (a) through (aj) above, or a polynucleotide which hybridizes under stringent hybridization conditions to a polynucleotide in (a) through (aj) above. This polynucleotide which hybridizes does not hybridize under stringent hybridization conditions to a polynucleotide having a nucleotide sequence consisting of only A residues or of only T residues. An additional nucleic acid embodiment of the invention relates to an isolated nucleic acid molecule comprising a polynucleotide which encodes the amino acid sequence of an epitope-bearing portion of a FcR-I, FcR-II, FcR-III, FcR-IV, or FcR-V polypeptide having an amino acid sequence in (a) through (ai) above.

The present invention also relates to recombinant vectors, which include the isolated nucleic acid molecules of the present invention, and to host cells containing the recombinant vectors, as well as to methods of making such vectors and host cells and for using them for production of an FcR-I, FcR-II, FcR-III, FcR-IV, or FcR-V polypeptides or peptides by recombinant techniques.

By a polynucleotide having a nucleotide sequence at least, for example, 95% "identical" to a reference nucleotide sequence encoding a FcR-I, FcR-II, FcR-III, FcR-IV, or FcR-V polypeptide is intended that the nucleotide sequence of the polynucleotide is identical to the reference sequence except that the polynucleotide sequence may include up to five point mutations per each 100 nucleotides of the reference nucleotide sequences encoding the FcR-I. FcR-II, FcR-III, FcR-IV, or FcR-V polypeptide. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5' or 3' terminal positions of the reference nucleotide sequence or anywhere between those terminal positions, interspersed either individually among nucleotides in the reference sequence or in one or more contiguous groups within the reference sequence.

As a practical matter, whether any particular nucleic acid molecule is at least 90%, 95%, 96%, 97%, 98% or 99% identical to, for instance, the nucleotide sequences shown in Figures 1A, 2A, 3A, 4A, or 5A or to the nucleotides sequence of the deposited cDNA clones can be determined conventionally using known computer programs such as the Bestfit program (Wisconsin Sequence Analysis Package, Version 8 for Unix, Genetics Computer Group, University Research Park, 575 Science Drive, Madison. WI 53711). Bestfit uses the local homology algorithm of Smith and Waterman (Advances in Applied Mathematics 2:482-489: 1981), to find the best segment of homology between two sequences. When using Bestfit or any other sequence alignment program to determine whether a particular sequence is, for instance, 95% identical to a reference sequence according to the present invention, the parameters are set, of course, such that the percentage of identity is calculated over the full length of the reference nucleotide sequence and that gaps in homology of up to 5% of the total number of nucleotides in the reference sequence are allowed.

The present application is directed to nucleic acid molecules at least 90%, 95%, 96%. 97%, 98% or 99% identical to the nucleic acid sequences shown in Figures 1A, 2A, 3A, 4A. or 5A (SEQ ID NO:1, SEQ ID NO:3, SEQ ID NO:5, SEQ ID NO:7, or SEQ ID NO:9, respectively), or to the nucleic acid sequence of the deposited cDNAs, irrespective of whether they encode a polypeptide having FcR-I, FcR-II, FcR-III, FcR-IV, or FcR-V activity. This is because even where a particular nucleic acid molecule does not encode a polypeptide having FcR-I, FcR-II, FcR-III, or FcR-IV activity, one of skill in the art would still know how to use the nucleic acid molecule, for instance, as a hybridization probe or a polymerase chain reaction (PCR) primer. Uses of the nucleic acid molecules of the present invention that do not encode a polypeptide having FcR-I, FcR-11, FcR-III, FcR-IV, or FcR-V activity include, inter alia, (1) isolating the FcR-I, FcR-II, FcR-III, or FcR-IV gene or allelic variants thereof in a cDNA library; (2) *in situ* hybridization (e.g., "FISH") to metaphase chromosomal spreads to provide a precise chromosomal location of the FcR-I, FcR-II, FcR-III, FcR-IV, or FcR-V gene (Verma, et al., (1988) Human Chromosomes: A Manual of Basic Techniques, Pergamon Press, New York); and Northern Blot analysis for detecting FcR-I, FcR-II, FcR-III, FcR-IV, or FcR-V mRNA expression in specific tissues.

Preferred, however, are nucleic acid molecules having sequences at least 90%. 95%, 96%, 97%, 98% or 99% identical to the nucleic acid sequences shown in Figures 1A, 2A. 3A, 4A, and 5A (SEQ ID NO:1, SEQ ID NO:3, SEQ ID NO:5, SEQ ID NO:7, and SEQ ID NO:9, respectively) or to the nucleic acid sequences of the deposited cDNAs which do, in fact, encode polypeptides having FcR-I, FcR-II, FcR-III, FcR-IV, or FcR-V protein activity, respectively. By "a polypeptide having FcR-I, FcR-II, FcR-III, FcR-IV, or FcR-V activity" is intended polypeptides exhibiting activity similar, but not necessarily identical, to an activity of the mature FcR-I, FcR-II, FcR-III, FcR-IV, or FcR-V protein of the invention, as measured in a particular biological assay. For example, the FcR-I, FcR-II, FcR-III, FcR-IV, or FcR-V protein of the present invention mediate phagocytosis of sheep red blood cells (SRBCs) opsonized with IgG2a (Takai. T., et al. (1994) Cell 76:519). Briefly, the assay involves incubation of cells which express FcR-I, FcR-II, FcR-III, FcR-IV, or FcR-V protein, either naturally or by transient or stable transfection, with IgG-coated SRBCs and visually inspecting the degree of internalization of the SRBCs. Such an activity is useful for the initial steps in the clearance of antibody-coated or antibody-associated molecules or cells from the immune system.

FcR-I, FcR-II, FcR-III, FcR-IV, or FcR-V proteins mediate phagocytosis in a dose-dependent manner in the above-described assay. Thus, "a polypeptide having FcR-I, FcR-II, FcR-III, FcR-IV, or FcR-V protein activity" includes polypeptides that also exhibit any of the same phagocytosis-mediating activities in the above-described assays in a dose-dependent manner. Although the degree of dose-dependent activity need not be identical to that of the FcR-I, FcR-II, FcR-III, FcR-IV, or FcR-V protein, preferably, "a polypeptide having FcR-I, FcR-II, FcR-III, FcR-IV, or FcR-V protein activity" will exhibit substantially similar dose-dependence in a given activity as compared to the FcR-I, FcR-II, FcR-III, FcR-IV, or FcR-V protein (i.e., the candidate polypeptide will exhibit greater activity or not more than about 25-fold less and, preferably, not more than about tenfold less activity relative to the reference FcR-I, FcR-II, FcR-III, FcR-IV, or FcR-V protein).

In addition to the assay described above, which is designed to determine the degree to which a molecule can participate in mediating phagocytosis, the direct interaction between FcR-I, FcR-II, FcR-III, FcR-IV, and FcR-V proteins and IgG can also be measured. As described by Sears and colleagues (J. Immunol. 144:371-378; 1990), a Scatchard analysis can be utilized to determine the degree to which IgG, or any other protein, binds to FcR-I, FcR-II, FcR-III, FcR-IV, and FcR-V proteins. In brief, ¹²⁵I-labeled IgG, or any other protein of interest, is mixed with cells which express, or do not express (as a control), FcR-I, FcR-II, FcR-III, FcR-IV, and FcR-V protein. The mixtures are incubated at 0°C for 60 minutes with occasional aggitation. Bound and unbound fractions are separated by centrifugation through a mixture of dibutyl phthalate and bis-(2-ethylhexyl) phthalate (3:2 by volume) oils in an Eppendorf centrifuge for 2 minutes. The radioactivity of each fraction is then measured, nonspecific binding is determined by using identical experimental conditions in the presence of >100-fold unlabeled IgG2a, or other protein of interest. The data are then presented, in a Scatchard analysis of (CPM bound/CPM free) versus (CPM bound). One of skill in the art would recognize that an analysis such as this may be an important, and often distinguishing, characteristic of the present invention of of any FcR.

Of course, due to the degeneracy of the genetic code, one of ordinary skill in the art will immediately recognize that a large number of the nucleic acid molecules having a sequence at least 90%, 95%, 96%, 97%, 98%, or 99% identical to the nucleic acid sequence of the deposited cDNAs or the nucleic acid sequences shown in Figures 1A, 2A, 3A, 4A, and 5A (SEQ ID NO:1, SEQ ID NO:3, SEQ ID NO:5, SEQ ID NO:7, and SEQ ID NO:9, respectively) will encode a polypeptide "having FcR-I, FcR-II, FcR-III, FcR-IV, or FcR-V protein activity." In fact, since degenerate variants of these nucleotide sequences all encode the same polypeptide, this will be clear to the skilled artisan even without performing the above described comparison assay. It will be further recognized in the art that, for such nucleic acid molecules that are not degenerate variants, a reasonable number will also encode a polypeptide having FcR-I, FcR-II, FcR-III, FcR-IV, or FcR- V protein activity. This is because the skilled artisan is fully aware of amino acid substitutions that are either less likely or not likely to significantly effect protein function (e.g., replacing one aliphatic amino acid with a second aliphatic amino acid), as further described below.

### Vectors and Host Cells

The present invention also relates to vectors which include the isolated DNA molecules of the present invention, host cells which are genetically engineered with the recombinant vectors, and the production of FcR-I, FcR-II, FcR-III, FcR-IV, and FcR-V polypeptides or fragments thereof by recombinant techniques. The vector may be, for example, a phage, plasmid, viral or retroviral vector. Retroviral vectors may be replication competent or replication defective. In the latter case, viral propagation generally will occur only in complementing host cells.

The polynucleotides may be joined to a vector containing a selectable marker for propagation in a host. Generally, a plasmid vector is introduced in a precipitate, such as a calcium phosphate precipitate, or in a complex with a charged lipid. If the vector is a virus, it may be packaged in vitro using an appropriate packaging cell line and then transduced into host cells.

The DNA insert should be operatively linked to an appropriate promoter, such as the phage lambda PL promoter, the *E. coli lac, trp, phoA* and *tac* promoters, the SV40 early and late promoters and promoters of retroviral LTRs, to name a few. Other suitable promoters will be known to the skilled artisan. The expression constructs will further contain sites for transcription initiation, termination and, in the transcribed region, a ribosome binding site for translation. The coding portion of the transcripts expressed by the constructs will preferably include a translation initiating codon at the beginning and a termination codon (UAA, UGA or UAG) appropriately positioned at the end of the polypeptide to be translated.

As indicated, the expression vectors will preferably include at least one selectable marker. Such markers include dihydrofolate reductase, G418 or neomycin resistance for eukaryotic cell culture and tetracycline, kanamycin or ampicillin resistance genes for culturing in *E. coli* and other bacteria. Representative examples of appropriate hosts include, but are not limited to, bacterial cells, such as *E. coli,* Streptomyces and *Salmonella typhimurium* cells; fungal cells, such as yeast cells; insect cells such as Drosophila S2 and Spodoptera Sf9 cells; animal cells such as CHO, COS, 293 and Bowes melanoma cells; and plant cells. Appropriate culture mediums and conditions for the above-described host cells are known in the art.

Among vectors preferred for use in bacteria include pQE70, pQE60 and pQE-9, available from QIAGEN, Inc., *supra*; pBS vectors, Phagescript vectors, Bluescript vectors, pNH8A, pNH16a, pNH18A, pNH46A, available from Stratagene; and ptrc99a, pKK223-3, pKK233-3, pDR540, pRIT5 available from Pharmacia. Among preferred eukaryotic vectors are pWLNEO, pSV2CAT, pOG44, pXT1 and pSG available from Stratagene: and pSVK3, pBPV, pMSG and pSVL available from Pharmacia. Other suitable vectors will be readily apparent to the skilled artisan.

Introduction of the construct into the host cell can be effected by calcium phosphate transfection, DEAE-dextran mediated transfection, cationic lipid-mediated transfection, electroporation, transduction, infection or other methods. Such methods are described in many standard laboratory manuals (e.g. Davis. et al., (1986) Basic Methods In Molecular Biology).

The polypeptide may be expressed in a modified form, such as a fusion protein, and may include not only secretion signals, but also additional heterologous functional regions. For instance, a region of additional amino acids, particularly charged amino acids, may be added to the N-terminus of the polypeptide to improve stability and persistence in the host cell, during purification, or during subsequent handling and storage. Also, peptide moieties may be added to the polypeptide to facilitate purification. Such regions may be removed prior to final preparation of the polypeptide. The addition of peptide moieties to polypeptides to engender secretion or excretion, to improve stability and to facilitate purification, among others, are familiar and routine techniques in the art. A preferred fusion protein comprises a heterologous region from immunoglobulin that is useful to stabilize and purify proteins. For example, EP-A-O 464 533 (Canadian counterpart 2045869) discloses fusion proteins comprising various portions of constant region of immunoglobulin molecules together with another human protein or part thereof. In many cases, the Fc part in a fusion protein is thoroughly advantageous for use in therapy and diagnosis and thus results, for example, in improved pharmacokinetic properties (EP-A 0232 262). On the other hand, for some uses it would be desirable to be able to delete the Fc part after the fusion protein has been expressed, detected and purified in the advantageous manner described. This is the case when Fc portion proves to be a hindrance to use in therapy and diagnosis, for example when the fusion protein is to be used as antigen for immunizations. In drug discovery, for example, human proteins, such as hIL-5. have been fused with Fc portions for the purpose of high-throughput screening assays to identify antagonists of hIL-5 (Bennett, D., et al.. J. Molecular Recognition 8:52-58; 1995 and Johanson. K., et al., J. Biol. Chem. 270:9459-9471; 1995).
□□□ FcR-I, FcR-II, FcR-III, FcR-IV, and FcR-V proteins can be recovered and purified from recombinant cell cultures by well-known methods including ammonium sulfate or ethanol precipitation, acid extraction, anion or cation exchange chromatography, phosphocellulose chromatography, hydrophobic interaction chromatography; affinity chromatography, hydroxylapatite chromatography and lectin chromatography. Most preferably, high performance liquid chromatography ("HPLC") is employed for purification. Polypeptides of the present invention include: products purified from natural sources, including bodily fluids, tissues and cells, whether directly isolated or cultured; products of chemical synthetic procedures; and products produced by recombinant techniques from a prokaryotic or eukaryotic host, including, for example, bacterial, yeast, higher plant, insect and mammalian cells. Depending upon the host employed in a recombinant production procedure, the polypeptides of the present invention may be glycosylated or may be non-glycosylated. In addition, polypeptides of the invention may also include an initial modified methionine residue, in some cases as a result of host-mediated processes. Thus, it is well know in the art that the N-terminal methionine encoded by the translation initiation codon generally is removed with high efficiency from any protein after translation in all eukaryotic cells. While the N-terminal methionine on most proteins also is efficiently removed in most prokaryotes, for some proteins this prokaryotic removal process is inefficient, depending on the nature of the amino acid to which the N-terminal methionine is covalently linked.

### Polypeptides and Fragments

The invention further provides isolated FcR-I, FcR-II, FcR-III, FcR-IV, and FcR-V polypeptides having the amino acid sequences encoded by the deposited cDNAs, or the amino acid sequences in SEQ ID NO:2, SEQ ID NO:4, SEQ ID NO:6, SEQ ID NO:8, and SEQ ID NO:10, respectively, or a peptide or polypeptide comprising a portion of the above polypeptides.

### Variant and Mutant Polypeptides

To improve or alter the characteristics of FcR-I, FcR-II, FcR-III, FcR-IV, and FcR-V polypeptides, protein engineering may be employed. Recombinant DNA technology known to those skilled in the art can be used to create novel mutant proteins or "muteins" including single or multiple amino acid substitutions, deletions, additions or fusion proteins. Such modified polypeptides can show, e.g., enhanced activity or increased stability. In addition, they may be purified in higher yields and show better solubility than the corresponding natural polypeptide, at least under certain purification and storage conditions.

### N-Terminal and C-Terminal Deletion Mutants

For instance, for many proteins, including the extracellular domain of a membrane associated protein or the mature form(s) of a secreted protein, it is known in the art that one or more amino acids may be deleted from the N-terminus or C-terminus without substantial loss of biological function. For instance, Ron and colleagues (J. Biol. Chem., 268:2984-298.8 (1993)) reported modified KGF proteins that had heparin binding activity even if 3, 8, or 27 amino-terminal amino acid residues were missing. In the present case, since the proteins of the invention are members of the Fc receptor polypeptide family, deletions of N-terminal amino acids up to the glycine residue located approximately 7 residues in the N-terminal direction from the first cysteine residue in the mature polypeptide sequence. This glycine residue signals the beginning of the β-turn in the predicted structure of the first Ig-like repeat in the extracellular domain of the FcR. The glycine which initiates the β-turn is located at positions 9, 28, 26, 26, and 26 of SEQ ID NO:2, SEQ ID NO:4, SEQ ID NO:6, SEQ ID NO:8, and SEQ ID NO:10, respectively. An FcR-I, FcR-II, FcR-III, FcR-IV. or FcR-V variant which contains an N-terminal deletion of sequences up to the above-described glycine residue may retain some biological activity such as the ability to bind to IgG. In addition, in the case of FcR-I and FcR-III, it may be possible to generate an N-terminal deletion variant in which the entire N-terminal-most Ig-like domain has been deleted and at least some of the biological activity of IgG binding is retained. In fact, Porges and colleagues (J. Clin. Invest. 90:2102-2109; 1992) and Hogarth and coworkers (Immunol. Res: 11:217-225; 1992) have characterized similar such mutations in the related FcR proteins FcγRI and FcγRII. In addition, polypeptides having deletions of up to about 10 additional N-terminal residues (i.e., up to the serine, proline, serine, tryptophan, and serine at positions 19, 38, 36, 36, and 36 in SEQ ID NO:2, SEQ ID NO:4, SEQ ID NO:6, SEQ ID NO:8, and SEQ ID NO:10, respectively, may retain some biological activity such as limited receptor binding or modulation of target cell activities. Polypeptides having further N-terminal deletions including the above-described, most-N-terminal, β-turn-glycine residues in SEQ ID NO:2, SEQ ID NO:4, SEQ ID NO:6, SEQ ID NO:8, and SEQ ID NO:10 would not be expected to retain such biological activities because it is known that this residue in many receptor-like proteins which contain extracelluar Ig-binding-like domains is required for correct tertiary structure of the Ig-binding-like domain and, in turn, for interaction with Ig molecules and the consequent biological response of such interaction.

However, even if deletion of one or more amino acids from the N-terminus of a protein results in modification of loss of one or more biological functions of the protein, other biological activities may still be retained. Thus, the ability of the shortened protein to induce and/or bind to antibodies which recognize the complete or mature form of the protein generally will be retained when less than the majority of the residues of the complete or mature protein are removed from the N-terminus. Whether a particular polypeptide lacking N-terminal residues of a complete protein retains such immunologic activities can readily be determined by routine methods described herein and otherwise known in the art.

Accordingly, the present invention further provides polypeptides having one or more residues deleted from the amino terminus of the amino acid sequence of FcR-I, FcR-II, FcR-III, FcR-IV, and FcR-V shown in SEQ ID NO:2, SEQ ID NO:4, SEQ ID NO:6, SEQ ID NO:8, and SEQ ID NO:10, respectively, up to the glycine residue at position number 9, 28, 26, 26, and 26 of SEQ ID NO:2, SEQ ID NO:4. SEQ ID NO:6, SEQ ID NO:8, and SEQ ID NO:10. respectively, and polynucleotides encoding such polypeptides. In particular, the present invention provides polypeptides comprising the amino acid sequence of residues n-406 of SEQ ID NO:2, where n is an integer in the range of -20 to 9 and 9 is the position of the first residue from the N-terminus of the complete FcR-I polypeptide (shown in SEQ ID NO:2) believed to be required for receptor binding activity of the FcR-I protein. In addition, the present invention also provides for polypeptides comprising the amino acid sequence of residues n-245 of SEQ ID NO:4, where n is an integer in the range of -17 to 28 and 28 is the position of the first residue from the N-terminus of the complete FcR-II polypeptide (shown in SEQ ID NO:4) believed to be required for receptor binding activity of the FcR-II protein. Also, the present invention also provides for polypeptides comprising the amino acid sequence of residues n-607 of SEQ ID NO:6, where n is an integer in the range of -15 to 26 and 26 is the position of the first residue from the N-terminus of the complete FcR-III polypeptide (shown in SEQ ID NO:6) believed to be required for receptor binding activity of the FcR-III protein. Likewise, the present invention also provides for polypeptides comprising the amino acid sequence of residues n-472 of SEQ ID NO:8, where n is an integer in the range of-15 to 26 and 26 is the position of the first residue from the N-terminus of the complete FcR-IV polypeptide (shown in SEQ ID NO:8) believed to be required for receptor binding activity of the FcR-IV protein. Finally, the present invention also provides for polypeptides comprising the amino acid sequence of residues n-498 of SEQ ID NO:8, where n is an integer in the range of -15 to 26 and 26 is the position of the first residue from the N-terminus of the complete FcR-IV polypeptide (shown in SEQ ID NO:8) believed to be required for receptor binding activity of the FcR-IV protein.

More in particular, the invention provides polynucleotides encoding polypeptides having the amino acid sequence of residues of -20 to 406, -19 to 406, -18 to 406, -17 to 406, -16 to 406, -15 to 406, -14 to 406, -13 to 406, -12 to 406, -11 to 406, -10 to 406, -9 to 406,-8 to 406, -7 to 406, -6 to 406, -5 to 406, -4 to 406, -3 to 406, -2 to 406, -1 to 406, 1 to 406, 2 to 406, 3 to 406, 4 to 406, 5 to 406, 6 to 406, 7 to 406, 8 to 406, and 9 to 406 of SEQ ID NO:2. Polynucleotides encoding these polypeptides also are provided. In addition, the invention provides polynucleotides encoding polypeptides having the amino acid sequence of residues of -17 to 245, -16 to 245, -15 to 245, -14 to 245, -13 to 245, -12 to 245, -11 to 245, -10 to 245. -9 to 245, -8 to 245, -7 to 245, -6 to 245, -5 to 245, -4 to 245, -3 to 245, -2 to 245, -1 to 245, 1 to 245, 2 to 245, 3 to 245, 4 to 245, 5 to 245, 6 to 245, 7 to 245, 8 to 245, 9 to 245, 10 to 245, 11 to 245, 12 to 245, 13 to 245, 14 to 245, 15 to 245, 16 to 245, 17 to 245, 18 to 245, 19 to 245, 20 to 245, 21 to 245, 22 to 245, 23 to 245, 24 to 245, 25 to 245, 26 to 245, 27 to 245, and 28 to 245, of SEQ ID NO:4. Polynucleotides encoding these polypeptides also are provided. Also, the invention provides polynucleotides encoding polypeptides having the amino acid sequence of residues of-15 to 607, -14 to 607, -13 -to 607, -12 to 607, -11 to 607; -10 to 607, -9 to 607, -8 to 607, -7 to 607, -6 to 607, -5 to 607, -4 to 607, -3 to 607, -2 to 607, -1 to 607, 1 to 607, 2 to 607, 3 to 607, 4 to 607, 5 to 607, 6 to 607, 7 to 607, 8 to 607, 9 to 607, 10 to 607, 11 to 607, 12 to 607, 13 to 607, 14 to 607, 15 to 607, 16 to 607, 17 to 607, 18 to 607, 19 to 607, 20 to 607, 21 to 607, 22 to 607, 23 to 607, 24 to 607, 25 to 607, and 26 to 607 of SEQ ID NO:6. Polynucleotides encoding these polypeptides also are provided. Furthermore, the invention provides polynucleotides encoding polypeptides having the amino acid sequence of residues of -15 to to 472, -14 to 472, -13 to 472, -12 to 472, -11 to 472, -10 to 472, -9 to 472, -8 to 472, -7 to 472, -6no 472, -5 to 472, -4 to 472, -3 to 472, -2 to 472, -1 to 472, 1 to 472, 2 to 472, 3 to 472, 4 to 472, 5 to 472, 6 to 472, 7 to 472, 8 to 472, 9 to 472, 10 to 472, 11 to 472, 12 to 472. 13 to 472, 14 to 472, 15 to 472, 16 to 472, 17 to 472, 18 to 472, 19 to 472, 20 to 472, 21 to 472,22 to 472, 23 to 472, 24 to 472, 25 to 472, and 26 to 472 of SEQ ID NO:8. Polynucleotides encoding these polypeptides also are provided. Furthermore, the invention provides polynucleotides encoding polypeptides having the amino acid sequence of residues of -15 to to 498, -14 to 498, -13 to 498, -12 to 498, -11 to 498, -10 to 498, -9 to 498, -8 to 498, -7 to 498, -6 to 498, -5 to 498, -4 to 498, -3 to 498, -2, to 498, -1 to 498, 1 to 498, 2 to 498, 3 to 498, 4 to 498, 5 to 498, 6 to 498, 7 to 498, 8 to 498, 9 to 498, 10 to 498, 11 to 498, 12 to 498, 13 to 498, 14 to 498, 15 to 498, 16 to 498, 17 to 498, 18 to 498, 19 to 498, 20 to 498, 21 to 498, 22 to 498, 23 to 498, 24 to 498, 25 to 498, and 26 to 498 of SEQ ID NO:10.

Similarly, many examples of biologically functional C-terminal deletion muteins are known. For instance, IFN-γ shows up to ten times higher activities by deleting 8-10 amino acid residues from the carboxy terminus of the protein (Dobeli, et al. (1988) J. Biotechnol. 7:199-216). In the present case, deletions of C-terminal amino acids up to the proline at position 396 of SEQ ID NO:2, the glutamine at postion 236 of SEQ ID NO:4, the proline at position 596 of SEQ ID NO:6, the glutamine at position 446 of SEQ ID NO:8, and the cysteine at position 488 of SEQ ID NO:10 may retain some biological activity such as mediation of ADCC, phagocytosis, and the release of inflammatory mediators such as cytokines and prostaglandins. In addition, polypeptides having deletions of up to about 10 additional C -terminal residues (i.e., up to the serine at position 386 of SEQ ID NO:2, the glycine at position 225 of SEQ ID NO:4, the arginine at position 586 of SEQ ID NO:6, the glycine at position 436 of SEQ ID NO:8, and the glutamine at position 478 of SEQ ID NO:10 may retain some biological activity such as mediation of ADCC, phagocytosis, and the release of inflammatory mediators such as cytokines and prostaglandins.

However, even if deletion of one or more amino acids from the C-terminus of a protein results in modification of loss of one or more biological functions of the protein, other biological activities may still be retained. Thus, the ability of the shortened protein to induce and/or bind to antibodies which recognize the complete or mature of the protein generally will be retained when less than the majority of the residues of the complete or mature protein are removed from the C-terminus. Whether a particular polypeptide lacking C-terminal residues of a complete protein retains such immunologic activities can readily be determined by routine methods described herein and otherwise known in the art.

Accordingly, the present invention further provides polypeptides having one or more residues from the carboxy terminus of the amino acid sequence of the FcR-I shown in SEQ ID NO:2, up to the glutamine residue at position 396 of SEQ ID NO:2, and polynucleotides encoding such polypeptides. In particular, the present invention provides polypeptides having the amino acid sequence of residues -20 to "m" of the amino acid sequence in SEQ ID NO:2, where "m" is any integer in the range of 396 to 405, and residue 396 is the position of the first residue from the C- terminus of the complete FcR-1 polypeptide (shown in SEQ ID NO:2) believed to be required for mediation of ADCC, phagocytosis, and the release of inflammatory mediators such as cytokines and prostaglandins. In addition, the present invention further provides polypeptides having one or more residues from the carboxy terminus of the amino acid sequence of the FcR-II shown in SEQ ID NO:4, up to the glutamine residue at position 236 of SEQ ID NO:4, and polynucleotides encoding such polypeptides. In particular, the present invention provides polypeptides having the amino acid sequence of residues -17 to "m" of the amino acid sequence in SEQ ID NO:4, where "m" is any integer in the range of 236 to 244, and residue 236 is the position of the first residue from the C-terminus of the complete FcR-II polypeptide (shown in SEQ ID NO:4) believed to be required for mediation of ADCC, phagocytosis, and the release of inflammatory mediators such as cytokines and prostaglandins. Also, the present invention further provides polypeptides having one or more residues from the carboxy terminus of the amino acid sequence of the FcR-III shown in SEQ ID NO:6, up to the proline residue at position 596 of SEQ ID NO:6, and polynucleotides encoding such polypeptides. In particular, the present invention provides polypeptides having the amino acid sequence of residues -15 to "m" of the amino acid sequence in. SEQ ID.N0:6, where "m" is any integer in the range of 596 to 607, and residue 596 is the position of the first residue from the C-terminus of the complete FcR-III polypeptide (shown in SEQ ID NO:6) believed to be required for mediation of ADCC, phagocytosis, and the release of inflammatory mediators such as cytokines and prostaglandins. Further, the present invention further provides polypeptides having one or more residues from the carboxy terminus of the amino acid sequence of the FcR-IV shown in SEQ ID NO:8, up to the glutamine residue at position 446 of SEQ ID NO:8, and polynucleotides encoding such polypeptides. In particular, the present invention provides polypeptides having the amino acid sequence of residues -15 to "m" of the amino acid sequence in SEQ ID NO:8, where "m" is any integer in the range of 446 to 456, and residue 446 is the position of the first residue from the C-terminus of the complete FcR-IV polypeptide (shown in SEQ ID NO:8) believed to be required for mediation of ADCC, phagocytosis, and the release of inflammatory mediators such as cytokines and prostaglandins of the FcR-IV protein. In addition, the present invention provides polypeptides having the amino acid sequence of residues -15 to "m" of the amino acid sequence in SEQ ID NO:10, where "m" is any integer in the range of 488 to 498, and residue 488 is the position of the first residue from the C-terminus of the complete FcR-V polypeptide (shown in SEQ ID NO:10) believed to be required for mediation of ADCC, phagocytosis, and the release of inflammatory mediators such as cytokines and prostaglandins of the FcR-V protein.

More in particular, the invention provides polynucleotides encoding polypeptides having the amino acid sequence of residues -20 to 396, -20 to 397, -20 to 398, -20 to 399, -20 to 400, -20 to 401, -20 to 402, -20 to 403, -20 to 404, and -20 to 405 of SEQ ID NO:2. Polynucleotides encoding these polypeptides also are provided. In addition, the invention provides polynucleotides encoding polypeptides having the amino acid sequence of residues -17 to 236, -17 to 237, -17 to 238, -17 to 239, -17 to 240, -17 to 241, -17 to 242, -17 to 243, and -17 to 244 of SEQ ID NO:4. Polynucleotides encoding these polypeptides also are provided. Also, the invention provides polynucleotides encoding polypeptides having the amino acid sequence of residues -15 to 596, -15 to 597, -15 to 598, -15 to 599, -15 to 600, -15 to 601, -15 to 602, -15 to 603, -15 to 604, -15 to 605, and -15 to 606 of SEQ ID NO:6. Polynucleotides encoding these polypeptides also are provided. Further, the invention provides polynucleotides encoding polypeptides having the amino acid sequence of residues -15 to 446, -15 to 447, -15 to 448, -15 to 449, -15 to 450, -15 to 451, -15 to 452, -15 to 453, -15 to 454, and -15 to 455 of SEQ ID N0:8. Polynucleotides encoding these polypeptides also are provided. Finally, the invention provides polynucleotides encoding polypeptides having the amino acid sequence of residues -15 to 488, -15 to 489, -15 to 490, -15 to 491, -15 to 492, -15 to 493, -15 to 494, -15 to 495, -15 to 496, and -15 to 497 of SEQ ID NO:10. Polynucleotides encoding these polypeptides also are provided.

The invention also provides polypeptides having one or more amino acids deleted from both the amino and the carboxyl termini, which may be described generally as having residues n-m of SEQ ID NO:2, SEQ ID NO:4, SEQ ID NO:6, SEQ ID NO:8, or SEQ ID NO:10, where n and m are integers as described above. Polynucleotides encoding such polypeptides are also provided.

Also included are a nucleotide sequence encoding a polypeptide consisting of a portion of the complete FcR-I amino acid sequence encoded by the cDNA clone contained in ATCC Deposit No. 97891, where this portion excludes from 1 to about 9 amino acids from the amino terminus of the complete amino acid sequence encoded by the FcR-I cDNA clone contained in ATCC Deposit No. 97891, or from I to about 10 amino acids from the carboxy terminus, or any combination of the above amino terminal and carboxy terminal deletions, of the complete amino acid sequence encoded by the FcR-I cDNA clone contained in ATCC Deposit No. 97891. In addition, a nucleotide sequence encoding a polypeptide consisting of a portion of the complete FcR-II amino acid sequence encoded by the FcR-II cDNA clone contained in ATCC Deposit No. 97891 is included, where this portion excludes from 1 to about 27 amino acids from the amino terminus of the complete amino acid sequence encoded by the FcR-II cDNA clone contained in ATCC Deposit No. 97891, or from 1 to about 10 amino acids from the carboxy terminus, or any combination of the above amino terminal and carboxy terminal deletions, of the complete amino acid sequence encoded by the FcR-II cDNA clone contained in ATCC Deposit No. 97891. Also, a nucleotide sequence encoding a polypeptide consisting of a portion of the complete FcR-III amino acid sequence encoded by the FcR-III cDNA clone contained in ATCC Deposit No. 97891 is included, where this portion excludes from 1 to about 26 amino acids from the amino terminus of the complete amino acid sequence encoded by the FcR-III cDNA clone contained in ATCC Deposit No. 97891, or from 1 to about 10 amino acids from the carboxy terminus, or any combination of the above amino terminal and carboxy terminal deletions, of the complete amino acid sequence encoded by the FcR-III cDNA clone contained in ATCC Deposit No. 97891. Further, a nucleotide sequence encoding a polypeptide consisting of a portion of the complete FcR-IV amino acid sequence encoded by the FcR-IV cDNA clone contained in ATCC Deposit No. 97891 is included, where this portion excludes from 1 to about 26 amino acids from the amino terminus of the complete amino acid sequence encoded by the cDNA clone contained in ATCC Deposit No. 97891, or from 1 to about 10 amino acids from the carboxy terminus, or any combination of the above amino terminal and carboxy terminal deletions, of the complete amino acid sequence encoded by the cDNA clone contained in ATCC Deposit No. 97891. Polynucleotides encoding all of the above deletion mutant polypeptide forms also are provided. Finally, a nucleotide sequence encoding a polypeptide consisting of a portion of the complete FcR-V amino acid sequence encoded by the FcR-V cDNA clone contained in ATCC Deposit No. 209100 is included, where this portion excludes from 1 to about 26 amino acids from the amino terminus of the complete amino acid sequence encoded by the cDNA clone contained in ATCC Deposit No. 209100, or from 1 to about 10 amino acids from the carboxy terminus, or any combination of the above amino terminal and carboxy terminal deletions, of the complete amino acid sequence encoded by the cDNA clone contained in ATCC Deposit No. 97891. Polynucleotides encoding all of the above deletion mutant polypeptide forms also are provided.

### Other Mutants

In addition to terminal deletion forms of the protein discussed above, it also will be recognized by one of ordinary skill in the art that some amino acid sequences of the FcR-I, FcR-II, FcR-III, FcR-IV, and FcR-V polypeptides can be varied without significant effect of the structure or function of the protein. If such differences in sequence are contemplated, it should be remembered that there will be critical areas on the protein which determine activity.

Thus, the invention further includes variations of the FcR-I, FcR-II, FcR-III, FcR-IV, and FcR-V polypeptides which show substantial FcR-I, FcR-II, FcR-III, FcR-IV, and FcR-V polypeptide activity or which include regions of FcR-I, FcR-II, FcR-III, FcR-IV, or FcR-V protein such as the protein portions discussed below. Such mutants include deletions, insertions, inversions, repeats, and type substitutions selected according to general rules known in the art so as have little effect on activity. For example, guidance concerning how to make phenotypically silent amino acid substitutions is provided by Bowie and colleagues (Science 247:1306-1310; 1990), wherein the authors indicate that there are two main approaches for studying the tolerance of an amino acid sequence to change. The first method relies on the process of evolution, in which mutations are either accepted or rejected by natural selection. The second approach uses genetic engineering to introduce amino acid changes at specific positions of a cloned gene and selections or screens to identify sequences that maintain functionality.

As the authors state, these studies have revealed that proteins are surprisingly tolerant of amino acid substitutions. The authors further indicate which amino acid changes are likely to be permissive at a certain position of the protein. For example, most buried amino acid residues require nonpolar side chains, whereas few features of surface side chains are generally conserved. Other such phenotypically silent substitutions are described by Bowie and colleagues (*supra,* and references cited therein). Typically seen as conservative substitutions are the replacements, one for another, among the aliphatic amino acids Ala, Val, Leu and Ile; interchange of the hydroxyl residues Ser and Thr, exchange of the acidic residues Asp and Glu, substitution between the amide residues Asn and Gln, exchange of the basic residues Lys and Arg and replacements among the aromatic residues Phe, Tyr.

Thus, the fragment, derivative or analog of the polypeptides of SEQ ID NO:2, SEQ ID NO:4, SEQ ID NO:6, SEQ ID NO: 8, and SEQ ID NO:10 or that encoded by the deposited cDNAs, may be (i) one in which one or more of the amino acid residues are substituted with a conserved or non-conserved amino acid residue (preferably a conserved amino acid residue) and such substituted amino acid residue may or may not be one encoded by the genetic code, or (ii) one in which one or more of the amino acid residues includes a substituent group, or (iii) one in which the complete mature form or extracellular domain of the polypeptide is fused with another compound, such as a compound to increase the half life of the polypeptide (for example, polyethylene glycol), or (iv) one in which the additional amino acids are fused to the above form of the polypeptide, such as an IgG Fc fusion region peptide or leader or secretory sequence or a sequence which is employed for purification of the above form of the polypeptide or a proprotein sequence. Such fragments, derivatives and analogs are deemed to be within the scope of those skilled in the art from the teachings herein Thus, the FcR-I, FcR-II, FcR-III, FcR-IV, and FcR-V proteins of the present invention may include one or more amino acid substitutions, deletions or additions, either from natural mutations or human manipulation. As indicated, changes are preferably of a minor nature, such as conservative amino acid substitutions that do not significantly affect the folding or activity of the protein (see Table 1).

**TABLE 1. Conservative Amino Acid Substitutions.**

| | |
|---|---|
| Aromatic | Phenylalanine |
| | Tryptophan |
| | Tyrosine |
| | |
| Hydrophobic | Leucine |
| | Isoleucine |
| | Valine |
| | |
| Polar | Glutamine |
| | Asparagine |
| | |
| Basic | Arginine |
| | Lysine |
| | Histidine |
| | |
| Acidic | Aspartic Acid |
| | Glutamic Acid |
| | |
| Small | Alanine |
| | Serine |
| | Threonine |
| | Methionine |
| | Glycine |

Amino acids in the FcR-I, FcR-II, FcR-III, and FcR-IV proteins of the present invention that are essential for function can be identified by methods known in the art, such as site-directed mutagenesis or alanine-scanning mutagenesis (Cunningham and Wells, Science 244:1081-1085; 1989). The latter procedure introduces single alanine mutations at every residue in the molecule. The resulting mutant molecules are then tested for biological activity such as receptor binding or *in vitro* or *in vitro* proliferative activity.

Of special interest are substitutions of charged amino acids with other charged or neutral amino acids which may produce proteins with highly desirable improved characteristics, such as less aggregation. Aggregation may not only reduce activity but also be problematic when preparing pharmaceutical formulations, because aggregates can be immunogenic (Pinckard, et al., Clin. Exp. Immunol. 2:331-340; 1967, Robbins, et al., Diabetes 36:838-845;1987, Cleland, et al., Crit. Rev. Therapeutic Drug Carrier Systems 10:307-377; 1993).

Since FcR-I, FcR-II, FcR-III, FcR-IV, and FcR-V are members of the Fc Receptor-related protein family, to modulate rather than completely eliminate biological activities of FcR-I, FcR-II, FcR-III, FcR-IV, and FcR-V preferably mutations are made in sequences encoding amino acids in the FcR-I, FcR-II, FcR-III, FcR-IV, and FcR-V conserved extracellular domain, i.e., in positions 1-289, 1-211, 1-421, 1-243, and 1-343 of SEQ ID NO:2, SEQ ID NO:4, SEQ ID NO:6, SEQ ID NO:8, and SEQ ID NO: 10, respectively, more preferably in residues within this region which are not conserved in all members of the Fc Receptor family. Also forming part of the present invention are isolated FcR-I, FcR-II, FcR-III, FcR-IV, and FcR-V mutants.

The polypeptides of the present invention are preferably provided in an isolated form, and preferably are substantially purified. Recombinantly produced versions of the FcR-I, FcR-II, FcR-III, FcR-IV, and FcR-V polypeptides can be substantially purified by the one-step method described by Smith and Johnson (Gene 67:31-40 (1988)). Polypeptides of the invention also can be purified from natural or recombinant sources using anti-FcR-I, FcR-II, FcR-III, FcR-IV, and FcR-V antibodies of the invention in methods which are well known in the art of protein purification.

The invention further provides isolated FcR-I, FcR-II, FcR-III, FcR-IV, and FcR-V polypeptides comprising an amino acid sequences selected from the group consisting of: (a) the amino acid sequence of the complete FcR-I polypeptide having the amino acid sequence at positions -21 to 406 of SEQ ID NO:2 or the complete FcR-I amino acid sequence encoded by the FcR-I cDNA clone contained in ATCC Deposit No. 97891; (b) the amino acid sequence of the complete FcR-I polypeptide having the amino acid sequence at positions -20 to 406 of SEQ ID NO:2 or the complete FcR-I amino acid sequence excepting the N-terminal methionine encoded by the FcR-I cDNA clone contained in ATCC Deposit No. 97891; (c) the amino acid sequence of the mature FcR-I polypeptide having the amino acid sequence at positions 1 to 406 in SEQ ID NO:2, or as encoded by the FcR-I cDNA clone contained in ATCC Deposit No. 97891; (d) the amino acid sequence of the extracellular domain of the FcR-I polypeptide having the amino acid sequence at positions 1-289 in SEQ ID NO:2, or as encoded by the FcR-I cDNA clone contained in ATCC Deposit No. 97891; (e) the amino acid sequence of the transmembrane domain of the FcR-I polypeptide having the amino acid sequence at positions 290-312 in SEQ ID NO:2, or as encoded by the FcR-I cDNA clone contained in ATCC Deposit No. 97891; (f) the amino acid sequence of the intracellular domain of the FcR-I polypeptide having the amino acid sequence at positions 313-406 in SEQ ID NO:2, or as encoded by the FcR-I cDNA clone contained in ATCC Deposit No. 97891; (g) the amino acid sequence of a soluble FcR-I polypeptide comprising the extracellular and intracelluar domains, but lacking the transmembrane domain; (h) the amino acid sequence of the complete FcR-II polypeptide having the amino acid sequence at positions -18 to 245 of SEQ ID NO:4 or the complete FcR-II amino acid sequence encoded by the FcR-II cDNA clone contained in ATCC Deposit No. 97891; (i) the amino acid sequence of the complete FcR-II polypeptide having the amino acid sequence at positions -17 to 245 of SEQ ID NO:4 or the complete FcR-II amino acid sequence excepting the N-terminal methionine encoded by the FcR-II cDNA clone contained in ATCC Deposit No. 97891; (j) the amino acid sequence of the mature FcR-II polypeptide having the amino acid sequence at positions 1 to 245 in SEQ ID NO:4, or as encoded by the FcR-II cDNA clone contained in ATCC Deposit No. 97891; (k) the amino acid sequence of the extracellular domain of the FcR-II polypeptide having the amino acid sequence at positions 1-211 in SEQ ID NO:4, or as encoded by the FcR-II cDNA clone contained in ATCC Deposit No. 97891; (l) the amino acid sequence of the transmembrane domain of the FcR-II polypeptide having the amino acid sequence at positions 212-229 in SEQ ID NO:4, or as encoded by the FcR-II cDNA clone contained in ATCC Deposit No. 97891; (m) the amino acid sequence of the intracellular domain of the FcR-II polypeptide having the amino acid sequence at positions 230-245 in SEQ ID NO:4, or as encoded by the FcR-II cDNA clone contained in ATCC Deposit No. 97891; (n) the amino acid sequence of a soluble FcR-II polypeptide comprising the extracellular and intracellular domains, but lacking the transmembrane domain; (o) the amino acid sequence of the complete FcR-III polypeptide having the amino acid sequence at positions -16 to 607 of SEQ ID NO:6 or the complete FcR-III amino acid sequence encoded by the FcR-III cDNA clone contained in ATCC Deposit No. 97891; (p) the amino acid sequence of the complete FcR-III polypeptide having the amino acid sequence at positions -15 to 607 of SEQ ID NO:6 or the complete FcR-III amino acid sequence excepting the N-terminal methionine encoded by the FcR-III cDNA clone contained in ATCC Deposit No. 97891; (q) the amino acid sequence of the mature FcR-III polypeptide having the amino acid sequence at positions 1 to 607 in SEQ ID NO:6, or as encoded by the FcR-III cDNA clone contained in ATCC Deposit No. 97891; (r) the amino acid sequence of the extracellular domain of the FcR-III polypeptide having the amino acid sequence at positions 1-421 in SEQ ID NO:6, or as encoded by the FcR-III cDNA clone contained in ATCC Deposit No. 97891; (s) the amino acid sequence of the transmembrane domain of the FcR-III polypeptide having the amino acid sequence at positions 422-448 in SEQ ID NO:6, or as encoded by the FcR-III cDNA clone contained in ATCC Deposit No. 97891; (t) the amino acid sequence of the intracellular domain of the FcR-III polypeptide having the amino acid sequence at positions 449-607 in SEQ ID NO:6, or as encoded by the FcR-III cDNA clone contained in ATCC Deposit No. 97891; (u) the amino acid sequence of a soluble FcR-III polypeptide comprising the extracellular and intracelluar domains, but lacking the transmembrane domain; (v) the amino acid sequence of the complete FcR-IV polypeptide having the amino acid sequence positions -16 to 456 of SEQ ID NO:8 or the complete FcR-IV amino acid sequence encoded by the FcR-IV cDNA clone contained in ATCC Deposit No. 97891; (w) the amino acid sequence of the complete FcR-IV polypeptide having the amino acid sequence positions -15 to 456 of SEQ ID NO:8 or the complete FcR-IV amino acid sequence excepting the N-terminal methionine encoded by the FcR-IV cDNA clone contained in ATCC Deposit No. 97891; (x) the amino acid sequence of the mature FcR-IV polypeptide having the amino acid sequence at positions 1 to 456 in SEQ ID NO:8, or as encoded by the FcR-IV cDNA clone contained in ATCC Deposit No. 97891; (y) the amino acid sequence of the extracellular domain of the FcR-IV polypeptide having the amino acid sequence at positions 1-243 in SEQ ID NO:8, or as encoded by the FcR-IV cDNA clone contained in ATCC Deposit No. 97891; (z) the amino acid sequence of the transmembrane domain of the FcR-IV polypeptide having the amino acid sequence at positions 244-264 in SEQ ID NO:8, or as encoded by the FcR-IV cDNA clone contained in ATCC Deposit No. 97891; (aa) the amino acid sequence of the intracellular domain of the FcR-IV polypeptide having the amino acid sequence at positions 265-456 in SEQ ID NO:8, or as encoded by the FcR-IV cDNA clone contained in ATCC Deposit No. 97891; (ab) the amino acid sequence of a soluble FcR-IV polypeptide comprising the extracellular and intracelluar domains, but lacking the transmembrane domain; (ac) the amino acid sequence of the complete FcR-V polypeptide having the amino acid sequence positions -16 to 498 of SEQ ID NO:10 or the complete FcR-V amino acid sequence encoded by the FcR-V cDNA clone contained in ATCC Deposit No. 209100; (ad) the amino acid sequence of the complete FcR-V polypeptide having the amino acid sequence positions -15 to 498 of SEQ ID NO: 10 or the complete FcR-V amino acid sequence excepting the N-terminal methionine encoded by the FcR-V cDNA clone contained in ATCC Deposit No. 209100; (ae) the amino acid sequence of the mature FcR-V polypeptide having the amino acid sequence at positions 1 to 498 in SEQ ID NO: 10, or as encoded by the FcR-V cDNA clone contained in ATCC Deposit No. 209100; (at) the amino acid sequence of the extracellular domain of the FcR-V polypeptide having the amino acid sequence at positions 1-343 in SEQ ID NO:10, or as encoded by the FcR-V cDNA clone contained in ATCC Deposit No. 209100; (ag) the amino acid sequence of the transmembrane domain of the FcR-V polypeptide having the amino acid sequence at positions 344-364 in SEQ ID NO:10, or as encoded by the FcR-V cDNA clone contained in ATCC Deposit No. 209100; (ah) the amino acid sequence of the intracellular domain of the FcR-V polypeptide having the amino acid sequence at positions 365-498 in SEQ ID NO:10, or as encoded by the FcR-V cDNA clone contained in ATCC Deposit No. 209100; (ai) the amino acid sequence of a soluble FcR-V polypeptide comprising the extracellular and intracelluar domains, but lacking the transmembrane domain. The polypeptides of the present invention also include polypeptides having an amino acid sequence at least 80% identical, more preferably at least 90% identical, and still more preferably 95%, 96%, 97%, 98% or 99% identical to those described in (a) through (ai) above, as well as polypeptides having an amino acid sequence with at least 90% similarity, and more preferably at least 95% similarity, to those above.

Further polypeptides of the present invention include polypeptides which have at least 90% similarity, more preferably at least 95% similarity, and still more preferably at least 96%, 97%, 98% or 99% similarity to those described above. The polypeptides of the invention also comprise those which are at least 80% identical, more preferably at least 90% or 95% identical, still more preferably at least 96%, 97%, 98% or 99% identical to the polypeptide encoded by the deposited cDNAs or to the polypeptides of SEQ ID NO:2, SEQ ID NO:4, SEQ ID NO:6, SEQ ID N0:8, SEQ ID NO:10 and also include portions of such polypeptides with at least 30 amino acids and more preferably at least 50 amino acids.

By "% similarity" for two polypeptides is intended a similarity score produced by comparing the amino acid sequences of the two polypeptides using the Bestfit program (Wisconsin Sequence Analysis Package, Version 8 for Unix, Genetics Computer Group, University Research Park, 575 Science Drive, Madison, WI 53711) and the default settings for determining similarity. Bestfit uses the local homology algorithm of Smith and Waterman (Advances in Applied Mathematics 2:482-489; 1981) to find the best segment of similarity between two sequences.

By a polypeptide having an amino acid sequence at least, for example, 95% "identical" to a reference amino acid sequence of a FcR-I, FcR-II, FcR-III, FcR-IV, and FcR-V polypeptide is intended that the amino acid sequence of the polypeptide is identical to the reference sequence except that the polypeptide sequence may include up to five amino acid alterations per each 100 amino acids of the reference amino acid of the FcR-I, FcR-II, FcR-III, FcR-IV, and FcR-V polypeptide. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

As a practical matter, whether any particular polypeptide is at least 90%, 95%, 96%, 97%, 98% or 99% identical to, for instance, the amino acid sequences shown in SEQ ID NO:2, SEQ ID NO:4, SEQ ID NO:6, SEQ ID NO:8, and SEQ ID NO:10 or to the amino acid sequences encoded by deposited cDNA clones can be determined conventionally using known computer programs such the Bestfit program (Wisconsin Sequence Analysis Package, Version 8 for Unix, Genetics Computer Group, University Research Park, 575 Science Drive, Madison, WI 53711). When using Bestfit or any other sequence alignment program to determine whether a particular sequence is, for instance, 95% identical to a reference sequence according to the present invention, the parameters are set, of course, such that the percentage of identity is calculated over the full length of the reference amino acid sequence and that gaps in homology of up to 5% of the total number of amino acid residues in the reference sequence are allowed.

The polypeptide of the present invention could be used as a molecular weight marker on SDS-PAGE gels or on molecular sieve gel filtration columns using methods well known to those of skill in the art.

As described in detail below, the polypeptides of the present invention can also be used to raise polyclonal and monoclonal antibodies, which are useful in assays for detecting FcR-I, FcR-II, FcR-III, FcR-IV, and FcR-V protein expression as described below or as agonists and antagonists capable of enhancing or inhibiting FcR-I, FcR-II, FcR-III, FcR-IV, and FcR-V protein function. Further, such polypeptides can be used in the yeast two-hybrid system to "capture" FcR-I, FcR-II, FcR-III, FcR-IV, and FcR-V protein binding proteins which are also candidate agonists and antagonists according to the present invention. The yeast two hybrid system is described by Fields and Song (Nature 340:245-246; 1989).

### Epitope-Bearing Portions

In another aspect, the invention provides a peptide or polypeptide comprising an epitope-bearing portion of a polypeptide of the invention. The epitope of this polypeptide portion is an immunogenic or antigenic epitope of a polypeptide of the invention. An "immunogenic epitope" is defined as a part of a protein that elicits an antibody response when the whole protein is the immunogen. On the other hand, a region of a protein molecule to which an antibody can bind is defined as an "antigenic epitope." The number of immunogenic epitopes of a protein generally is less than the number of antigenic epitopes (Geysen, et al., Proc. Natl. Acad Sci. USA 81:3998- 4002; 1983).

As to the selection of peptides or polypeptides bearing an antigenic epitope (i.e., that contain a region of a protein molecule to which an antibody can bind), it is well known in that art that relatively short synthetic peptides that mimic part of a protein sequence are routinely capable of eliciting an antiserum that reacts with the partially mimicked protein (Sutcliffe, J. G., et al., (1983) Science, 219:660-666). Peptides capable of eliciting protein-reactive sera are frequently represented in the primary sequence of a protein, can be characterized by a set of simple chemical rules, and are confined neither to immunodominant regions of intact proteins (i.e., immunogenic epitopes) nor to the amino or carboxyl terminals. Antigenic epitope-bearing peptides and polypeptides of the invention are therefore useful to raise antibodies, including monoclonal antibodies, that bind specifically to a polypeptide of the invention (Wilson et al., (1984) Cell 37:767-778).

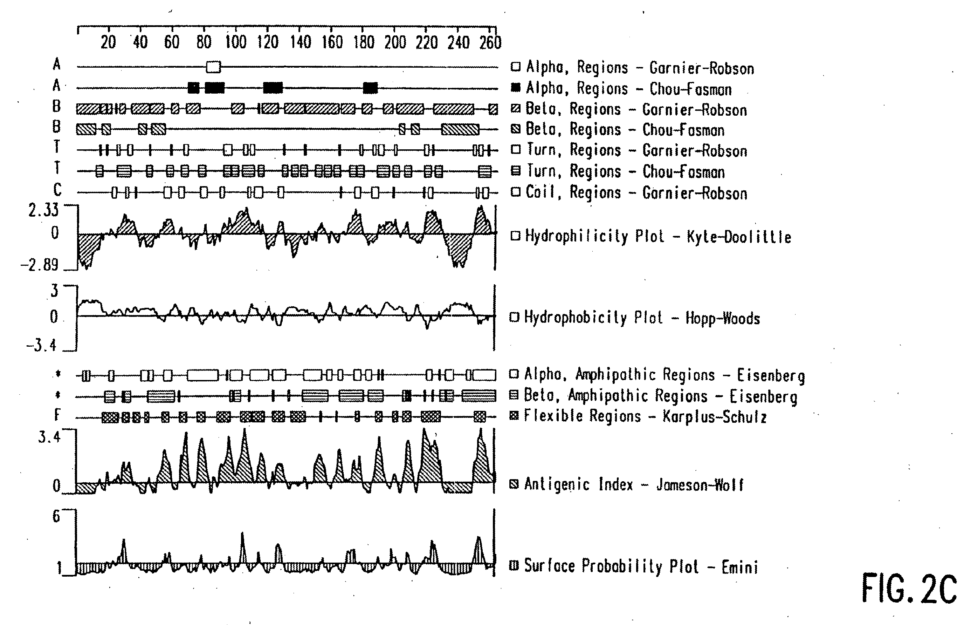

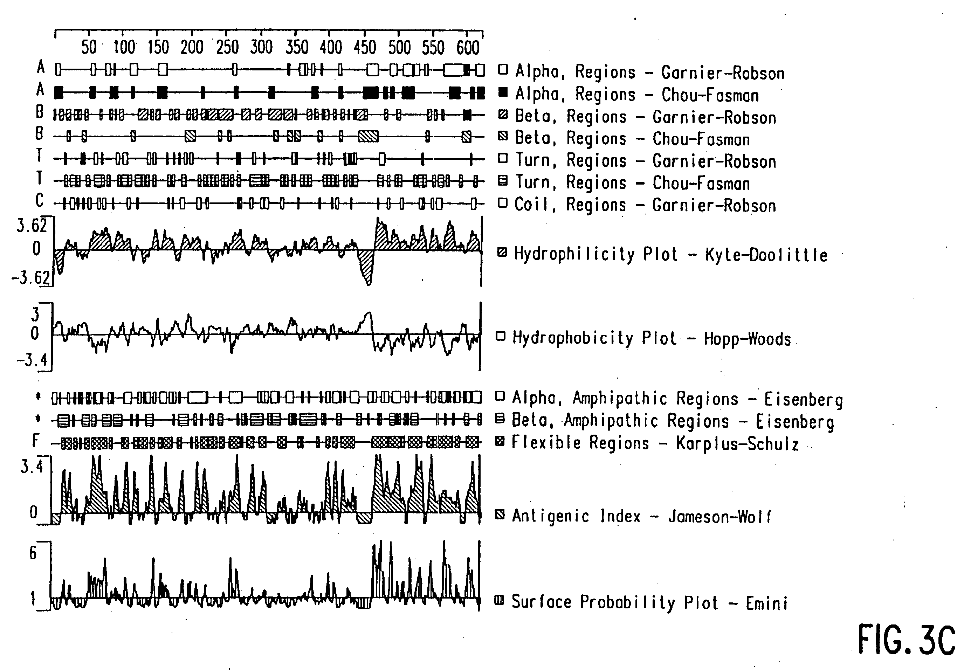

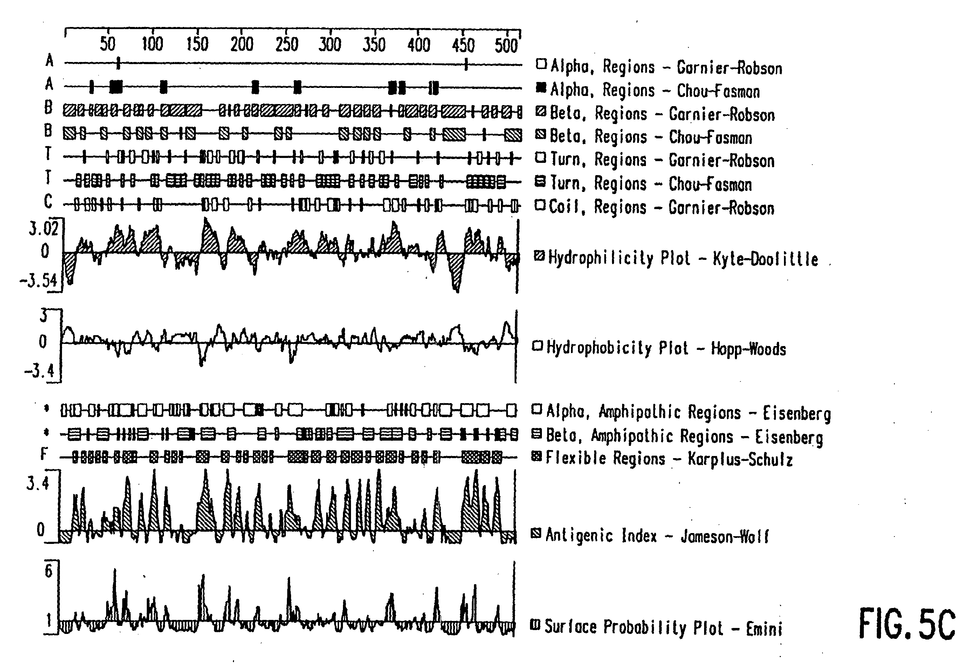

Antigenic epitope-bearing peptides and polypeptides of the invention preferably contain a sequence of at least seven, more preferably at least nine and most preferably between about 15 to about 30 amino acids contained within the amino acid sequence of a polypeptide of the invention. Non-limiting examples of antigenic polypeptides or peptides that can be used to generate FcR-I-specific antibodies include: a polypeptide comprising amino acid residues from about Cys-37 to about Tyr-46, from about Ile-61 to about Phe-71, from about Gly-94 to about Glu-103, from about Cys-145 to about Ala-168, from about Ser-176 to about Pro-193, from about Lys-247 to about Ala-263, from about Ser-293 to about Ile-304, from about Thr-346 to about Ala-368, and from about Thr-413 to about Glu-427 in SEQ ID NO:2. These polypeptide fragments have been determined to bear antigenic epitopes of the FcR-I protein by the analysis of the Jameson-Wolf antigenic index, as shown in Figure 1C above. Likewise, non-limiting examples of antigenic polypeptides or peptides that can be used to generate FcR-11-specific antibodies include: a polypeptide comprising amino acid residues from about Leu-51 to about Trp-60, from about Val-90 to about Arg-99, from about Cys-101 to about Trp-112, from about Ser-216 to about Val-231, and from about Trp-251 to about Ile-261 in SEQ ID NO:4. These polypeptide fragments have been determined to bear antigenic epitopes of the FeR-II protein by the analysis of the Jameson-Wolf antigenic index, as shown in Figure 2C above.. In addition, non-limiting examples of antigenic polypeptides or peptides that can be used to generate FcR-III-specific antibodies include: a polypeptide comprising amino acid residues from about Leu-37 to about Ile-69, from about His-76 to about Leu-110. from about Leu-126 to about His-135, from about Glu-142 to about Gln-155, from about Asn-162 to about Phe-178, from about Ser-192 to about Leu-212, from about Lys-242 to about Leu-260, from about Ser-271 to about Leu-297, from about Tyr-381 to about Glu-427, and from about Arg-450 to about Ala-606 in SEQ ID NO:6. These polypeptide fragments have been determined to bear antigenic epitopes of the FcR-III protein by the analysis of the Jameson-Wolf antigenic index, as shown in Figure 3C above.. In addition, non-limiting examples of antigenic polypeptides or peptides that can be used to generate FcR-IV-specific antibodies include: a polypeptide comprising amino acid residues from about Thr-36 to about Ile-69, from about Ser-71 to about Leu-99, from about Ala-104 to about Ala-112, from about Thr-119 to about Phe-137, from about Ser-191 to about Ile-200, from about Ser-204 to about Met-278, from about His-235 to about Val-244, and from about Arg-268 to about Gln-456 in SEQ ID NO: 8. These polypeptide fragments have been determined to bear antigenic epitopes of the FcR-IV protein by the analysis of the Jameson-Wolf antigenic index, as shown in Figure 4C above. Finally, non-limiting examples of antigenic polypeptides or peptides that can be used to generate FcR-V-specific antibodies include: a polypeptide comprising amino acid residues from about Cys-140 to about Ser-160, from about Val-169 to about Val-189, from about Val-204 to about Pro-216, from about Val-238 to about Gln-258, from about Ser-270 to about Asp-297, from about Phe-304 to about Val-312, from about Pro-320 to about Val-369, from about Gly-404 to about Asn-416, and from about Gln-439 to about Ile-483 in SEQ ID NO:10. These polypeptide fragments have been determined to bear antigenic epitopes of the FcR-V protein by the analysis of the Jameson-Wolf antigenic index, as shown in Figure 5C above.

The epitope-bearing peptides and polypeptides of the invention may be produced by any conventional means *Houghten. R. A. (1985) Proc. Natl. Acad Sci. USA 82:5131-5135) This "Simultaneous Multiple Peptide Synthesis (SMPS)" process is further described in U.S. Patent No. 4,631,211 to Houghten and colleagues (1986).

Epitope-bearing peptides and polypeptides of the invention are used to induce antibodies according to methods well known in the art (Sutcliffe, *et al., supra,* Wilson, *et al.*, *supra,* Chow, M., et al., Proc. Natl. Acad. Sci. USA 82:910-914, and Bittle, F. J., et al., (1985) J. Gen. Virol. 66:2347-2354). Immunogenic epitope-bearing peptides of the invention, i.e.. those parts of a protein that elicit an antibody response when the whole protein is the immunogen, are identified according to methods known in the art (Geysen, *et al., supra*). Further still, U.S. Patent No. 5.194.392 to Geysen (1990) describes a general method of detecting or determining the sequence of monomers (amino acids or other compounds) which is a topological equivalent of the epitope (i.e., a "mimotope") which is complementary to a particular paratope (antigen binding site) of an antibody of interest. More generally, U.S. Patent No. 4,433,092 to Geysen (1989) describes a method of detecting or determining a sequence of monomers which is a topographical equivalent of a ligand which is complementary to the ligand binding site of a particular receptor of interest. Similarly, U.S. Patent No. 5,480,971 to Houghten, R. A. and coworkers (1996) on Peralkylated Oligopeptide Mixtures discloses linear C1-C7-alkyl peralkylated oligopeptides and sets and libraries of such peptides, as well as methods for using such oligopeptide sets and libraries for determining the sequence of a peralkylated oligopeptide that preferentially binds to an acceptor molecule of interest. Thus, non-peptide analogs of the epitope-bearing peptides of the invention also can be made routinely by these methods.

### Fusion Proteins

As one of skill in the art will appreciate, FcR-I, FcR-II, FcR-III, FcR-IV, and FcR-V polypeptides of the present invention and the epitope-bearing fragments thereof described above can be combined with parts of the constant domain of immunoglobulins (IgG), resulting in chimeric polypeptides. These fusion proteins facilitate purification and show an increased half-life *in vivo*. This has been shown, e.g., for chimeric proteins consisting of the first two domains of the human CD4-polypeptide and various domains of the constant regions of the heavy or light chains of mammalian immunoglobulins (EP A 394,827; Traunecker, et al., (1988) Nature 331:84-86). Fusion proteins that have a disulfide-linked dimeric structure due to the IgG part can also be more efficient in binding and neutralizing other molecules than the monomeric FcR-I, FcR-II, FcR-III, FcR-IV, and FcR-V proteins or protein fragments alone (Fountoulakis et al., (1995) J. Biochem. 270:3958-3964).

### Antibodies

FcR-I, FcR-II, FcR-III, FcR-IV, and FcR-V-protein specific antibodies for use in the present invention can be raised against the intact FcR-I, FcR-II, FcR-III, FcR-IV, and FcR-V proteins or an antigenic polypeptide fragments thereof, which may be presented together with a carrier protein, such as an albumin, to an animal system (such as rabbit or mouse) or, if it is long enough (at least about 25 amino acids), without a carrier.

As used herein, the term "antibody" (Ab) or "monoclonal antibody" (Mab) is meant to include intact molecules as well as antibody fragments (such as, for example, Fab and F(ab')2 fragments) which are capable of specifically binding to FcR-I, FcR-II, FcR-III, FcR-IV, or FcR-V proteins. Fab and F(ab')2 fragments lack the Fc fragment of intact antibody, clear more rapidly from the circulation, and may have less non-specific tissue binding of an intact antibody (Wahl *et al.,* (1083) *J. Nucl. Med.* **24:**316-325). Thus, these fragments are preferred.

The antibodies of the present invention may be prepared by any of a variety of methods. For example, cells expressing the FcR-I, FcR-II, FcR-III, FcR-IV. and FcR-V proteins or an antigenic fragments thereof can be administered to an animal in order to induce the production of sera containing polyclonal antibodies. In a preferred method, preparations of FcR-I, FcR-II, FcR-III, FcR-IV, or FcR-V protein are prepared and purified to render them substantially free of natural contaminants. Such preparations are then introduced into an animal in order to produce polyclonal antisera of greater specific activity.

In the most preferred method, the antibodies of the present invention are monoclonal antibodies (or FcR-I, FcR-II, FcR-III, FcR-IV, and FcR-V protein binding fragments thereof). Such monoclonal antibodies can be prepared using hybridoma technology (Köhler et al., (1975) Nature 256:495; Köhler et al., (1976) Eur. J. Immunol. 6:511; Köhler et al., (1976) Eur. J. Immunol. 6:292; Hammerling, et al., (1981) in: Monoclonal Antibodies and T-Cell Hybridomas, Elsevier, NY, pp. 563-681). In general, such procedures involve immunizing an animal (preferably a mouse) with FcR-I, FcR-II, FcR-III, FcR-IV, or FcR-V protein antigen or, more preferably, with a FcR-I, FcR-II, FcR-III, FcR-IV, or FcR-V protein-expressing cell. Suitable cells can be recognized by their capacity to bind anti-FcR-I, FcR-II, FcR-III, FcR-IV, or FcR-V protein antibody. Such cells may be cultured in any suitable tissue culture medium; however, it is preferable to culture cells in Earle's modified Eagle's medium supplemented with 10% fetal bovine serum (inactivated at about 56° C), and supplemented with about 10 g/l of nonessential amino acids, about 1,000 U/ml of penicillin, and about 100 µg/ml of streptomycin. The splenocytes of such mice are extracted and fused with a suitable myeloma cell line. Any suitable myeloma cell line may be employed in accordance with the present invention; however, it is preferable to employ the parent myeloma cell line (SP2O), available from the American Type Culture Collection, Rockville, Maryland. After fusion, the resulting hybridoma cells are selectively maintained in HAT medium, and then cloned by limiting dilution as described by Wands and colleagues (Gastroenterology 80:225-232; 1981). The hybridoma cells obtained through such a selection are then assayed to identify clones which secrete antibodies capable of binding the FcR-I, FcR-II, FcR-III, FcR-IV, and FcR-V protein antigens.

Alternatively, additional antibodies capable of binding to FcR-I, FcR-II, FcR-III, FcR-IV, or FcR-V protein antigens may be produced in a two-step procedure through the use of anti-idiotypic antibodies. Such a method makes use of the fact that antibodies are themselves antigens, and that, therefore, it is possible to obtain an antibody which binds to a second antibody. In accordance with this method, FcR-I, FcR-II, FcR-III, FcR-IV, or FcR-V-protein specific antibodies are used to immunize an animal, preferably a mouse. The splenocytes of such an animal are then used to produce hybridoma cells, and the hybridoma cells are screened to identify clones which produce an antibody whose ability to bind to the FcR-I, FcR-II, FcR-III, FcR-IV, and FcR-V protein-specific antibodies can be blocked by the FcR-I, FcR-II, FcR-III, FcR-IV, and FcR-V protein antigens. Such antibodies comprise anti-idiotypic antibodies to the FcR-I, FcR-II, FcR-III, FcR-IV, and FcR-V protein-specific antibodies and can be used to immunize an animal to induce formation of further FcR-I, FcR-II, FcR-III, FcR-IV, and FcR-V protein-specific antibodies.

It will be appreciated that Fab and F(ab')2 and other fragments of the antibodies of the present invention may be used according to the methods disclosed herein. Such fragments are typically produced by proteolytic cleavage, using enzymes such as papain (to produce Fab fragments) or pepsin (to produce F(ab')2 fragments). Alternatively, FcR-I, FcR-II, FcR-III, FcR-IV, and FcR-V protein-binding fragments can be produced through the application of recombinant DNA technology or through synthetic chemistry.

For *in vivo* use of anti-FcR-I, FcR-II, FcR-III, FcR-IV, and FcR-V in humans, it may be preferable to use "humanized" chimeric monoclonal antibodies. Such antibodies can be produced using genetic constructs derived from hybridoma cells producing the monoclonal antibodies described above. Methods for producing chimeric antibodies are known in the art (for review, Morrison, (1985) Science 229:1202; Oi, et al., (1986) BioTechniques 4:214; Cabilly, et al., U.S. Patent No. 4,816.567; Taniguchi, et al., EP 171496; Morrison, et al., EP 173494; Neuberger, et al., WO 8601533; Robinson, et al., WO 8702671; Boulianne, et al., (1984) Nature 322:643; Neuberger, et al., (1985) Nature 314:268).

### Immune System-Related Disorders

### Diagnosis

The present inventors have discovered that FcR-I, FcR-II, FcR-III, FcR-IV. and FcR-V are expressed in a variety of hematopoietic cells and tissues including monocytes, macrophages, dendritic cells, and spleen. For a number of immune system-related disorders, substantially altered (increased or decreased) levels of FcR-I, FcR-II, FcR-III, FcR-IV, or FcR-V gene expression can be detected in immune system tissue or other cells or bodily fluids (e.g., sera, plasma, urine, synovial fluid or spinal fluid) taken from an individual having such a disorder, relative to a "standard" FcR-I, FcR-II, FcR-III, FcR-IV, and FcR-V gene expression levels, that is, the FcR-I, FcR-II, FcR-III, FcR-IV, and FcR-V expression levels in immune system tissues or bodily fluids from an individual not having the immune system disorder. Thus, the invention provides a diagnostic method useful during diagnosis of a immune system disorder, which involves measuring the expression level of the genes encoding the FcR-I, FcR-II, FcR-III, FcR-IV, or FcR-V proteins in immune system tissue or other cells or body fluid from an individual and comparing the measured gene expression level with a standard FcR-I, FcR-II, FcR-III, FcR-IV,. and FcR-V FcR-IV gene expression level, whereby an increase or decrease in the gene expression level compared to the standard is indicative of an immune system disorder.

In particular, it is believed that certain tissues in mammals with various cancers of the immune system express significantly enhanced or reduced levels of the FcR-I, FcR-II, FcR-III, FcR-IV and FcR-V proteins and mRNA encoding the FcR-I, FcR-II, FcR-III, FcR-IV, and FcR-V proteins when compared to a corresponding "standard" level. Further, it is believed that enhanced levels of the FcR-I, FcR-II, FcR-III, FcR-IV, and FcR-V proteins can be detected in certain body fluids (e.g., sera, plasma, urine, and spinal fluid) from mammals with such a cancer when compared to sera from mammals of the same species not having the cancer.

Thus, the invention provides a diagnostic method useful during diagnosis of an immune system disorder, including cancers of the immune system, which involves measuring the expression level of the genes encoding the FcR-I, FcR-II, FcR-III, FcR-IV, or FcR-V protein in immune system tissue or other cells or body fluid from an individual and comparing the measured gene expression level with a standard FcR-I, FcR-II, FcR-III, FcR-IV, or FcR-V gene expression level, whereby an increase or decrease in the gene expression level compared to the standard is indicative of an immune system disorder.

Where a diagnosis of a disorder in the immune system including diagnosis of a cancer or tumor, has already been made according to conventional methods, the present invention is useful as a prognostic indicator, whereby patients exhibiting enhanced or depressed FcR-I, FcR-II, FcR-III. FcR-IV, or FcR-V gene expression will experience a worse clinical outcome relative to patients expressing the gene at a level nearer the standard level.

By "assaying the expression level of the genes encoding the FcR-I, FcR-II, FcR-III, FcR-IV, or FcR-V proteins" is intended qualitatively or quantitatively measuring or estimating the level of the FcR-I, FcR-II, FcR-III, FcR-IV, or FcR-V proteins or the level of the mRNA encoding the FcR-I, FcR-II, FcR-III, FcR-IV, or FcR-V proteins in a first biological sample either directly (e.g., by determining or estimating absolute protein level or mRNA level) or relatively (e.g., by comparing to the FcR-I, FcR-II, FcR-III, FcR-IV, or FcR-V protein levels or mRNA levels in a second biological sample). Preferably, the FcR-I, FcR-II, FcR-III, FcR-IV, and FcR-V protein level or mRNA level in the first biological sample is measured or estimated and compared to a standard FcR-I, FcR-II, FcR-III, FcR-IV, and FcR-V protein levels or mRNA levels, the standard being taken from a second biological sample obtained from an individual not having the disorder or being determined by averaging levels from a population of individuals not having a disorder of the immune system. As will be appreciated in the art, once a standard FcR-I, FcR-II, FcR-III, FcR-IV, and FcR-V protein level or mRNA level is known, it can be used repeatedly as a standard for comparison.

By "biological sample" is intended any biological sample obtained from an individual, body fluid, cell line, tissue culture, or other source which contains FcR-I, FcR-II, FcR-III, FcR-IV, and FcR-V protein or mRNA. As indicated, biological samples include body fluids (such as sera, plasma, urine, synovial fluid and spinal fluid) which contain free FcR-I, FcR-II, FcR-III, FcR-IV, and FcR-V or "extracellular domains of" FcR-I, FcR-II, FcR-III, FcR-IV, and FcR-V protein, immune system tissue, and other tissue sources found to express complete or mature form or the extracellular domain of the FcR-I, FcR-II, FcR-III, FcR-IV, or FcR-V proteins. Methods for obtaining tissue biopsies and body fluids from mammals are well known in the art. Where the biological sample is to include mRNA, a tissue biopsy is the preferred source.

The present invention is useful for diagnosis or treatment of various immune system-related disorders in mammals, preferably humans. Such disorders include immune-complex related inflammatory diseases such as rheumatoid arthritis, systemic lupus erythematosis, autoimmune hemolytic anemia, thrombocytopenia and IgG- or IgE-mediated inflammation, anaphylaxis, allergy, and any disregulation of immune cell function affecting, or including, but not limited to, leukemias, lymphomas, immunosuppression, immunity, humoral immunity, inflammatory bowel disease, myelo suppression, and the like.

Total cellular RNA can be isolated from a biological sample using any suitable technique such as the single-step guanidinium-thiocyanate-phenol-chloroform method described by Chomczynski and Sacchi (Anal. Biochem. (1987) 162:156-159). Levels of mRNA encoding the FcR-I, FcR-11, FcR-III, FcR-IV, and FcR-V proteins are then assayed using any appropriate method. These include Northern blot analysis. S 1 nuclease mapping, the polymerase chain reaction (PCR), reverse transcription in combination with the polymerase chain reaction (RT-PCR), and reverse transcription in combination with the ligase chain reaction (RT-LCR).

Assaying FcR-1. FcR-11, FcR-111, FcR-IV, or FcR-V protein levels in a biological sample can occur using antibody-based techniques. For example, expression of FcR-I. FcR-11, FcR-III, or FcR-IV proteins in tissues can be studied with classical immunohistological methods (Jalkanen, M., et al., (1985) J. Cell. Biol. 101:976-985: Jalkanen, M., et al., (1987) J. Cell . Biol. 105:3087-3096). Other antibody-based methods useful for detecting FcR-I, FcR-11, FcR-111, FcR-IV, or FcR-V protein gene expression include immunoassays, such as the enzyme linked immunosorbent assay (ELISA) and the radioimmunoassay (RIA). Suitable antibody assay labels are known in the art and include enzyme labels, such as, glucose oxidase, and radioisotopes, such as iodine (¹²⁵I, ¹²¹I), carbon (¹⁴C), sulfur (³⁵S), tritium (³H), indium (¹¹²In), and technetium (^{99m}Tc), and fluorescent labels, such as fluorescein and rhodamine, and biotin.

In addition to assaying FcR-I, FcR-II, FcR-III, FcR-IV, or FcR-V protein levels in a biological sample obtained from an individual, FcR-I, FcR-II, FcR-III, FcR-IV, or FcR-V proteins can also be detected *in vivo* by imaging. Antibody labels or markers for *in vivo* imaging of FcR-I, FcR-II, FcR-III, FcR-IV, or FcR-V proteins include those detectable by X-radiography, NMR or ESR. For X-radiography, suitable labels include radioisotopes such as barium or cesium, which emit detectable radiation but are not overtly harmful to the subject. Suitable markers for NMR and ESR include those with a detectable characteristic spin, such as deuterium, which may be incorporated into the antibody by labeling of nutrients for the relevant hybridoma.

An FcR-I, FcR-II, FcR-III, FcR-IV, or FcR-V protein-specific antibody or antibody fragment which has been labeled with an appropriate detectable imaging moiety, such as a radioisotope (for example, ¹³¹I, ¹¹²In, ^{99m}Tc), a radio-opaque substance, or a material detectable by nuclear magnetic resonance, is introduced (for example, parenterally, subcutaneously or intraperitoneally) into the mammal to be examined for immune system disorder. It will be understood in the art that the size of the subject and the imaging system used will determine the quantity of imaging moiety needed to produce diagnostic images. In the case of a radioisotope moiety, for a human subject, the quantity of radioactivity injected will normally range from about 5 to 20 millicuries of ^{99m}Tc. The labeled antibody or antibody fragment will then preferentially accumulate at the location of cells which contain FcR-I, FcR-II, FcR-III, FcR-IV. or FcR-V protein. *In vivo* tumor imaging is described in S. W. Burchiel and colleagues ("Immunopharmacokinetics of Radiolabeled Antibodies and Their Fragments", Chapter 13 in Tumor Imaging: The Radiochemical Detection of Cancer. S.W. Burchiel and B. A. Rhodes, eds.. Masson Publishing Inc.; 1982).

### Treatment

As noted above, FcR-I, FcR-II, FcR-III, FcR-IV, and FcR-V polynucleotides and polypeptides are useful for diagnosis of conditions involving abnormally high or low expression of FcR-I, FcR-II, FcR-III, FcR-IV, and FcR-V activities. Given the cells and tissues where FcR-I, FcR-II, FcR-III, FcR-IV, and FcR-V is expressed as well as the activities modulated by FcR-I, FcR-II, FcR-III, FcR-IV, and FcR-V. it is readily apparent that a substantially altered (increased or decreased) level of expression of FcR-I, FcR-II, FcR-III, FcR-IV, or FcR-V in an individual compared to the standard or "normal" level produces pathological conditions related to the bodily system(s) in which FcR-I, FcR-II, FcR-III, FcR-IV, or FcR-V are expressed and/or are active.

It will also be appreciated by one of ordinary skill that; since the FcR-I, FcR-II, FcR-III, FcR-IV, and FcR-V proteins of the invention are members of the Fc Receptor Family, the extracellular domain of the protein may be released by proteolytic cleavage as a soluble form from the cells which express the FcR-I, FcR-II, FcR-III, FcR-IV, and FcR-V polypeptides. Therefore, when the soluble, extracellular domain of the FcR-I, FcR-II, FcR-III, FcR-IV, and FcR-V polypeptides is added from an exogenous source to cells, tissues or the body of an individual, the protein will exert its physiological activities on its target cells of that individual.

Therefore, it will be appreciated that conditions caused by a increase in the standard or normal level of FcR-I, FcR-II, FcR-III, FcR-IV, and FcR-V activity in an individual, particularly disorders of the immune system, can be treated by administration of FcR-I, FcR-II, FcR-III, FcR-IV, and FcR-V polypeptides (in the form of soluble extracellular domains or cells expressing the complete proteins). Thus, the invention also provides a method of treatment of an individual in need of an increased level of FcR-I, FcR-II, FcR-III, FcR-IV, and FcR-V activity comprising administering to such an individual a pharmaceutical composition comprising an amount of an isolated FcR-I, FcR-II, FcR-III, FcR-IV, and FcR-V polypeptide of the invention effective to decrease the FcR-I, FcR-II, FcR-III, FcR-IV, and FcR-V activity level in such an individual.

Since a soluble form of an FcR lacks, by definition, the transmembrane and intracellular domains of the complete or mature form of the protein, such polypeptides may be useful as a mechanism to compete for the binding of ligand or other proteins to the functional and naturally-occurring membrane-associated form of the polypeptide. As a result, stimulation of the normal function of the naturally-occurring, membrane-associated form of the FcR-like polypeptide by a ligand or other binding protein may be dimished by the presence of an excess of soluble form of the FcR-like polypeptide. Those of skill in the art will also recognize that there are a large number of possible uses of such FcR-like polypetide variants including attentuation of an immune response by competition for FcR-like polypeptide-specific antibodies, stimulation of an unrelated signal transduction pathway through the generation and use of membrane-associated, chimeric receptor molecules which are comprised of the extracellular domain of the FcR-like polypeptide and the transmembrane and intracellular domains of another naturally-occurring or non-naturally-occurring polypeptide, and the like. Thus, such extracellular forms are useful for treating a number of disease states including systemic lupus erythematosus (SLE), autoimmune hemolytic anemia (AIHA), idiopathic thrombocytopenia purpura (ITP), colorectal cancer, breast cancer. Hodgekin's Lymphoma and other lymphomas, leukemia, and intracellular pathogenic disease such as that caused by *Toxoplasma gondii.* In addition, such molecules are useful for modulating the immune response by interfering with the functions of their membrane-bound counterparts in such situations or processes as phagocytosis, endocytosis, antibody-dependent cell-mediated cytotoxicity (ADCC), the release of mediators of inflammation, and the regulation of B-cell activation and antibody production. Extracellular forms of FcR-I, FcR-II, FcR-III, FcR-IV, and FcR-V may also be useful in the treatment of HIV. Dengue, or other viral infection by interefering with a hypothesized component of the mechanism of cellular entry by these pathogens.

### Formulations

The FcR-I, FcR-II, FcR-III, FcR-IV, and FcR-V polypeptide compositions will be formulated and dosed in a fashion consistent with good medical practice, taking into account the clinical condition of the individual patient (especially the side effects of treatment with FcR-I, FcR-II, FcR-III, FcR-IV, or FcR-V polypeptides alone), the site of delivery of the FcR-I, FcR-II, FcR-III, FcR-IV, and FcR-V polypeptide compositions, the method of administration, the scheduling of administration, and other factors known to practitioners. The "effective amount" of FcR-I, FcR-II, FcR-III, FcR-IV, or FcR-V polypeptide for purposes herein is thus determined by such considerations.

AS a general proposition, the total pharmaceutically effective amount of FcR-I, FcR-II, FcR-III, FcR-IV, and FcR-V polypeptide administered parenterally per dose will be in the range of about 1 µg/kg/day to 10 mg/kg/day of patient body weight, although, as noted above, this will be subject to therapeutic discretion. More preferably, this dose is at least 0.01 mg/kg/day, and most preferably for humans between about 0.01 and 1 mg/kg/day for the hormone. If given continuously, the FcR-I, FcR-II, FcR-III, FcR-IV, and FcR-V polypeptide is typically administered at a dose rate of about 1 µg/kg/hour to about 50 µg/kg/hour, either by 1-4 injections per day or by continuous subcutaneous infusions, for example, using a mini-pump. An intravenous bag solution may also be employed. The length of treatment needed to observe changes and the interval following treatment for responses to occur appears to vary depending on the desired effect.

Pharmaceutical compositions containing the FcR-I, FcR-II, FcR-III, FcR-IV, and FcR-V polypeptides of the invention may be administered orally, rectally, parenterally, intracistemally, intravaginally, intraperitoneally, topically (as by powders, ointments, drops or transdermal patch), bucally, or as an oral or nasal spray. By "pharmaceutically acceptable carrier" is meant a non-toxic solid, semisolid or liquid filler, diluent, encapsulating material or formulation auxiliary of any type. The term "parenteral" as used herein refers to modes of administration which include intravenous, intramuscular, intraperitoneal, intrasternal, subcutaneous and intraarticular injection and infusion.

The FcR-I, FcR-II, FcR-III, FcR-IV, and FcR-V polypeptides are also suitably administered by sustained-release systems. Suitable examples of sustained-release compositions include semi-permeable polymer matrices in the form of shaped articles, e.g., films, or mirocapsules. Sustained-release matrices include polylactides (U.S. Pat. No. 3,773,919, EP 58,481), copolymers of L-glutamic acid and gamma-ethyl-L-glutamate (Sidman, U. et al., (1983) Biopolymers 22:547-556), poly (2- hydroxyethyl methacrylate) (R. Langer et al., (1981) J. Biomed Mater. Res. 15:167-277; and R. Langer, (1982) Chem. Tech. 12:98-105), ethylene vinyl acetate (Langer, R., *et al., Id.*) or poly-D- (-)-3-hydroxybutyric acid (EP 133,988). Sustained-release FcR-I, FcR-II, FcR-III, FcR-IV, and FcR-V polypeptide compositions also include liposomally entrapped FcR-I, FcR-II, FcR-III, FcR-IV, and FcR-V polypeptides. Liposomes containing FcR-I, FcR-II, FcR-III, FcR-IV, and FcR-V polypeptides are prepared by methods known per se (DE 3,218,121; Epstein, et al., (1985) Proc. Natl. Acad Sci. (USA) 82:3688-3692; Hwang, et al., (1980) Proc. Natl. Acad Sci. (USA) 77:4030-4034; EP 52,322, EP 36,676, EP 88,046, EP 143,949, EP 142,641, Japanese Pat. Appl. 83-118008, U.S. Pat. Nos. 4,485,045 and 4,544,545, and EP 102,324). Ordinarily; the liposomes are of the small (about 200-800 Angstroms) unilamellar type in which the lipid content is greater than about 30 mol, percent cholesterol, the selected proportion being adjusted for the optimal FcR-I, FcR-II, FcR-III, FcR-IV, and FcR-V polypeptide therapy.

For parenteral administration, in one embodiment, the FcR-I, FcR-II, FcR-III, FcR-IV, and FcR-V polypeptides are formulated generally by mixing at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation. For example, the formulation preferably does not include oxidizing agents and other compounds that are known to be deleterious to polypeptides.

Generally, the formulations are prepared by contacting the FcR-I, FcR-II, FcR-III, FcR-IV, and FcR-V polypeptide uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non-aqueous vehicles such as fixed oils and ethyl oleate are also useful herein, as well as liposomes.

The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) polypeptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, manose, or dextrins; chelating agents such as EDTA: sugar alcohols such as mannitol or sorbitol; counterions such as sodium; and/or nonionic surfactants such as polysorbates, poloxamers, or PEG.

The FcR-I, FcR-II, FcR-III, FcR-IV, and FcR-V polypeptides are typically formulated in such vehicles at a concentration of about 0.1 mg/ml to 100 mg/ml, preferably 1-10 mg/ml, at a pH of about 3 to 8. It will be understood that the use of certain of the foregoing excipients, carriers, or stabilizers will result in the formation of FcR-I, FcR-II, FcR-III, FcR-IV, and FcR-V polypeptide salts.

FcR-I, FcR-II, FcR-III, FcR-IV, and FcR-V polypeptides to be used for therapeutic administration must be sterile. Sterility is readily accomplished by filtration through sterile filtration membranes (e.g., 0.2 micron membranes). Therapeutic FcR-I, FcR-II, FcR-III, FcR-IV, and FcR-V polypeptide compositions generally are placed into a container having a sterile access port, for example, an intravenous solution bag or vial having a stopper pierceable by a hypodermic injection needle.

FcR-I, FcR-II, FcR-III, FcR-IV, and FcR-V polypeptides ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous FcR-I, FcR-II, FcR-III. FcR-IV, and FcR-V polypeptide solution, and the resulting mixture is lyophilized. The infusion solution is prepared by reconstituting the lyophilized FcR-I, FcR-II, FcR-III. FcR-IV. and FcR-V polypeptides using bacteriostatic Water-for-Injection.

The invention also provides a pharmaceutical pack or kit comprising one or more containers filled with one or more of the ingredients of the pharmaceutical compositions of the invention. Associated with such container(s) can be a notice in the form prescribed by a governmental agency regulating the manufacture, use or sale of pharmaceuticals or biological products, which notice reflects approval by the agency of manufacture, use or sale for human administration. In addition, the polypeptides of the present invention may be employed in conjunction with other therapeutic compounds.

### Agonists and Antagonists -Assays and Molecules

The invention also provides a method of screening compounds to identify those which enhance or block the action of FcR-I, FcR-II, FcR-III, FcR-IV, and FcR-V on cells, such as its interaction with FcR-I, FcR-II, FcR-III, FcR-IV, and FcR-V-binding molecules such as ligand molecules or the Fc portion of an antibody. An agonist is a compound which increases the natural biological functions of FcR-I, FcR-II, FcR-III, FcR-IV, or FcR-V or which functions in a manner similar to FcR-I, FcR-II, FcR-III, FcR-IV, or FcR-V, while antagonists decrease or eliminate such functions.

In another aspect of this embodiment the invention provides a method for identifying a receptor protein or other ligand-binding protein which binds specifically to a FcR-I, FcR-II, FcR-III, FcR-IV, or FcR-V polypeptide. For example, a cellular compartment, such as a membrane or a preparation thereof, may be prepared from a cell that expresses a molecule that binds FcR-I, FCR-II, FcR-III, FcR-IV, or FcR-V. The preparation is incubated with labeled FcR-I, FcR-II, FcR-III, FcR-IV, or FcR-V and complexes of FcR-I, FcR-II, FcR-III, FcR-IV, or FcR-V bound to the receptor or other binding protein are isolated and characterized according to routine methods known in the art. Alternatively, the FcR-I, FcR-II, FcR-III, FcR-IV, or FcR-V polypeptide may be bound to a solid support so that binding molecules solubilized from cells are bound to the column and then eluted and characterized according to routine methods.

In the assay of the invention for agonists or antagonists, a cellular compartment, such as a membrane or a preparation thereof, may be prepared from a cell that expresses a molecule that binds FcR-I, FcR-II, FcR-III, FcR-IV, or FcR-V, such as a molecule of a signaling or regulatory pathway modulated by FcR-I, FcR-II, FcR-III, FcR-IV. or FcR-V. The preparation is incubated with labeled FcR-I, FcR-II, FcR-III, FcR-IV, or FcR-V in the absence or the presence of a candidate molecule which may be a FcR-I, FcR-II, FcR-III, FcR-IV, or FcR-V agonist or antagonist. The ability of the candidate molecule to bind the binding molecule is reflected in decreased binding of the labeled ligand. Molecules which bind gratuitously, i.e.. without inducing the effects of FcR-I, FcR-II, FcR-III, FcR-IV, or FcR-V on binding the FcR-I, FcR-II, FcR-III, FcR-IV, or FcR-V binding molecule, are most likely to be good antagonists. Molecules that bind well and elicit effects that are the same as or closely related to FcR-I, FcR-II, FcR-III, FcR-IV, and FcR-V are agonists.

FcR-I, FcR-II, FcR-III, FcR-IV, or FcR-V-like effects of potential agonists and antagonists may by measured, for instance, by determining activity of a second messenger system following interaction of the candidate molecule with a cell or appropriate cell preparation, and comparing the effect with that of FcR-I, FcR-II, FcR-III, FcR-IV, or FcR-V or molecules that elicit the same effects as FcR-I, FcR-II, FcR-III, FcR-IV, or FcR-V. Second messenger systems that may be useful in this regard include but are not limited to AMP guanylate cyclase, ion channel or phosphoinositide hydrolysis second messenger systems.

Another example of an assay for FcR-I, FcR-II, FcR-III, FcR-IV, and FcR-V antagonists is a competitive assay that combines FcR-I, FcR-II, FcR-III, FcR-IV, and FcR-V and a potential antagonist with membrane-bound FcR-I, FcR-II, FcR-III, FcR-IV, or FcR-V receptor molecules or recombinant FcR-I, FcR-II, FcR-III, FcR-IV, or FcR-V receptor molecules under appropriate conditions for a competitive inhibition assay. FcR-I, FcR-II, FcR-III, FcR-IV, or FcR-V can be labeled, such as by radioactivity, such that the number of FcR-I, FcR-II, FcR-III, FcR-IV, or FcR-V molecules bound to a receptor molecule can be determined accurately to assess the effectiveness of the potential antagonist.

Potential antagonists include small organic molecules, peptides, polypeptides and antibodies that bind to a polypeptide of the invention and thereby inhibit or extinguish its activity. Potential antagonists also may be small organic molecules, a peptide, a polypeptide such as a closely related protein or antibody that binds the same sites on a binding molecule, such as a receptor molecule, without inducing FcR-I, FcR-II, FcR-III, FcR-IV, or FcR-V- induced activities, thereby preventing the action of FcR-I, FcR-II, FcR-III, FcR-IV, or FcR-V by excluding FcR-I, FcR-II, FcR-III, FcR-IV, or FcR-V from binding.

Other potential antagonists include antisense molecules. Antisense technology can be used to control gene expression through antisense DNA or RNA or through triple-helix formation. Antisense techniques are discussed, for example, by Okano (J. Neurochem. 56:560; 1991)_{:} Triple helix formation is discussed in the literature (Lee, et al., (1979) Nucleic Acids Research 6:3073; Cooney, et al., (1988) Science 241:456; and Dervan, et al., (1991) Science 251:1360). The methods are based on binding of a polynucleotide to a complementary DNA or RNA. For example, the 5' coding portion of a polynucleotide that encodes the mature polypeptide of the present invention may be used to design an antisense RNA oligonucleotide of from about 10 to 40 base pairs in length. A DNA oligonucleotide is designed to be complementary to a region of the gene involved in transcription thereby preventing transcription and the production of FcR-I, FcR-II, FcR-III, FcR-IV, and FcR-V. The antisense RNA oligonucleotide hybridizes to the mRNA *in vivo* and blocks translation of the mRNA molecule into FcR-I, FcR-II, FcR-III, FcR-IV, and FcR-V polypeptides. The oligonucleotides described above can also be delivered to cells such that the antisense RNA or DNA may be expressed *in vivo* to inhibit production of FcR-I, FcR-II, FcR-III, FcR-IV, and FcR-V proteins.

The agonists and antagonists may be employed in a composition with a pharmaceutically acceptable carrier, e.g., as described above. The antagonists may be employed for instance to inhibit the ability of an FcR-like polypeptide to mediate ADCC, phagocytosis, and the release of inflammatory mediators such as cytokines and prostaglandins. Antibodies against FcR-I, FcR-II, FcR-III, FcR-IV, or FcR-V may be employed to bind to and inhibit FcR-I, FcR-II, FcR-III, FcR-IV, or FcR-V activity to treat cancers (including colorectal cancer, breast cancer, leukemia, and Hodgekin's Lymphoma and other lymphomas), autoimmune disorders (including systemic lupus erythematosus (SLE), autoimmune hemolytic anemia (AIHA), idiopathic thrombocytopenia purpura (ITP)), and infectious diseases (including toxoplasmosis, HIV, Dengue Virus, and other viral and bacterial infections). Any of the above antagonists may be employed in a composition with a pharmaceutically acceptable carrier, e.g., as hereinafter described.

### Gene Mapping

The nucleic acid molecules of the present invention are also valuable for chromosome identification. The sequence is specifically targeted to and can hybridize with a particular location on an individual human chromosome. Moreover, there is a current need for identifying particular sites on the chromosome. Few chromosome marking reagents based on actual sequence data (repeat polymorphisms) are presently available for marking chromosomal location. The mapping of DNAs to chromosomes according to the present invention is an important first step in correlating those sequences with genes associated with disease.

In certain preferred embodiments in this regard, the cDNAs herein disclosed are used to clone genomic DNA of FcR-I, FcR-II, FcR-III, FcR-IV, and FcR-V protein genes. This can be accomplished using a variety of well known techniques and libraries, which generally are available commercially. The genomic DNA then is used for *in situ* chromosome mapping using well known techniques for this purpose.

In addition, in some cases, sequences can be mapped to chromosomes by preparing PCR primers (preferably 15-25 bp) from the cDNA. Computer analysis of the 3' untranslated region of the gene is used to rapidly select primers that do not span more than one exon in the genomic DNA, thus complicating the amplification process. These primers are then used for PCR screening of somatic cell hybrids containing individual human chromosomes. Fluorescence *in situ* hybridization ("FISH") of a cDNA clone to a metaphase chromosomal spread can be used to provide a precise chromosomal location in one step. This technique can be used with probes from the cDNA as short as 50 or 60 bp (for review, see Verma et al., Human Chromosomes: A Manual Of Basic Techniques, Pergamon Press, New York; 1988).

Once a sequence has been mapped to a precise chromosomal location, the physical position of the sequence on the chromosome can be correlated with genetic map data (such data are found, for example, in McKusick, V., *Mendelian Inheritance In Man,* available on-line through Johns Hopkins University, Welch Medical Library). The relationship between genes and diseases that have been mapped to the same chromosomal region are then identified through linkage analysis (coinheritance of physically adjacent genes).

Next, it is necessary to determine the differences in the cDNA or genomic sequence between affected and unaffected individuals. If a mutation is observed in some or all of the affected individuals but not in any normal individuals, then the mutation is likely to be the causative agent of the disease.

Having generally described the invention, the same will be more readily understood by reference to the following examples, which are provided by way of illustration and are not intended as limiting.

### Examples

### Example 1: Expression and Purification of "His-tagged" FcR proteins in E. coli

The bacterial expression vector pQE70 is used for bacterial expression in this example (QIAGEN, Inc., 9259 Eton Avenue, Chatsworth, CA, 91311). pQE70 encodes ampicillin antibiotic resistance ("Ampr") and contains a bacterial origin of replication ("ori"), an IPTG inducible promoter, a ribosome binding site ("RBS"), six codons encoding histidine residues that allow affinity purification using nickel-nitrilo-tri-acetic acid ("Ni-NTA") affinity resin sold by QIAGEN, Inc., *supra,* and suitable single restriction enzyme cleavage sites. These elements are arranged such that an inserted DNA fragment encoding a polypeptide expresses that polypeptide with the six His residues (i.e., a "6 X His tag") covalently linked to the carboxy terminus of that polypeptide.

The DNA sequence encoding the desired portion of the FcR-I protein comprising the mature form of the FcR-I amino acid sequence is amplified from the deposited cDNA clone using PCR oligonucleotide primers which anneal to the amino terminal sequences of the desired portion of the FcR-I protein and to sequences in the deposited construct 3' to the cDNA coding sequence. Additional nucleotides containing restriction sites to facilitate cloning in the pQE70 vector are added to the 5' and 3' primer sequences, respectively.

For cloning the mature form of the FcR-I protein, the 5' primer has the sequence 5' GACTCATGACTGAGCCAGGCTCTGTGATCACCC 3' (SEQ ID NO: 25) containing the underlined *Bsp HI* restriction site containing and followed by 24 nucleotides of the amino terminal coding sequence of the mature FcR-I sequence in SEQ ID NO:2. One of ordinary skill in the art would appreciate, of course, that the point in the protein coding sequence where the 5' primer begins may be varied to amplify a DNA segment encoding any desired portion of the complete FcR-I protein shorter or longer than the mature form of the protein. The 3' primer has the sequence 5' GACAGATCTCTCACCAGCCTTGGAGTC 3' (SEQ ID NO:26) containing the underlined *Bgl II* restriction site followed by 18 nucleotides complementary to the 3' end of the coding sequence of the FcR-I DNA sequence in Figure 1A.

The amplified FcR-I DNA fragments are digested with *Bsp* HI and *Bgl* II, the vector pQE70 is digested with Sph I and Bgl II, and the digested DNAs are then ligated together. Insertion of the FcR-I DNA into the restricted pQE70 vector places the FcR-I protein coding region downstream from the IPTG-inducible promoter and in-frame with an initiating AUG and the six histidine codons.

The skilled artisan appreciates that a similar approach could easily be designed and utilized to generate pQE70-based bacterial expression constructs for the expression of FcR-II. FcR-III, FcR-IV, and FcR-V protein in *E. coli.* This would be done by designing PCR primers containing similar restriction endonuclease recognition sequences combined with gene-specific sequences for FcR-II, FcR-III, FcR-IV, and FcR-V and proceeding as described above.

The bacterial expression vector pQE70 (QIAGEN, Inc., 9259 Eton Avenue, Chatsworth, CA, 91311), used in the construction of the above-described plasmids, encodes ampicillin antibiotic resistance ("Ampr"), and contains a bacterial origin of replication ("ori"), an IPTG inducible promoter, a ribosome binding site ("RBS"), six codons encoding histidine residues that allow affinity purification using nickel-nitrilo-tri-acetic acid ("Ni-NTA") affinity resin sold by QIAGEN. Inc., *supra,* and suitable single restriction enzyme cleavage sites. These elements are arranged such that an inserted DNA fragment encoding a polypeptide expresses that polypeptide with the six His residues (i.e.. a "6 X His tag") covalently linked to the carboxyl terminus of that polypeptide.

The ligation mixture is transformed into competent *E. coli* cells using standard procedures such as those described in Sambrook et al., Molecular Cloning: a Laboratory Manual, 2nd Ed.: Cold Spring Harbor Laboratory Press. Cold Spring Harbor. NY (1989). *E. coli* strain M15/rep4, containing multiple copies of the plasmid pREP4, which expresses the lac repressor and confers kanamycin resistance ("Kanr"), is used in carrying out the illustrative example described herein. This strain, which is only one of many that are suitable for expressing FcR-I. FcR-II. FcR-III. FcR-IV. or FcR-V protein, is available commercially from QIAGEN. Inc., *supra*. Transformants are identified by their ability to grow on LB plates in the presence of ampicillin and kanamycin. Plasmid DNA is isolated from resistant colonies and the identity of the cloned DNA confirmed by restriction analysis. PCR and DNA sequencing.

Clones containing the desired constructs are grown overnight ("O/N") in liquid culture in LB media supplemented with both ampicillin (100 µg/ml) and kanamycin (25 µg/ml). The O/N culture is used to inoculate a large culture, at a dilution of approximately 1:25 to 1:250. The cells are grown to an optical density at 600 nm ("OD600") of between 0.4 and 0.6. Isopropyl-β-D-thiogalactopyranoside ("IPTG") is then added to a final concentration of 1 mM to induce transcription from the lac repressor sensitive promoter, by inactivating the lacI repressor. Cells subsequently are incubated further for 3 to 4 hours. Cells then are harvested by centrifugation.

The cells are then stirred for 3-4 hours at 4° C in 6M guanidine-HCl, pH 8. The cell debris is removed by centrifugation, and the supernatant containing the FcR-I, FcR-II. FcR-III, FcR-IV, or FcR-V is loaded onto a nickel-nitrilo-tri-acetic acid ("Ni-NTA") affinity resin column (available from QIAGEN, Inc., *supra*). Proteins with a 6 x His tag bind to the Ni-NTA resin with high affinity and can be purified in a simple one-step procedure (for details see: The QIAexpressionist, 1995, QIAGEN, Inc., *supra*). Briefly the supernatant is loaded onto the column in 6 M guanidine-HCl, pH 8, the column is first washed with 10 volumes of 6 M guanidine-HCl, pH 8, then washed with 10 volumes of 6 M guanidine-HCl pH 6, and finally the FcR-I, FcR-II, FcR-III, FcR-IV, or FcR-V is eluted with 6 M guanidine-HCl, pH 5.

The purified protein is then renatured by dialyzing it against phosphate-buffered saline (PBS) or 50 mM Na-acetate, pH 6 buffer plus 200 mM NaCl. Alternatively, the protein can be successfully refolded while immobilized on the Ni-NTA column. The recommended conditions are as follows: renature using a linear 6M-1M urea gradient in 500 mM NaCl, 20% glycerol. 20 mM Tris/HCl pH 7.4. containing protease inhibitors. The renaturation should be performed over a period of 1.5 hours or more. After renaturation the proteins can be eluted by the addition of 250 mM immidazole. Immidazole is removed by a final dialyzing step against PBS or 50 mM sodium acetate pH 6 buffer plus 200 mM NaCl. The purified protein is stored at 4°C or frozen .at -80° C.

The following alternative method may be used to purify FcR-I, FcR-II, FcR-III, FcR-IV, or FcR-V expressed in *E coli* when it is present in the form of inclusion bodies. Unless otherwise specified, all of the following steps are conducted at 4-10°C.

Upon completion of the production phase of the *E. coli* fermentation, the cell culture is cooled to 4-10°C and the cells are harvested by continuous centrifugation at 15,000 rpm (Heraeus Sepatech). On the basis of the expected yield of protein per unit weight of cell paste and the amount of purified protein required, an appropriate amount of cell paste, by weight, is suspended in a buffer solution containing 100 mM Tris, 50 mM EDTA, pH 7.4. The cells are dispersed to a homogeneous suspension using a high shear mixer.

The cells ware then lysed by passing the solution through a microfluidizer (Microfuidics, Corp. or APV Gaulin, Inc.) twice at 4000-6000 psi. The homogenate is then mixed with NaCl solution to a final concentration of 0.5 M NaCl, followed by centrifugation at 7000 xg for 15 min. The resultant pellet is washed again using 0.5M NaCl, 100 mM Tris, 50 mM EDTA, pH 7.4.

The resulting washed inclusion bodies are solubilized with 1.5 M guanidine hydrochloride (GuHCl) for 2-4 hours. After 7000 xg centrifugation for 15 min.. the pellet is discarded and the FcR-I, FcR-II, FcR-III, FcR-IV, or FcR-V polypeptide-containing supernatant is incubated at 4°C overnight to allow further GuHCl extraction.

Following high speed centrifugation (30,000 xg) to remove insoluble particles, the GuHCl solubilized protein is refolded by quickly mixing the GuHCl extract with 20 volumes of buffer containing 50 mM sodium, pH 4.5,150 mM NaCl, 2 mM EDTA by vigorous stirring. The refolded diluted protein solution is kept at 4°C without mixing for 12 hours prior to further purification steps.

To clarify the refolded FcR-I, FcR-II, FcR-III, FcR-IV, or FcR-V polypeptide solution, a previously prepared tangential filtration unit equipped with 0.16 µm membrane filter with appropriate surface area (e.g., Filtron), equilibrated with 40 mM sodium acetate, pH 6.0 is employed. The filtered sample is loaded onto a cation exchange resin (e.g., Poros HS-50, Perseptive Biosystems). The column is washed with 40 mM sodium acetate, pH 6.0 and eluted with 250 mM, 500 mM, 1000 mM. and 1500 mM NaCl in the same buffer, in a stepwise manner. The absorbance at 280 mm of the effluent is continuously monitored. Fractions are collected and further analyzed by SDS-PAGE.

Fractions containing the FcR-I, FcR-II, FcR-III, FcR-IV, or FcR-V polypeptide are then pooled and mixed with 4 volumes of water. The diluted sample is then loaded onto a previously prepared set of tandem columns of strong anion (Poros HQ-50. Perseptive Biosystems) and weak anion (Poros CM-20, Perseptive Biosystems) exchange resins. The columns are equilibrated with 40 mM sodium acetate, pH 6.0. Both columns are washed with 40 mM sodium acetate, pH 6.0, 200 mM NaCl. The CM-20 column is then eluted using a 10 column volume linear gradient ranging from 0.2 M NaCl, 50 mM sodium acetate, pH 6.0 to 1.0 M NaCl. 50 mM sodium acetate, pH 6.5. Fractions are collected under constant A₂₈₀ monitoring of the effluent. Fractions containing the FcR-I, FcR-II, FcR-III, FcR-IV, or FcR-V polypeptide (determined, for instance, by 16% SDS-PAGE) are then pooled.

The resultant FcR-I, FcR-II, FcR-III. FcR-IV. or FcR-V polypeptide exhibits greater than 95% purity after the above refolding and purification steps. No major contaminant bands are observed from Commassie blue stained 16% SDS-PAGE gel when 5 µg of purified protein is loaded. The purified protein is also tested for endotoxin/LPS contamination, and typically the LPS content is less than 0.1 ng/ml according to LAL assays.

### Example 2: Cloning and Expression of FcR proteins in a Baculovirus Expression System

In this illustrative example, the plasmid shuttle vector pA2GP is used to insert the cloned DNA encoding the mature protein, lacking its naturally associated secretory signal (leader) sequence, into a baculovirus to express the mature FcR-I, FcR-II, FcR-III, FcR-IV, and FcR-V proteins using a baculovirus leader and standard methods as described in Summers et al., A Manual of Methods for Baculovirus Vectors and Insect Cell Culture Procedures, Texas Agricultural Experimental Station Bulletin No. 1555 (1987). This expression vector contains the strong polyhedrin promoter of the *Autographa californica* nuclear polyhedrosis virus (AcMNPV) followed by the secretory signal peptide (leader) of the baculovirus gp67 protein and convenient restriction sites such as *Bam* HI, *Xba I* and *Asp* 718. The polyadenylation site of the simian virus 40 ("SV40") is used for efficient polyadenylation. For easy selection of recombinant virus, the plasmid contains the beta-galactosidase gene from *E. coli* under control of a weak Drosophila promoter in the same orientation, followed by the polyadenylation signal of the polyhedrin gene. The inserted genes are flanked on both sides by viral sequences for cell-mediated homologous recombination with wild-type viral DNA to generate viable virus that expresses the cloned polynucleotide.

Many other baculovirus vectors could be used in place of the vector above, such as pAc373. pVL941 and pAcIM1, as one skilled in the art would readily appreciate, as long as the construct provides appropriately located signals for transcription, translation, secretion and the like, including a signal peptide and an in-frame AUG as required. Such vectors are described, for instance, by Luckow and colleagues (*Virology* **170:**31-39; 1989). The cDNA sequence encoding the mature FcR-I protein in the deposited clone, lacking the AUG initiation codon and the naturally associated leader sequence shown in SEQ ID NO:2 is amplified using PCR oligonucleotide primers corresponding to the 5' and 3' sequences of the gene. The 5' primer has the sequence 5' GACAGATCTGAGCCAGGCTCTGTGATCACCC 3' (SEQ ID NO:27) containing the underlined *Bgl II* restriction enzyme site followed by 22 nucleotides of the sequence of the mature FcR-I protein shown in SEQ ID NO:2, beginning with the indicated N-terminus of the mature form of the FcR-I protein. The 3' primer has the sequence 5 GACTCTAGAGTCCACCCAGGACACCCA GC 3' (SEQ ID NO:28) containing the underlined *Xba I* restriction site followed by 18 nucleotides complementary to the 3' coding sequence in Figure 1A.

The skilled artisan appreciates that a similar approach could easily be designed and utilized to generate pA2GP-based bacterial expression constructs for the expression of FcR-II, FcR-III, FcR-IV, and FcR-V protein by baculovirus. This would be done by designing PCR primers containing the same restriction endonuclease recognition sequences combined with gene-specific sequences for FcR-II, FcR-III, FcR-IV, and FcR-V and proceeding as described above.

The amplified fragment is isolated from a 1% agarose gel using a commercially available kit ("Geneclean," BIO 101 Inc., La Jolla, Ca.). The fragment then is digested with *Bgl II* and *Xba I* and again is purified on a I % agarose gel. This fragment is designated herein F1.

The plasmid is digested with the restriction enzymes *Bgl II* and *Xba I* and optionally, can be dephosphorylated using calf intestinal phosphatase, using routine procedures known in the art. The DNA is then isolated from a 1% agarose gel using a commercially available kit ("Geneclean" BIO 101 Inc.. La Jolla. Ca.). This vector DNA is designated herein "V1".

Fragment F1 and the dephosphorylated plasmid V1 are ligated together with T4 DNA ligase. *E. coli* HB101 or other suitable *E. coli* hosts such as XL-1 Blue (Statagene Cloning Systems. La Jolla, CA) cells are transformed with the ligation mixture and spread on culture plates. Bacteria are identified that contain the plasmid with the human FcR-I gene by digesting DNA from individual colonies using *Bgl II* and *Xba I* and then analyzing the digestion product by gel electrophoresis. The sequence of the cloned fragment is confirmed by DNA sequencing. This plasmid is designated herein pA2GPFcR-I.

Five µg of the plasmid pA2GPFcR-I is co-transfected with 1.0 µg of a commercially available linearized baculovirus DNA ("BaculoGold^{™} baculovirus DNA". Pharmingen, San Diego, CA), using the lipofection method described by Felgner et al., Proc. Natl. Acad Sci. USA 84: 7413-7417 (1987). One µg of BaculoGold^{™} virus DNA and 5 µg of the plasmid pA2GPFcR-I are mixed in a sterile well of a microtiter plate containing 50 µl of serum-free Grace's medium (Life Technologies Inc.. Gaithersburg, MD). Afterwards. 10 µl Lipofectin plus 90 µl Grace's medium are added, mixed and incubated for 15 minutes at room temperature. Then the transfection mixture is added drop-wise to Sf9 insect cells (ATCC CRL 1711) seeded in a 35 mm tissue culture plate with 1 ml Grace's medium without serum. The plate is then incubated for 5 hours at 27° C. The transfection solution is then removed from the plate and 1 ml of Grace's insect medium supplemented with 10% fetal calf serum is added. Cultivation is then continued at 27° C for four days.

After four days the supernatant is collected and a plaque assay is performed, as described by Summers and Smith, *supra.* An agarose gel with "Blue Gal" (Life Technologies Inc., Gaithersburg) is used to allow easy identification and isolation of gal-expressing clones, which produce blue-stained plaques. (A detailed description of a "plaque assay" of this type can also be found in the user's guide for insect cell culture and baculovirology distributed by Life Technologies Inc., Gaithersburg, page 9-10). After appropriate incubation, blue stained plaques are picked with the tip of a micropipettor (e.g., Eppendorf). The agar containing the recombinant viruses is then resuspended in a microcentrifuge tube containing 200 µl of Grace's medium and the suspension containing the recombinant baculovirus is used to infect Sf9 cells seeded in 35 mm dishes. Four days later the supernatants of these culture dishes are harvested and then they are stored at 4° C. The recombinant virus is called V-FcR-I.

To verify the expression of the FcR-I gene Sf9 cells are grown in Grace's medium supplemented with 10% heat-inactivated FBS. The cells are infected with the recombinant baculovirus V-FcR-I at a multiplicity of infection ("MOI") of about 2. If radiolabeled proteins are desired, 6 hours later the medium is removed and is replaced with SF900 II medium minus methionine and cysteine (available from Life Technologies Inc., Rockville, MD). After 42 hours, 5 µCi of ³⁵S-methionine and 5 µCi ³⁵S-cysteine (available from Amersham) are added. The cells are further incubated for 16 hours and then are harvested by centrifugation. The proteins in the supernatant as well as the intracellular proteins are analyzed by SDS-PAGE followed by autoradiography (if radiolabeled).

Microsequencing of the amino acid sequence of the amino terminus of purified protein may be used to determine the amino terminal sequence of the mature form of the FcR-I protein.

### Example 3: Cloning and Expression of FcR proteins in Mammalian Cells

A typical mammalian expression vector contains the promoter element, which mediates the initiation of transcription of mRNA. the protein coding sequence, and signals required for the termination of transcription and polyadenylation of the transcript. Additional elements include enhancers, Kozak sequences and intervening sequences flanked by donor and acceptor sites for RNA splicing. Highly efficient transcription can be achieved with the early and late promoters from SV40, the long terminal repeats (LTRs) from Retroviruses, e.g., RSV, HTLVI, HIVI and the early promoter of the cytomegalovirus (CMV). However, cellular elements can also be used (e.g., the human actin promoter). Suitable expression vectors for use in practicing the present invention include, for example, vectors such as pSVL and pMSG (Pharmacia, Uppsala. Sweden), pRSVcat (ATCC 37152), pSV2dhfr (ATCC 37146) and pBC12MI (ATCC 67109). Mammalian host cells that could be used include, human Hela, 293, H9 and Jurkat cells, mouse NIH3T3 and C127 cells, Cos 1, Cos 7 and CV1, quail QC1-3 cells, mouse L cells and Chinese hamster ovary (CHO) cells.

Alternatively, the gene can be expressed in stable cell lines that contain the gene integrated into a chromosome. The co-transfection with a selectable marker such as dhfr, gpt, neomycin, hygromycin allows the identification and isolation of the transfected cells.

The transfected gene can also be amplified to express large amounts of the encoded protein. The DHFR (dihydrofolate reductase) marker is useful to develop cell lines that carry several hundred or even several thousand copies of the gene of interest. Another useful selection marker is the enzyme glutamine synthase (GS) (Murphy et al., Biochem J. 227:277-279 (1991); Bebbington et al., Bio/Technology 10:169-175 (1992)). Using these markers, the mammalian cells are grown in selective medium and the cells with the highest resistance are selected. These cell lines contain the amplified gene(s) integrated into a chromosome. Chinese hamster ovary (CHO) and NSO cells are often used for the production of proteins.

The expression vectors pC 1 and pC4 contain the strong promoter (LTR) of the Rous Sarcoma Virus (Cullen et al., Molecular and Cellular Biology, 438-447 (March, 1985)) plus a fragment of the CMV-enhancer (Boshart et al., Cell 41:521-530 (1985)). Multiple cloning sites, e.g., with the restriction enzyme cleavage sites. *Bam* HI*, Xba* I and *Asp* 718, facilitate the cloning of the gene of interest. The vectors contain in addition the 3' intron, the polyadenylation and termination signal of the rat preproinsulin gene.

The skilled artisan appreciates that a similar approach could easily be designed and utilized to generate eukaryotic expression constructs for the expression of FcR-II. FcR-III. FcR-IV, and FcR-V protein in COS or CHO cells. This would be done by designing PCR primers containing the same restriction endonuclease recognition sequences combined with gene-specific sequences for FcR-II, FcR-III, FcR-IV, and FcR-V and proceeding as described below.

### Example 3(a): Cloning and Expression in COS Cells

The expression plasmid, pFcR-IHA, is made by cloning a portion of the cDNA encoding the mature form of the FcR-I protein into the expression vector pcDNAI/Amp or pcDNAIII (which can be obtained from Invitrogen, Inc.).

The expression vector pcDNAI/amp contains: (1) an *E. coli* origin of replication effective for propagation in *E. coli* and other prokaryotic cells; (2) an ampicillin resistance gene for selection of plasmid-containing prokaryotic cells; (3) an SV40 origin of replication for propagation in eukaryotic cells; (4) a CMV promoter, a polylinker, an SV40 intron; (5) several codons encoding a hemagglutinin fragment (i.e., an "HA" tag to facilitate purification) followed by a termination codon and polyadenylation signal arranged so that a cDNA can be conveniently placed under expression control of the CMV promoter and operably linked to the SV40 intron and the polyadenylation signal by means of restriction sites in the polylinker. The HA tag corresponds to an epitope derived from the influenza hemagglutinin protein described by Wilson et al., Cell 37: 767 (1984). The fusion of the HA tag to the target protein allows easy detection and recovery of the recombinant protein with an antibody that recognizes the HA epitope, pcDNAIII contains, in addition, the selectable neomycin marker.

A DNA fragment encoding the complete FcR-I polypeptide is cloned into the polylinker region of the vector so that recombinant protein expression is directed by the CMV promoter. The plasmid construction strategy is as follows. The FcR-I cDNA of the deposited clone is amplified using primers that contain convenient restriction sites, much as described above for construction of vectors for expression of FcR-I *in E. coli.* Suitable primers include the following, which are used in this example. The 5' primer, containing the underlined *Bgl* II site, a Kozak sequence, an AUG start codon, a sequence encoding the secretory leader peptide from the human IL-6 gene, and 22 nucleotides of the 5' coding region of the mature FcR-I polypeptide, has the following sequence:
5' CTAGCCAGATCTGCCACCATGAACTCCTTCTCCACAAGCGCCTTCGGTCCAGTT GCCTTCTCCCTGGGGCTGCTCCTGGTGTTGCCTGCTGCCTTCCCTGCCCCAGTTGTG AGAGAGCCAGGCTCTGTGATCACCC 3' (SEQ ID NO:29). The 3' primer. containing the underlined *Xho* I and 18 nucleotides complementary to the 3' coding sequence immediately before the stop codon, has the following sequence:
5' CAGCTCGAGCTCACCAGCCTTGGAG TC 3' (SEQ ID NO:30).

The PCR amplified DNA fragment and the vector, pcDNAI/Amp, are digested with *Bgl* II and *Xho* I and then ligated. The ligation mixture is transformed into *E. coli* strain SURE (available from Stratagene Cloning Systems, 11099 North Torrey Pines Road, La Jolla, CA 92037), and the transformed culture is plated on ampicillin media plates which then are incubated to allow growth of ampicillin resistant colonies. Plasmid DNA is isolated from resistant colonies and examined by restriction analysis or other means for the presence of the fragment encoding the complete FcR-I polypeptide.

For expression of recombinant FcR-I protein, COS cells are transfected with an expression vector, as described above, using DEAE-DEXTRAN, as described, for instance, by Sambrook and colleagues (Molecular Cloning: a Laboratory Manual, Cold Spring Laboratory Press, Cold Spring Harbor, New York (1989)). Cells are incubated under conditions for expression of FcR-I protein by the vector.

Expression of the FcR-I-HA fusion protein is detected by radiolabeling and immunoprecipitation, using methods described in, for example Harlow et al., Antibodies: A Laboratory Manual, 2nd Ed.; Cold Spring Harbor Laboratory Press, Cold Spring Harbor. New York (1988). To this end, two days after transfection, the cells are labeled by incubation in media containing ³⁵S-cysteine for 8 hours. The cells and the media are collected, and the cells are washed and the lysed with detergent-containing RIPA buffer: 150 mM NaCl, 1% NP-40. 0.1% SDS, 1% NP-40, 0.5% DOC. 50 mM TRIS, pH 7.5. as described by Wilson et al. cited above. Proteins are precipitated from the cell lysate and from the culture media using an HA-specific monoclonal antibody. The precipitated proteins then are analyzed by SDS-PAGE and autoradiography. An expression product of the expected size is seen in the cell lysate, which is not seen in negative controls.

### Example 3(b): Cloning and Expression in CHO Cells

The vector pC4 is used for the expression of FcR-I polypeptide. Plasmid pC4 is a derivative of the plasmid pSV2-dhfr (ATCC Accession No. 37146). To produce a soluble, secreted form of the polypeptide, the complete form is fused to the secretory leader sequence of the human IL-6 gene. The plasmid contains the mouse DHFR gene under control of the SV40 early promoter. Chinese hamster ovary (CHO) or other cells lacking dihydrofolate activity that are transfected with these plasmids can be selected by growing the cells in a selective medium (alpha minus MEM, Life Technologies) supplemented with the chemotherapeutic agent methotrexate. The amplification of the DHFR genes in cells resistant to methotrexate (MTX) has been well documented (see, e.g., Alt, F. W., Kellems. R. M., Bertino, J. R., and Schimke, R. T., 1978, J. Biol. Chem. 253:1357-1370; Hamlin, J. L. and Ma. C. 1990, Biochem. et Biophys. Acta, 1097:107-143, Page, M. J. and Sydenham, M. A. 1991. Biotechnology 9:64-68). Cells grown in increasing concentrations of MTX develop resistance to the drug by overproducing the target enzyme, DHFR, as a result of amplification of the DHFR gene. If a second gene is linked to the DHFR gene, it is usually co-amplified and over-expressed. It is known in the art that this approach may be used to develop cell lines carrying more than 1,000 copies of the amplified gene(s). Subsequently, when the methotrexate is withdrawn, cell lines are obtained which contain the amplified gene integrated into one or more chromosome(s) of the host cell.

Plasmid pC4 contains for expressing the gene of interest the strong promoter of the long terminal repeat (LTR) of the Rouse Sarcoma Virus (Cullen, et al., Mol. Cell. Biol. 1985:438-447) plus a fragment isolated from the enhancer of the immediate early gene of human cytomegalovirus (CMV) (Boshart et al., Cell 41:521-530 (1985)). Downstream of the promoter are the following single restriction enzyme cleavage sites that allow the integration of the genes: BamHI, *Xbu I*, and *Asp718*. Behind these cloning sites the plasmid contains the 3' intron and polyadenylation site of the rat preproinsulin gene. Other high efficiency promoters can also be used for the expression, e.g., the human βß-actin promoter, the SV40 early or late promoters or the long terminal repeats from other retroviruses, e.g., HIV and HTLVI. Clontech's Tet-Off and Tet-On gene expression systems and similar systems can be used to express the FcR-I polypeptide in a regulated way in mammalian cells (Gossen, M., & Bujard, H. 1992. Proc. Natl. Acad. Sci. USA 89:5547-5551). For the polyadenylation of the mRNA other signals, e.g., from the human growth hormone or globin genes can be used as well. Stable cell lines carrying a gene of interest integrated into the chromosomes can also be selected upon co-transfection with a selectable marker such as gpt. G418 or hygromycin. It is advantageous to use more than one selectable marker in the beginning, e.g., G418 plus methotrexate.

The plasmid pC4 is digested with the restriction enzymes *Bam HI* and *Xba I* and then dephosphorylated using calf intestinal phosphates by procedures known in the art. The vector is then isolated from a 1% agarose gel.

The DNA sequence encoding the complete FcR-I polypeptide is amplified using PCR oligonucleotide primers corresponding to the 5' and 3' sequences of the desired portion of the gene. The 5' primer containing the underlined *Bam* HI site, a Kozak sequence, an AUG start codon, a sequence encoding the secretory leader peptide from the human IL-6 gene, and 20 nucleotides of the 5' coding region of the mature FcR-I polypeptide, has the following sequence (where Kozak is in italics):
5' CTAGCCGGAT*CCGCCACC*ATGAACTCCTTCTCCACAAGCGC CTTCGGTCCAGTTGCCTTCTCCCTGGGGCTGCTCCTGGTGTTGCCTGCTGCCTTCCC TGCCCCAGTTGTGAGAGAGCCAGGCTCTGTGATCACCC 3' (SEQ ID NO:31). The 3' primer, containing the underlined *Xba* I restriction site and 20 nucleotides complementary to the 3' coding sequence immediately before the stop codon as shown in Figure 1A (SEQ ID NO:1), has the following sequence: 5' GCTCTAGAGTCCACCCAGGACACCCAGC 3' (SEQ ID NO:32).

The amplified fragment is digested with the endonucleases *Bam* HI and *Xba* I and then purified again on a I % agarose gel. The isolated fragment and the dephosphorylated vector are then ligated with T4 DNA ligase. *E. coli* HB101 or XL-1 Blue cells are then transformed and bacteria are identified that contain the fragment inserted into plasmid pC4 using, for instance, restriction enzyme analysis.

Chinese hamster ovary cells lacking an active DHFR gene are used for transfection. Five µg of the expression plasmid pC4 is cotransfected with 0.5 µg of the plasmid pSVneo using lipofectin (Felgner et al., *supra*). The plasmid pSV2-neo contains a dominant selectable marker, the *neo* gene from Tn5 encoding an enzyme that confers resistance to a group of antibiotics including G418. The cells are seeded in alpha minus MEM supplemented with 1 mg/ml G418. After 2 days, the cells are trypsinized and seeded in hybridoma cloning plates (Greiner. Germany) in alpha minus MEM supplemented with 10, 25, or 50 ng/ml of metothrexate plus 1 mg/ml G418. After about 10-14 days single clones are trypsinized and then seeded in 6-well petri dishes or 10 ml flasks using different concentrations of methotrexate (50 nM, 100 nM, 200 nM, 400 nM, 800 nM). Clones growing at the highest concentrations of methotrexate are then transferred to new 6-well plates containing even higher concentrations of methotrexate (1 µM, 2 µM, 5 µM, 10 mM, 20 mM). The same procedure is repeated until clones are obtained which grow at a concentration of 100 - 200 µM, Expression of the desired gene product is analyzed, for instance, by SDS-PAGE and Western blot or by reversed phase HPLC analysis.

### Example 4: Tissue distribution of FcR-I, FcR-II, FcR-III, FcR-IV, and FcR-V mRNA expression

Northern blot analysis is carried out to examine FcR-I, FcR-II, FcR-III, FcR-IV, and FcR-V gene expression in human tissues, using methods described by, among others, Sambrook *et al.*, cited above. A cDNA probe containing the entire nucleotide sequence of the FcR-I, FcR-II, FcR-III, FcR-IV, or FcR-V protein (SEQ ID NO:1. SEQ ID NO:3, SEQ ID NO:5, SEQ ID NO:7, SEQ ID NO:9, respectively) is labeled with ³²P using the *redi*prime^{™} DNA labeling system (Amersham Life Science), according to manufacturer's instructions. After labeling, the probe is purified using a CHROMA SPIN-100^{™} column (Clontech Laboratories. Inc.), according to manufacturer's protocol number PT1200-1. The purified labeled probe is then used to examine various human tissues for FcR-I, FcR-II, FcR-III, FcR-IV. and FcR-V mRNA.

Multiple Tissue Northern (MTN) blots containing various human tissues (H) or human immune system tissues (IM) are obtained from Clontech and are examined with the labeled probe using ExpressHyb^{™} hybridization solution (Clontech) according to manufacturer's protocol number PT1190-1. Following hybridization and washing, the blots are mounted and exposed to film at -70° C overnight, and films developed according to standard procedures.

It will be clear that the invention may be practiced otherwise than as particularly described in the foregoing description and examples. Numerous modifications and variations of the present invention are possible in light of the above teachings and, therefore, are within the scope of the appended claims.

The entire disclosure of all publications (including patents patent applications, journal articles, laboratory manuals, books, or other documents) cited herein are hereby incorporated by reference.

## Claims

1. An isolated nucleic acid molecule comprising a polynucleotide having a nucleotide sequence at least 95% identical to a sequence selected from the group consisting of:
(a) a nucleotide sequence encoding the FcR-II polypeptide having the amino acid sequence at positions -18 to 245 of SEQ ID NO:4 or the complete amino acid sequence encoded by the FcR-II cDNA clone contained in ATCC Deposit No. 97891;
(b) a nucleotide sequence encoding the FcR-III polypeptide having the amino acid sequence at positions -16 to 607 of SEQ ID NO:6 or the complete amino acid sequence encoded by the FcR-III cDNA clone contained in ATCC Deposit No. 97891;
(c) a nucleotide sequence encoding the FcR-IV polypeptide having the amino acid sequence at positions -16 to 456 of SEQ ID NO:8 or the complete amino acid sequence encoded by the FcR-IV cDNA clone contained in ATCC Deposit No. 97891;
(d) a nudeotide sequence encoding the FcR-V polypeptide having the amino acid sequence at positions -16 to 498 of SEQ ID NO:10 or the complete amino acid sequence encoded by the FcR-V cDNA clone contained in ATCC Deposit No. 209100;
(e) a nucleotide sequence encoding the FcR-II polypeptide having the amino acid sequence at positions -17 to 245 of SEQ ID NO:4 or the complete amino acid sequence excepting the N-terminal methionine encoded by the FcR-II cDNA clone contained in ATCC Deposit No. 97891;
(f) a nucleotide sequence encoding the FcR-III polypeptide having the amino acid sequence at positions -15 to 607 of SEQ ID NO:6 or the complete amino acid sequence excepting the N-terminal methionine encoded by the FcR-III cDNA clone contained in ATCC Deposit No. 97891;
(g) a nucleotide sequence encoding the FcR-IV polypeptide having the amino acid sequence at positions -15 to 456 of SEQ ID NO:8 or the complete amino acid sequence excepting the N-terminal methionine encoded by the FcR-IV cDNA clone contained in ATCC Deposit No. 97891;
(h) a nucleotide sequence encoding the FcR-V polypeptide having the amino acid sequence at positions -15 to 498 of SEQ ID NO:10 or the complete amino acid sequence excepting the N-terminal methionine encoded by the FcR-V cDNA clone contained in ATCC Deposit No. 209100;
(l) a nucleotide sequence encoding the mature form of the FcR-II polypeptide having the amino acid sequence at positions 1 to 245 in SEQ ID NO:4 or as encoded by the FcR-II cDNA clone contained in ATCC Deposit No. 97891;
(j) a nucleotide sequence encoding the mature form of the FcR-III polypeptide having the amino acid sequence at positions 1 to 607 in SEQ ID NO:6 or as encoded by the FcR-III cDNA clone contained in ATCC Deposit No. 97891;
(k) a nucleotide sequence encoding the mature form of the FcR-IV polypeptide having the amino acid sequence at positions 1 to 456 in SEQ ID NO:8 or as encoded by the FcR-IV cDNA clone contained in ATCC Deposit No. 97891;
(l) a nucleotide sequence encoding the mature form of the FcR-V polypeptide having the amino acid sequence at positions 1 to 498 in SEQ ID NO:10 or as encoded by the FcR-V cDNA clone contained in ATCC Deposit No. 209100;
(m) a nucleotide sequence encoding a polypeptide comprising the extracellular domain of the FcR-II polypeptide having the amino acid sequence at positions 1 to 211 in SEQ ID NO:4 or as encoded by the FcR-II cDNA clone contained in ATCC Deposit No. 97891;
(n) a nucleotide sequence encoding a polypeptide comprising the extracellular domain of the FcR-III polypeptide having the amino acid sequence at positions 1 to 421 in SEQ ID NO:6 or as encoded by the FcR-III cDNA clone contained in ATCC Deposit No. 97891;
(o) a nucleotide sequence encoding a polypeptide comprising the extracellular domain of the FcR-IV polypeptide having the amino acid sequence at positions 1 to 243 in SEQ ID NO:8 or as encoded by the FcR-IV cDNA clone contained in ATCC Deposit No. 97891;
(p) a nucleotide sequence encoding a polypeptide comprising the extracellular domain of the FcR-V polypeptide having the amino acid sequence at positions 1 to 343 in SEQ ID NO:10 or as encoded by the FcR-V cDNA clone contained in ATCC Deposit No. 209100;
(q) a nucleotide sequence encoding a polypeptide comprising the transmembrane domain of the FcR-II polypeptide having the amino acid sequence at positions 212 to 229 in SEQ ID NO:4 or as encoded by the FcR-II cDNA clone contained in ATCC Deposit No. 97891;
(r) a nucleotide sequence encoding a polypeptide comprising the transmembrane domain of the FcR-III polypeptide having the amino acid sequence at positions 422 to 448 in SEQ ID NO:6 or as encoded by the FcR-III cDNA clone contained in ATCC Deposit No. 97891;
(s) a nucleotide sequence encoding a polypeptide comprising the transmembrane domain of the FcR-IV polypeptide having the amino acid sequence at positions 244 to 264 in SEQ ID NO:8 or as encoded by the FcR-IV cDNA clone contained in ATCC Deposit No. 97891;
(t) a nucleotide sequence encoding a polypeptide comprising the transmembrane domain of the FcR-V polypeptide having the amino acid sequence at positions 344 to 364 in SEQ ID NO:10 or as encoded by the FcR-V cDNA clone contained in ATCC Deposit No. 209100;
(u) a nucleotide sequence encoding a polypeptide comprising the intracellular domain of the FcR-II polypeptide having the amino acid sequence at positions 230 to 245 in SEQ ID NO:4 or as encoded by the FcR-II cDNA clone contained in ATCC Deposit No. 97891;
(v) a nucleotide sequence encoding a polypeptide comprising the intracellular domain of the FcR-III polypeptide having the amino acid sequence at positions 449 to 607 in SEQ ID NO:6 or as encoded by the FcR-III cDNA clone contained in ATCC Deposit No. 97891;
(w) a nucleotide sequence encoding a polypeptide comprising the intracellular domain of the FcR-IV polypeptide having the amino acid sequence at positions 265 to 456 in SEQ ID NO:8 or as encoded by the FcR-IV cDNA clone contained in ATCC Deposit No. 97891;
(x) a nucleotide sequence encoding a polypeptide comprising the intracellular domain of the FcR-V polypeptide having the amino acid sequence at positions 365 to 498 in SEQ ID NO:10 or as encoded by the FcR-V cDNA clone contained in ATCC Deposit No. 97891;
(y) a nucleotide sequence encoding a soluble FcR-II polypeptide having the extracellular and intracellular domains but lacking the transmembrane domain;
(z) a nucleotide sequence encoding a soluble FcR-III polypeptide having the extracellular and intracellular domains but lacking the transmembrane domain;
(aa) a nucleotide sequence encoding a soluble FcR-IV polypeptide having the extracellular and intracellular domains but lacking the transmembrane domain; and
(ab) a nucleotide sequence encoding a soluble FcR-V polypeptide having the extracellular and intracellular domains but lacking the transmembrane domain or the complementary strand of such a polynucleotide;
or the complementary strand of such a polynucleotide.

2. The nucleic acid molecule of claim 1 wherein said polynucleotide has a nucleotide sequence selected from the group consisting of:
(a) the complete nucleotide sequence of SEQ ID NO:3;
(b) the complete nucleotide sequence of SEQ ID NO:5;
(c) the complete nucleotide sequence of SEQ ID NO:7 and
(d) the complete nucleotide sequence of SEQ ID NO:9.

3. The nucleic acid molecule of claim 1 wherein said polynucleotide has a nucleotide sequence selected from the group consisting of:
(a) the complete nucleotide sequence of SEQ ID NO:3 encoding the FcR-II polypeptide having the amino acid sequence in positions -18 to 245 of SEQ ID NO:4;
(b) the complete nucleotide sequence of SEQ ID NO:5 encoding the FcR-III polypeptide having the amino acid sequence in positions -16 to 607 of SEQ ID NO:6;
(c) the complete nucleotide sequence of SEQ ID NO:7 encoding the FcR-IV polypeptide having the amino acid sequence in positions -16 to 456 of SEQ ID NO:8; and
(d) the complete nucleotide sequence of SEQ ID NO:9 encoding the FcR-V polypeptide having the amino acid sequence in positions -16 to 498 of SEQ ID NO:10.

4. The nucleic acid molecule of claim 1 wherein said polynucleotide has a nucleotide sequence selected from the group consisting of:
(a) the complete nucleotide sequence of SEQ ID NO:3 encoding the FcR-II polypeptide having the amino acid sequence in positions -17 to 245 of SEQ ID NO:4;
(b) the complete nucleotide sequence of SEQ ID NO:5 encoding the FcR-III polypeptide having the amino acid sequence in positions -15 to 607 of SEQ ID NO:6;
(c) the complete nucleotide sequence of SEQ ID NO:7 encoding the FcR-IV polypeptide having the amino acid sequence in positions -15 to 456 of SEQ ID NO:8; and
(d) the complete nucleotide sequence of SEQ ID NO:9 encoding the FcR-V polypeptide having the amino acid sequence in positions -15 to 498 of SEQ ID NO:10.

5. The nucleic acid molecule of claim 1 wherein said polynucleotide has a nucleotide sequence selected from the group consisting of:
(a) the complete nucleotide sequence of SEQ ID NO:3 encoding the mature FcR-II polypeptide having the amino acid sequence from about 1 to about 245 in SEQ ID NO:4;
(b) the complete nucleotide sequence of SEQ ID NO:5 encoding the mature FcR-III polypeptide having the amino acid sequence from about 1 to about 607 in SEQ ID NO:4;
(c) the complete nucleotide sequence of SEQ ID NO:7 encoding the mature FcR-IV polypeptide having the amino acid sequence from about 1 to about 456 in SEQ ID NO:8; and
(d) the complete nucleotide sequence of SEQ ID NO:9 encoding the mature FcR-V polypeptide having the amino acid sequence from about 1 to about 498 in SEQ ID NO: 10.

6. An isolated nucleic acid molecule comprising a polynucleotide having a nucleotide sequence at least 95% identical to a sequence selected from the group consisting of:
(a) a nucleotide sequence encoding a polypeptide comprising the amino acid sequence of residues n to 245 of SEQ ID NO:4, where n is an integer in the range of -18 to 28;
(b) a nucleotide sequence encoding a polypeptide comprising the amino acid sequence of residues -18 to m of SEQ ID NO:4, where m is an integer in the range of 236 to 244;
(c) a nucleotide sequence encoding a polypeptide having the amino acid sequence consisting of residues n to m of SEQ ID NO:4, where n and m are integers as defined respectively in (a) and (b), above;
(d) a nucleotide sequence encoding a polypeptide consisting of a portion of the complete FcR-II amino acid sequence encoded by the FcR-II cDNA clone contained in ATCC Deposit No. 97891 wherein said portion excludes from 1 to about 27 amino acids from the amino terminus of said complete amino acid sequence encoded by the FcR-II cDNA clone contained in ATCC Deposit No. 97891;
(e) a nucleotide sequence encoding a polypeptide consisting of a portion of the complete FcR-II amino acid sequence encoded by the FcR-II cDNA clone contained in ATCC Deposit No. 97891 wherein said portion excludes from 1 to about 10 amino acids from the carboxy terminus of said complete amino acid sequence encoded by the FcR-II cDNA clone contained in ATCC Deposit No. 97891;
(f) a nucleotide sequence encoding a polypeptide consisting of a portion of the complete FcR-II amino acid sequence encoded by the FcR-II cDNA clone contained in ATCC Deposit No. 97891 wherein said portion includes a combination of any of the amino terminal and carboxy terminal deletions in (d) and (e), above;
(g) a nucleotide sequence encoding a polypeptide comprising the amino acid sequence of residues n to 607 of SEQ ID NO:6, where n is an integer in the range of -16 to 26;
(h) a nucleotide sequence encoding a polypeptide comprising the amino acid sequence of residues -16 to m of SEQ ID NO:6, where m is an integer in the range of 596 to 607;
(i) a nucleotide sequence encoding a polypeptide having the amino acid sequence consisting of residues n to m of SEQ ID NO:6, where n and m are integers as defined respectively in (g) and (h), above;
(j) a nucleotide sequence encoding a polypeptide consisting of a portion of the complete FcR-III amino acid sequence encoded by the FcR-III cDNA clone contained in ATCC Deposit No. 97891 wherein said portion excludes from 1 to about 25 amino acids from the amino terminus of said complete amino acid sequence encoded by the FcR-III cDNA clone contained in ATCC Deposit No. 97891;
(k) a nucleotide sequence encoding a polypeptide consisting of a portion of the complete FcR-III amino acid sequence encoded by the FcR-III cDNA clone contained in ATCC Deposit No. 97891 wherein said portion excludes from 1 to about 10 amino acids from the carboxy terminus of said complete amino acid sequence encoded by the FcR-III cDNA clone contained in ATCC Deposit No. 97891;
(l) a nucleotide sequence encoding a polypeptide consisting of a portion of the complete FcR-III amino acid sequence encoded by the FcR-III cDNA clone contained in ATCC Deposit No. 97891 wherein said portion include a combination of any of the amino terminal and carboxy terminal deletions in (j) and (k), above;
(m) a nucleotide sequence encoding a polypeptide comprising the amino acid sequence of residues n to 456 of SEQ ID NO:8, where n is an integer in the range of -16 to 26;
(n) a nucleotide sequence encoding a polypeptide comprising the amino acid sequence of residues -16 to m of SEQ ID NO:8, where m is an integer in the range of 446 to 456;
(o) a nucleotide sequence encoding a polypeptide having the amino acid sequence consisting of residues n to m of SEQ ID NO:8, where n and m are integers as defined respectively in (m) and (n), above;
(p) a nucleotide sequence encoding a polypeptide consisting of a portion of the complete FcR-IV amino acid sequence encoded by the FcR-IV cDNA clone contained in ATCC Deposit No. 97891 wherein said portion excludes from 1 to about 25 amino acids from the amino terminus of said complete amino acid sequence encoded by the FcR-IV cDNA clone contained in ATCC Deposit No. 97891;
(q) a nucleotide sequence encoding a polypeptide consisting of a portion of the complete FcR-IV amino acid sequence encoded by the FcR-IV cDNA clone contained in ATCC Deposit No. 97891 wherein said portion excludes from 1 to about 10 amino acids from the carboxy terminus of said complete amino acid sequence encoded by the FcR-IV cDNA clone contained in ATCC Deposit No. 97891;
(r) a nucleotide sequence encoding a polypeptide consisting of a portion of the complete FcR-IV amino acid sequence encoded by the FcR-IV cDNA clone contained in ATCC Deposit No. 97891 wherein said portion includes a combination of any of the amino terminal and carboxy terminal deletions in (p) and (q), above;
(s) a nucleotide sequence encoding a polypeptide comprising the amino acid sequence of residues n to 498 of SEQ ID NO:10, where n is an integer in the range of -16 to 26;
(t) a nucleotide sequence encoding a polypeptide comprising the amino acid sequence of residues -16 to m of SEQ ID NO:10, where m is an integer in the range of 488 to 498;
(u) a nucleotide sequence encoding a polypeptide having the amino acid sequence consisting of residues n to m of SEQ ID NO:10, where n and m are integers as defined respectively in (s) and (t), above;
(v) a nucleotide sequence encoding a polypeptide consisting of a portion of the complete FcR-V amino acid sequence encoded by the FcR-V cDNA clone contained in ATCC Deposit No. 209100 wherein said portion excludes from 1 to about 25 amino acids from the amino terminus of said complete amino acid sequence encoded by the FcR-V cDNA clone contained in ATCC Deposit No. 209100;
(w) a nucleotide sequence encoding a polypeptide consisting of a portion of the complete FcR-V amino acid sequence encoded by the FcR-V cDNA clone contained in ATCC Deposit No. 209100 wherein said portion excludes from 1 to about 10 amino acids from the carboxy terminus of said complete amino acid sequence encoded by the FcR-V cDNA clone contained in ATCC Deposit No. 209100; and
(x) a nucleotide sequence encoding a polypeptide consisting of a portion of the complete FcR-V amino acid sequence encoded by the FcR-V cDNA clone contained in ATCC Deposit No. 209100 wherein said portion includes a combination of any of the amino terminal and carboxy terminal deletions in (v) and (w), above.

7. The nucleic acid molecule of claim 1 wherein said polynucleotide has the complete nucleotide sequence of an FcR-II, FcR-III, or FcR-IV cDNA clone contained in ATCC Deposit No. 97891 or the complete nucleotide sequence of an FcR-V cDNA clone contained in ATCC Deposit No. 209100.

8. An isolated nucleic acid moiecuie comprising a polynucleotide which hybridizes under stringent hybridization conditions to a nucleotide sequence as defined in any one of (a) to (ab) of claim 1 wherein said polynucleotide which hybridizes does not hybridize under stringent hybridization conditions to a polynucleotide having a nucleotide sequence consisting of only A residues or of only T residues.

9. An isolated nucleic acid molecule comprising a polynucleotide which encodes the amino acid sequence of an epitope-bearing portion of an FcR-II, FcR-111, FcR-IV, or FcR-V polypeptide having an amino acid sequence in (a) through (x) of claim 1.

10. The nucleic acid molecule of claim 9, which encodes an epitope-bearing portion of an FcR-II polypeptide wherein the amino acid sequence of said portion is selected from the group of sequences in SEQ ID NO:4 consisting of:
about Leu-51 to about Trp-60, about Val-90 to about Arg-99, about Cys-101 to about Trp-112, about Ser-216 to about Val-231, and about Trp-251 to about Ile-261.

11. The nucleic acid molecule of claim 9, which encodes an epitope-bearing portion of an FcR-III polypeptide wherein the amino acid sequence of said portion is selected from the group of sequences in SEQ ID NO:6 consisting of:
about Leu-37 to about Ile-69, from about His-76 to about Leu-110, from about Leu-126 to about His-135, from about Glu-142 to about Gln-155, from about Asn-162 to about Phe-178, from about Ser-192 to about Leu-212, from about Lys-242 to about Leu-260, from about Ser-271 to about Leu-297, from about Tyr-381 to about Glu-427, and from about Arg-450 to about Ala-606.

12. The nucleic acid molecule of claim 9, which encodes an epitope-bearing portion of an FcR-IV polypeptide wherein the amino acid sequence of said portion is selected from the group of sequences in SEQ ID NO:8 consisting of:
about Thr-36 to about Ile-69, from about Ser-71 to about Leu-99, from about Ala-104 to about Ala-112, from about Thr-119 to about Phe-137, from about Ser-191 to about Ile-200, from about Ser-204 to about Met-278, from about His-235 to about Val-244, and from about Arg-268 to about Gln-456.

13. The nucleic acid molecule of claim 9, which encodes an epitope-bearing portion of an FcR-V polypeptide wherein the amino acid sequence of said portion is selected from the group of sequences in SEQ ID NO:10 consisting of: about Cys-140 to about Ser-160, from about Val-169 to about Val-189, from about Val-204 to about Pro-216, from about Val-238 to about Gln-258, from about Ser-270 to about Asp-297, from about Phe-304 to about Val-312, from about Pro-320 to about Val-369, from about Gly-404 to about Asn-416, and from about Gln-439 to about Ile-483.

14. A nucleic acid molecule comprising a polynucleotide having a sequence at least 95% identical to a sequence selected from the group consisting of:
(a) the nucleotide sequence of any one of SEQ ID NOS: 16 to 24;
(b) the nucleotide sequence of a portion of SEQ ID NO:3 wherein said portion comprises at least 50 contiguous nucleotides from nucleotide 1 to 1070;
(c) the nucleotide sequence of a portion of SEQ ID NO:3 wherein said portion consists of nucleotides 100-1070, 250-1070, 500-1070, 750-1070, 100-750, 100-500, 100-250, 250-750, 250-500, 500-750, 1-1100, 500-1100, 1000-1100, 1-1200, 500-1200, 1000-1200, 1-1300, 500-1300, 1000-1300, 1-1400, 500-1400, or 1000-1400;
(d) the nucleotide sequence of a portion of SEQ ID NO:5 wherein said portion comprises at least 50 contiguous nucleotides from nucleotide 1-650 and from 1350-1650;
(e) the nucleotide sequence of a portion of SEQ ID NO:5 wherein said portion consists of nucleotides 100-1900, 250-1900, 500-1900, 750-1900, 1000-1900, 1250-1900, 1500-1900, 100-1600, 250-1600, 500-1600, 750-1600, 1000-1600, 1250-1600, 100-1250, 250-1250, 500-1250, 750-1250, 1000-1250, 100-1000, 250-1000, 500-1000, 750-1000, 100-750, 250-750, 500-750, 100-500, or 250-500;
(f) the nucleotide sequence of a portion of SEQ ID NO:7 wherein said portion comprises at least 50 contiguous nucleotides from nucleotide 1 to residue 1000;
(g) the nucleotide sequence of a portion of SEQ ID NO:7 wherein said portion consists of nucleotides 100-1400, 250-1400, 500-1400, 750-1400, 1000-1400, 100-1000, 250-1000, 500-1000, 750-1000, 100-750, 250-750, 500-750, 100-500, 250-500, or 100-250;
(h) the nucleotide sequence of a portion of SEQ ID NO:9 wherein said portion comprises at least 50 contiguous nucleotides from nucleotide 1 to residue 650;
(i) the nucleotide sequence of a portion of SEQ ID NO:9 wherein said portion consists of nucleotides 100-1650, 250-1650, 500-1650, 750-1650, 1000-1650, 1250-1650, 250-1000, 500-1000, 750-1000, 100-750, 250-750, 500-750, 100-500, 250-500, or 1-250; and
(j) a nucleotide sequence complementary to any of the nucleotide sequences in (a) through (i), above.

15. A method for making a recombinant vector comprising inserting a nucleic acid molecule of any one of claims 1 to 13 into a vector.

16. A recombinant vector produced by the method of claim 15.

17. A method of making a recombinant host cell comprising introducing the recombinant vector of claim 16 into a host cell.

18. A recombinant host cell produced by the method of claim 17.

19. A recombinant method for producing an FcR-II, FcR-III, FcR-IV, or FcR-V polypeptide, comprising culturing the recombinant host cell of claim 18 under conditions such that said polypeptide is expressed and recovering said polypeptide.

20. An isolated FcR-II, FcR-III, FcR-IV, or FcR-V polypeptide comprising an amino acid sequence of a polypeptide encoded by the nucleic acid molecule of claim 1 or obtainable by the recombinant method of claim 19.

21. An isolated polypeptide comprising an epitope-bearing portion of the FcR-II, FcR-III, FcR-IV, or FcR-V protein, wherein said portion is encoded by the nucleic acid molecule of any one of claims 9 to 13.

22. An isolated antibody that binds specifically to an FcR-II, FeR-III, FcR-IV, or FcR-V polypeptide of claim 20.


SEQUENCE LISTING

(1) GENERAL INFORMATION:

(i) APPLICANT: Human Genome Sciences, Inc. et al
OLSEN, HENRIK S.
RUBEN, STEVEN
NI, JIAN
MURPHY, MARIANNE
GENTZ, REINER

(ii) TITLE OF INVENTION: FC RECEPTORS AND POLYPEPTIDES

(iii) NUMBER OF SEQUENCES: 32

(iv) CORRESPONDENCE ADDRESS:
(A) ADDRESSEE: HUMAN GENOME SCIENCES, INC.
(B) STREET: 9410 KEY WEST AVENUE
(C) CITY: ROCKVILLE
(D) STATE: MD
(E) COUNTRY: US
(F) ZIP: 20850

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30

(vi) CURRENT APPLICATION DATA:
(A) APPLICATION NUMBER: PCT/US98/01184
(B) FILING DATE: Jan-20-98
(C) CLASSIFICATION:

(viii) ATTORNEY/AGENT INFORMATION:
(A) NAME: BROOKES, ALLAN A.
(B) REGISTRATION NUMBER: 36,373
(C) REFERENCE/DOCKET NUMBER: PF363PCT

(ix) TELECOMMUNICATION INFORMATION:
(A) TELEPHONE: (301) 309-8504
(B) TELEFAX: (3010 309-8512

(2) INFORMATION FOR SEQ ID NO:1:

(i) SEQUENCE CHARACTERISTICS:
(A) LENGTH: 1552 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: CDS
(B) LOCATION: 82..1362

(ix) FEATURE:
(A) NAME/KEY: sig_peptide
(B) LOCATION: 82..142

(ix) FEATURE:
(A) NAME/KEY: mat_peptide
(B) LOCATION: 145..1362

(xi) SEQUENCE DESCRIPTION: SEQ ID NO:1:

GCAGGAATTC GGCACGAGCC TCTGTCCTGC CAGCACCGAG GGCTCATCCA TCCACAGAGC 60

AGTGCAGTGG GAGGAGACGC C ATG ACC CCC ATC CTC ACG GTC CTG ATC TGT 111
Met Thr Pro Ile Leu Thr Val Leu Ile Cys
-21 -20 -15

CTC GGG CCC CTC CCC AAG CCC ACC CTC TGG GCT GAG CCA GGC TCT GTG 159
Leu Gly Pro Leu Pro Lys Pro Thr Leu Trp Ala Glu Pro Gly Ser Val
-10 -5 1 5

ATC ACC CAA GGG AGT CCT GTG ACC CTC AGG TGT CAG GGG AGC CTG GAG 207
Ile Thr Gln Gly Ser Pro Val Thr Leu Arg Cys Gln Gly Ser Leu Glu
10 15 20

ACG CAG GAG TAC CAT CTA TAT AGA GAA AAG AAA ACA GCA CTC TGG ATT 255
Thr Gln Glu Tyr His Leu Tyr Arg Glu Lys Lys Thr Ala Leu Trp Ile
25 30 35

ACA CGG ATC CCA CAG GAG CTT GTG AAG AAG GGC CAG TTC CCC ATC CTA 303
Thr Arg Ile Pro Gln Glu Leu Val Lys Lys Gly Gln Phe Pro Ile Leu
40 45 50

TCC ATC ACC TGG GAA CAT GCA GGG CGG TAT TGC TGT ATC TAT GGC AGC 351
Ser Ile Thr Trp Glu His Ala Gly Arg Tyr Cys Cys Ile Tyr Gly Ser
55 60 65

CAC ACT GCA GGC CTC TCA GAG AGC AGT GAC CCC CTG GAG CTG GTG GTG 399
His Thr Ala Gly Leu Ser Glu Ser Ser Asp Pro Leu Glu Leu Val Val
70 75 80 85

ACA GGA GCC TAC AGC AAA CCC ACC CTC TCA GCT CTG CCC AGC CCT GTG 447
Thr Gly Ala Tyr Ser Lys Pro Thr Leu Ser Ala Leu Pro Ser Pro Val
90 95 100

GTG ACC TCA GGA GGG AAT GTG ACC ATC CAG TGT GAC TCA CAG GTG GCA 495
Val Thr Ser Gly Gly Asn Val Thr Ile Gln Cys Asp Ser Gln Val Ala
105 110 115

TTT GAT GGC TTC ATT CTG TGT AAG GAA GGA GAA GAT GAA CAC CCA CAA 543
Phe Asp Gly Phe Ile Leu Cys Lys Glu Gly Glu Asp Glu His Pro Gln
120 125 130

TGC CTG AAC TCC CAT TCC CAT GCC CGT GGG TCA TCC CGG GCC ATC TTC 591
Cys Leu Asn Ser His Ser His Ala Arg Gly Ser Ser Arg Ala Ile Phe
135 140 145

TCC GTG GGC CCC GTG AGC CCA AGT CGC AGG TGG TCG TAC AGG TGC TAT 639
Ser Val Gly Pro Val Ser Pro Ser Arg Arg Trp Ser Tyr Arg Cys Tyr
150 155 160 165

GGT TAT GAC TCG CGC GCT CCC TAT GTG TGG TCT CTA CCC AGT GAT CTC 687
Gly Tyr Asp Ser Arg Ala Pro Tyr Val Trp Ser Leu Pro Ser Asp Leu
170 175 180

CTG GGG CTC CTG GTC CCA GGT GTT TCT AAG AAG CCA TCA CTC TCA GTG 735
Leu Gly Leu Leu Val Pro Gly Val Ser Lys Lys Pro Ser Leu Ser Val
185 190 195

CAG CCG GGT CCT GTC GTG GCC CCT GGG GAG AAG CTG ACC TTC CAG TGT 783
Gln Pro Gly Pro Val Val Ala Pro Gly Glu Lys Leu Thr Phe Gln Cys
200 205 210

GGC TCT GAT GCC GGC TAC GAC AGA TTT GTT CTG TAC AAG GAG TGG GGA 831
Gly Ser Asp Ala Gly Tyr Asp Arg Phe Val Leu Tyr Lys Glu Trp Gly
215 220 225

CGT GAC TTC CTC CAG CGC CCT GGC CGG CAG CCC CAG GCT GGG CTC TCC 879
Arg Asp Phe Leu Gln Arg Pro Gly Arg Gln Pro Gln Ala Gly Leu Ser
230 235 240 245

CAG GCC AAC TTC ACC CTG GGC CCT GTG AGC CGC TCC TAC GGG GGC CAG 927
Gln Ala Asn Phe Thr Leu Gly Pro Val Ser Arg Ser Tyr Gly Gly Gln
250 255 260

TAC ACA TGC TCC GGT GCA TAC AAC CTC TCC TCC GAG TGG TCG GCC CCC 975
Tyr Thr Cys Ser Gly Ala Tyr Asn Leu Ser Ser Glu Trp Ser Ala Pro
265 270 275

AGC GAC CCC CTG GAC ATC CTG ATC ACA GGA CAG ATC CGT GCC AGA CCC 1023
Ser Asp Pro Leu Asp Ile Leu Ile Thr Gly Gln Ile Arg Ala Arg Pro
280 285 290

TTC CTC TCC GTG CGG CCG GGC CCC ACA GTG GCC TCA GGA GAG AAC GTG 1071
Phe Leu Ser Val Arg Pro Gly Pro Thr Val Ala Ser Gly Glu Asn Val
295 300 305

ACC CTG CTG TGT CAG TCA CAG GGA GGG ATG CAC ACT TTC CTT TTG ACC 1119
Thr Leu Leu Cys Gln Ser Gln Gly Gly Met His Thr Phe Leu Leu Thr
310 315 320 325

AAG GAG GGG GCA GCT GAT TCC CCG CTG CGT CTA AAA TCA AAG CGC CAA 1167
Lys Glu Gly Ala Ala Asp Ser Pro Leu Arg Leu Lys Ser Lys Arg Gln
330 335 340

TCT CAT AAG TAC CAG GCT GAA TTC CCC ATG AGT CCT GTG ACC TCG GCC 1215
Ser His Lys Tyr Gln Ala Glu Phe Pro Met Ser Pro Val Thr Ser Ala
345 350 355

CAC GCG GGG ACC TAC AGG TGC TAC GGC TCA CTC AGC TCC AAC CCC TAC 1263
His Ala Gly Thr Tyr Arg Cys Tyr Gly Ser Leu Ser Ser Asn Pro Tyr
360 365 370

CTG CTG ACT CAC CCC AGT GAC CCC CTG GAG CTC GTG GTC TCA GGA GCA 1311
Leu Leu Thr His Pro Ser Asp Pro Leu Glu Leu Val Val Ser Gly Ala
375 380 385

GCT GAG ACC CTC AGC CCA CCA CAA AAC AAG TCC GAC TCC AAG GCT GGT 1359
Ala Glu Thr Leu Ser Pro Pro Gln Asn Lys Ser Asp Ser Lys Ala Gly
390 395 400 405

GAG TGAGGAGATG CTTGCCGTGA TGACGCTGGG CACAGAGGGT CAGGTCCTGT 1412
Glu

CAAGAGGAGC TGGGTGTCCT GGGTGGACAT TTGAAGAATT ATATTCATTC CAACTTGAAG 1472

AATTATTCAA CACCTTTAAC AATGTATATG TGAAGTACTT TATTCTTTCA TATTTTAAAA 1532

ATAAAAGATA ATTATCCATG 1552

(2) INFORMATION FOR SEQ ID NO:2:

(i) SEQUENCE CHARACTERISTICS:
(A) LENGTH: 427 amino acids
(B) TYPE: amino acid
(D) TOPOLOGY: linear

(ii) MOLECULE TYPE: protein

(xi) SEQUENCE DESCRIPTION: SEQ ID NO:2:

Met Thr Pro Ile Leu Thr Val Leu Ile Cys Leu Gly Pro Leu Pro Lys
-21 -20 -15 -10

Pro Thr Leu Trp Ala Glu Pro Gly Ser Val Ile Thr Gln Gly Ser Pro
-5 1 5 10

Val Thr Leu Arg Cys Gln Gly Ser Leu Glu Thr Gln Glu Tyr His Leu
15 20 25

Tyr Arg Glu Lys Lys Thr Ala Leu Trp Ile Thr Arg Ile Pro Gln Glu
30 35 40

Leu Val Lys Lys Gly Gln Phe Pro Ile Leu Ser Ile Thr Trp Glu His
45 50 55

Ala Gly Arg Tyr Cys Cys Ile Tyr Gly Ser His Thr Ala Gly Leu Ser
60 65 70 75

Glu Ser Ser Asp Pro Leu Glu Leu Val Val Thr Gly Ala Tyr Ser Lys
80 85 90

Pro Thr Leu Ser Ala Leu Pro Ser Pro Val Val Thr Ser Gly Gly Asn
95 100 105

Val Thr Ile Gln Cys Asp Ser Gln Val Ala Phe Asp Gly Phe Ile Leu
110 115 120

Cys Lys Glu Gly Glu Asp Glu His Pro Gln Cys Leu Asn Ser His Ser
125 130 135

His Ala Arg Gly Ser Ser Arg Ala Ile Phe Ser Val Gly Pro Val Ser
140 145 150 155

Pro Ser Arg Arg Trp Ser Tyr Arg Cys Tyr Gly Tyr Asp Ser Arg Ala
160 165 170

Pro Tyr Val Trp Ser Leu Pro Ser Asp Leu Leu Gly Leu Leu Val Pro
175 180 185

Gly Val Ser Lys Lys Pro Ser Leu Ser Val Gln Pro Gly Pro Val Val
190 195 200

Ala Pro Gly Glu Lys Leu Thr Phe Gln Cys Gly Ser Asp Ala Gly Tyr
205 210 215

Asp Arg Phe Val Leu Tyr Lys Glu Trp Gly Arg Asp Phe Leu Gln Arg
220 225 230 235

Pro Gly Arg Gln Pro Gln Ala Gly Leu Ser Gln Ala Asn Phe Thr Leu
240 245 250

Gly Pro Val Ser Arg Ser Tyr Gly Gly Gln Tyr Thr Cys Ser Gly Ala
255 260 265

Tyr Asn Leu Ser Ser Glu Trp Ser Ala Pro Ser Asp Pro Leu Asp Ile
270 275 280

Leu Ile Thr Gly Gln Ile Arg Ala Arg Pro Phe Leu Ser Val Arg Pro
285 290 295

Gly Pro Thr Val Ala Ser Gly Glu Asn Val Thr Leu Leu Cys Gln Ser
300 305 310 315

Gln Gly Gly Met His Thr Phe Leu Leu Thr Lys Glu Gly Ala Ala Asp
320 325 330

Ser Pro Leu Arg Leu Lys Ser Lys Arg Gln Ser His Lys Tyr Gln Ala
335 340 345

Glu Phe Pro Met Ser Pro Val Thr Ser Ala His Ala Gly Thr Tyr Arg
350 355 360

Cys Tyr Gly Ser Leu Ser Ser Asn Pro Tyr Leu Leu Thr His Pro Ser
365 370 375

Asp Pro Leu Glu Leu Val Val Ser Gly Ala Ala Glu Thr Leu Ser Pro
380 385 390 395

Pro Gln Asn Lys Ser Asp Ser Lys Ala Gly Glu
400 405

(2) INFORMATION FOR SEQ ID NO:3:

(i) SEQUENCE CHARACTERISTICS:
(A) LENGTH: 1410 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: CDS
(B) LOCATION: 37..826

(ix) FEATURE:
(A) NAME/KEY: sig_peptide
(B) LOCATION: 37..88

(ix) FEATURE:
(A) NAME/KEY: mat_peptide
(B) LOCATION: 91..826

(xi) SEQUENCE DESCRIPTION: SEQ ID NO:3:

ACCCACGCGT CCGCACTCTA GCGGTATCTG CCCACC ATG GCC CTG GTG CTG ATC 54
Met Ala Leu Val Leu Ile
-18 -15

CTC CAG CTG CTG ACC CTC TGG CCT CTG TGT CAC ACA GAC ATC ACT CCG 102
Leu Gln Leu Leu Thr Leu Trp Pro Leu Cys His Thr Asp Ile Thr Pro
-10 -5 1

TCT GTC CCC CCA GCT TCA TAC CAC CCT AAG CCA TGG CTG GGA GCT CAG 150
Ser Val Pro Pro Ala Ser Tyr His Pro Lys Pro Trp Leu Gly Ala Gln
5 10 15 20

CCG GCT ACA GTT GTG ACC CCT GGG GTC AAC GTG ACC TTG AGA TGC CGG 198
Pro Ala Thr Val Val Thr Pro Gly Val Asn Val Thr Leu Arg Cys Arg
25 30 35

GCA CCC CAA CCC GCT TGG AGA TTT GGA CTT TTC AAG CCT GGA GAG ATC 246
Ala Pro Gln Pro Ala Trp Arg Phe Gly Leu Phe Lys Pro Gly Glu Ile
40 45 50

GCT CCC CTT CTC TTC CGG GAT GTG TCC TCC GAG CTG GCA GAA TTC TTT 294
Ala Pro Leu Leu Phe Arg Asp Val Ser Ser Glu Leu Ala Glu Phe Phe
55 60 65

CTG GAG GAG GTG ACT CCA GCC CAA GGG GGA AGT TAC CGC TGC TGC TAC 342
Leu Glu Glu Val Thr Pro Ala Gln Gly Gly Ser Tyr Arg Cys Cys Tyr
70 75 80

CGA AGG CCA GAC TGG GGG CCG GGT GTC TGG TCC CAG CCC AGC GAT GTC 390
Arg Arg Pro Asp Trp Gly Pro Gly Val Trp Ser Gln Pro Ser Asp Val
85 90 95 100

CTG GAG CTG CTG GTG ACA GAG GAG CTG CCG CGG CCG TCG CTG GTG GCG 438
Leu Glu Leu Leu Val Thr Glu Glu Leu Pro Arg Pro Ser Leu Val Ala
105 110 115

CTG CCC GGG CCG GTG GTG GGT CCT GGC GCC AAC GTG AGC CTG CGC TGC 486
Leu Pro Gly Pro Val Val Gly Pro Gly Ala Asn Val Ser Leu Arg Cys
120 125 130

GCG GGC CGC CTG CGG AAC ATG AGC TTC GTG CTG TAC CGC GAG GGC GTG 534
Ala Gly Arg Leu Arg Asn Met Ser Phe Val Leu Tyr Arg Glu Gly Val
135 140 145

GCG GCC CCG CTG CAG TAC CGC CAC TCC GCG CAG CCC TGG GCC GAC TTC 582
Ala Ala Pro Leu Gln Tyr Arg His Ser Ala Gln Pro Trp Ala Asp Phe
150 155 160

ACG CTG CTG GGC GCC CGC GCC CCC GGC ACC TAC AGC TGC TAC TAT CAC 630
Thr Leu Leu Gly Ala Arg Ala Pro Gly Thr Tyr Ser Cys Tyr Tyr His
165 170 175 180

ACG CCC TCC GCG CCC TAC GTG CTG TCG CAG CGC AGC GAG GTG CTG GTC 678
Thr Pro Ser Ala Pro Tyr Val Leu Ser Gln Arg Ser Glu Val Leu Val

185 190 195

ATC AGC TGG GAA GAC TCT GGC TCC TCC GAC TAC ACC CGG GGG AAC CTA 726
Ile Ser Trp Glu Asp Ser Gly Ser Ser Asp Tyr Thr Arg Gly Asn Leu
200 205 210

GTC CGC CTG GGG CTG GCC GGG CTG GTC CTC ATC TCC CTG GGC GCG CTG 774
Val Arg Leu Gly Leu Ala Gly Leu Val Leu Ile Ser Leu Gly Ala Leu
215 220 225

GTC ACT TTT GAC TGG CGC AGT CAG AAC CGC GCT CCT GCT GGT ATC CGC 822
Val Thr Phe Asp Trp Arg Ser Gln Asn Arg Ala Pro Ala Gly Ile Arg
230 235 240

CCC T GAGCCCCAGG AGCACTGCAG CCCGAGACTT CCAACCTGAG TGGCGGAGAA 876
Pro
245

GCTGGGACCC TGGGCTGGAC TGTCCTTTCC TGCAGCCCCA CAGTCCTGCT GGCTGAGCTC 936

CGCGGAACGG TCCTTAGACC CCGCTGTGCC CTGTGCTGTA GCTTCTTTCC AGGCCTTTCC 996

CAAGGAGTAG CTGAAAGGAA GACGCGATTA GTGGTTAAGA CTTCCAAGCC AGAAGACAGA 1056

GGGTTCGAAT CCCAGCACTG CCGTCTACTC ACTGTAGTAG TAGCAGCTAC AGAAAGGTAG 1116

TAGTGAGACG TGAAGCCAGC TGGACTTCCT GGGTTGAATG GGGACCTGGA GAACTTTTCT 1176

GTCTTACAAG AGGATTGTAA AATGGACCAA TCAGCACTCT GTAAGATGGA CCAATCAGCG 1236

CTCTGTAAAA TGGACCAATC AGCAGGACAT GGGCGGGGAC AATAAGGGAA TAAAAGCTGG 1296

CGAGCGCGGC ACCCCACCAG AGTCTGCTTC CACGCTGTGG GAGCTTTGTT CTCTTGCTCT 1356

ACACAATAAA TCTTGCTGCT GCTAAAAAAA AAAAAAAAAA AAAAAAAAAA AAAA 1410

(2) INFORMATION FOR SEQ ID NO:4:

(i) SEQUENCE CHARACTERISTICS:
(A) LENGTH: 263 amino acids
(B) TYPE: amino acid
(D) TOPOLOGY: linear

(ii) MOLECULE TYPE: protein

(xi) SEQUENCE DESCRIPTION: SEQ ID NO:4:

Met Ala Leu Val Leu Ile Leu Gln Leu Leu Thr Leu Trp Pro Leu Cys
-18 -15 -10 -5

His Thr Asp Ile Thr Pro Ser Val Pro Pro Ala Ser Tyr His Pro Lys
1 5 10

Pro Trp Leu Gly Ala Gln Pro Ala Thr Val Val Thr Pro Gly Val Asn
15 20 25 30

Val Thr Leu Arg Cys Arg Ala Pro Gln Pro Ala Trp Arg Phe Gly Leu
        35                  40                  45

Phe Lys Pro Gly Glu Ile Ala Pro Leu Leu Phe Arg Asp Val Ser Ser

    50                  55                  60

Glu Leu Ala Glu Phe Phe Leu Glu Glu Val Thr Pro Ala Gln Gly Gly
65 70 75

Ser Tyr Arg Cys Cys Tyr Arg Arg Pro Asp Trp Gly Pro Gly Val Trp
80 85 90

Ser Gln Pro Ser Asp Val Leu Glu Leu Leu Val Thr Glu Glu Leu Pro
95 100 105 110

Arg Pro Ser Leu Val Ala Leu Pro Gly Pro Val Val Gly Pro Gly Ala
        115                 120                 125

Asn Val Ser Leu Arg Cys Ala Gly Arg Leu Arg Asn Met Ser Phe Val
    130                 135                 140

Leu Tyr Arg Glu Gly Val Ala Ala Pro Leu Gln Tyr Arg His Ser Ala
145 150 155

Gln Pro Trp Ala Asp Phe Thr Leu Leu Gly Ala Arg Ala Pro Gly Thr
160 165 170

Tyr Ser Cys Tyr Tyr His Thr Pro Ser Ala Pro Tyr Val Leu Ser Gln
175 180 185 190

Arg Ser Glu Val Leu Val Ile Ser Trp Glu Asp Ser Gly Ser Ser Asp
        195                 200                 205

Tyr Thr Arg Gly Asn Leu Val Arg Leu Gly Leu Ala Gly Leu Val Leu
    210                 215                 220

Ile Ser Leu Gly Ala Leu Val Thr Phe Asp Trp Arg Ser Gln Asn Arg
225 230 235

Ala Pro Ala Gly Ile Arg Pro
240 245

(2) INFORMATION FOR SEQ ID NO:5:

(i) SEQUENCE CHARACTERISTICS:
(A) LENGTH: 1991 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: CDS
(B) LOCATION: 73..1942

(ix) FEATURE:
(A) NAME/KEY: sig_peptide
(B) LOCATION: 73..118

(ix) FEATURE:
(A) NAME/KEY: mat_peptide
(B) LOCATION: 121..1942

(xi) SEQUENCE DESCRIPTION: SEQ ID NO:5:

GCAGGAATTC GGCACGAGCA GCACTGAGGG CTCATCCCTC TGCAGAGCGC GGGGTCACCG 60

GAAGGAGACG CC ATG ACG CCC GCC CTC ACA GCC CTG CTC TGC CTT GGG 108
Met Thr Pro Ala Leu Thr Ala Leu Leu Cys Leu Gly
-16 -15 -10 -5

CTG AGT CTG GGC CCC AGG ACC CGC GTG CAG GCA GGG CCC TTC CCC AAA 156
Leu Ser Leu Gly Pro Arg Thr Arg Val Gln Ala Gly Pro Phe Pro Lys
1 5 10

CCC ACC CTC TGG GCT GAG CCA GGC TCT GTG ATC AGC TGG GGG AGC CCC 204
Pro Thr Leu Trp Ala Glu Pro Gly Ser Val Ile Ser Trp Gly Ser Pro
15 20 25

GTG ACC ATC TGG TGT CAG GGG AGC CTG GAG GCC CAG GAG TAC CAA CTG 252
Val Thr Ile Trp Cys Gln Gly Ser Leu Glu Ala Gln Glu Tyr Gln Leu
30 35 40

GAT AAA GAG GGA AGC CCA GAG CCC TTG GAC AGA AAT AAC CCA CTG GAA 300
Asp Lys Glu Gly Ser Pro Glu Pro Leu Asp Arg Asn Asn Pro Leu Glu
45 50 55 60

CCC AAG AAC AAG GCC AGA TTC TCC ATC CCA TCC ATG ACA CAG CAC CAT 348
Pro Lys Asn Lys Ala Arg Phe Ser Ile Pro Ser Met Thr Gln His His
65 70 75

GCA GGG AGA TAC CGC TGC CAC TAT TAC AGC TCT GCA GGC TGG TCA GAG 396
Ala Gly Arg Tyr Arg Cys His Tyr Tyr Ser Ser Ala Gly Trp Ser Glu
80 85 90

CCC AGC GAC CCC CTG GAG CTG GTG ATG ACA GGA GCC TAT AGC AAA CCC 444
Pro Ser Asp Pro Leu Glu Leu Val Met Thr Gly Ala Tyr Ser Lys Pro
95 100 105

ACC CTC TCA GCC CTG CCC AGC CCT GTG GTG GCC TCA GGG GGG AAT ATG 492
Thr Leu Ser Ala Leu Pro Ser Pro Val Val Ala Ser Gly Gly Asn Met
110 115 120

ACC CTC CGA TGT GGC TCA CAG AAG AGA TAT CAC CAT TTT GTT CTG ATG 540
Thr Leu Arg Cys Gly Ser Gln Lys Arg Tyr His His Phe Val Leu Met
125 130 135 140

AAG GAA GGA GAA CAC CAG CTC CCC CGG ACC CTG GAC TCA CAG CAG CTC 588
Lys Glu Gly Glu His Gln Leu Pro Arg Thr Leu Asp Ser Gln Gln Leu
145 150 155

CAC AGT GGG GGG TTC CAG GCC CTG TTC CCT GTG GGC CCC GTG AAC CCC 636
His Ser Gly Gly Phe Gln Ala Leu Phe Pro Val Gly Pro Val Asn Pro
160 165 170

AGC CAC AGG TGG AGG TTC ACA TGC TAT TAC TAT TAT ATG AAC ACC CCC 684
Ser His Arg Trp Arg Phe Thr Cys Tyr Tyr Tyr Tyr Met Asn Thr Pro
175 180 185

CGG GTG TGG TCC CAC CCC AGT GAC CCC CTG GAG ATT CTG CCC TCA GGC 732
Arg Val Trp Ser His Pro Ser Asp Pro Leu Glu Ile Leu Pro Ser Gly
190 195 200

GTG TCT AGG AAG CCC TCC CTC CTG ACC CTG CAG GGC CCT GTC CTG GCC 780
Val Ser Arg Lys Pro Ser Leu Leu Thr Leu Gln Gly Pro Val Leu Ala
205 210 215 220

CCT GGG CAG AGC CTG ACC CTC CAG TGT GGC TCT GAT GTC GGC TAC GAC 828
Pro Gly Gln Ser Leu Thr Leu Gln Cys Gly Ser Asp Val Gly Tyr Asp
225 230 235

AGA TTT GTT CTG TAT AAG GAG GGG GAA CGT GAC TTC CTC CAG CGC CCT 876
Arg Phe Val Leu Tyr Lys Glu Gly Glu Arg Asp Phe Leu Gln Arg Pro
240 245 250

GGC CAG CAG CCC CAG GCT GGG CTC TCC CAG GCC AAC TTC ACC CTG GGC 924
Gly Gln Gln Pro Gln Ala Gly Leu Ser Gln Ala Asn Phe Thr Leu Gly
255 260 265

CCT GTG AGC CCC TCC AAT GGG GGC CAG TAC AGG TGC TAC GGT GCA CAC 972
Pro Val Ser Pro Ser Asn Gly Gly Gln Tyr Arg Cys Tyr Gly Ala His
270 275 280

AAC CTC TCC TCC GAG TGG TCG GCC CCC AGC GAC CCC CTG AAC ATC CTG 1020
Asn Leu Ser Ser Glu Trp Ser Ala Pro Ser Asp Pro Leu Asn Ile Leu
285 290 295 300

ATG GCA GGA CAG ATC TAT GAC ACC GTC TCC CTG TCA GCA CAG CCG GGC 1068
Met Ala Gly Gln Ile Tyr Asp Thr Val Ser Leu Ser Ala Gln Pro Gly
305 310 315

CCC ACA GTG GCC TCA GGA GAG AAC GTG ACC CTG CTG TGT CAG TCA TGG 1116
Pro Thr Val Ala Ser Gly Glu Asn Val Thr Leu Leu Cys Gln Ser Trp
320 325 330

TGG CAG TTT GAC ACT TTC CTT CTG ACC AAA GAA GGG GCA GCC CAT CCC 1164
Trp Gln Phe Asp Thr Phe Leu Leu Thr Lys Glu Gly Ala Ala His Pro
335 340 345

CCA CTG CGT CTG AGA TCA ATG TAC GGA GCT CAT AAG TAC CAG GCT GAA 1212
Pro Leu Arg Leu Arg Ser Met Tyr Gly Ala His Lys Tyr Gln Ala Glu
350 355 360

TTC CCC ATG AGT CCT GTG ACC TCA GCC CAC GCG GGG ACC TAC AGG TGC 1260
Phe Pro Met Ser Pro Val Thr Ser Ala His Ala Gly Thr Tyr Arg Cys
365 370 375 380

TAC GGC TCA CGC AGC TCC AAC CCC TAC CTG CTG TCT CAC CCC AGT GAG 1308
Tyr Gly Ser Arg Ser Ser Asn Pro Tyr Leu Leu Ser His Pro Ser Glu
385 390 395

CCC CTG GAG CTC GTG GTC TCA GGA CAC TCT GGA GGC TCC AGC CTC CCA 1356
Pro Leu Glu Leu Val Val Ser Gly His Ser Gly Gly Ser Ser Leu Pro
400 405 410

CCC ACA GGG CCG CCC TCC ACA CCT GGT CTG GGA AGA TAC CTG GAG GTT 1404
Pro Thr Gly Pro Pro Ser Thr Pro Gly Leu Gly Arg Tyr Leu Glu Val
415 420 425

TTG ATT GGG GTC TCG GTG GCC TTC GTC CTG CTG CTC TTC CTC CTC CTC 1452
Leu Ile Gly Val Ser Val Ala Phe Val Leu Leu Leu Phe Leu Leu Leu
430 435 440

TTC CTC CTC CTC CGA CGT CAG CGT CAC AGC AAA CAC AGG ACA TCT GAC 1500

Phe Leu Leu Leu Arg Arg Gln Arg His Ser Lys His Arg Thr Ser Asp
445                 450                 455                 460

CAG AGA AAG ACT GAT TTC CAG CGT CCT GCA GGG GCT GCG GAG ACA GAG      1548
Gln Arg Lys Thr Asp Phe Gln Arg Pro Ala Gly Ala Ala Glu Thr Glu
                465                 470                 475

CCC AAG GAC AGG GGC CTG CTG AGG AGG TCC AGC CCA GCT GCT GAC GTC      1596
Pro Lys Asp Arg Gly Leu Leu Arg Arg Ser Ser Pro Ala Ala Asp Val
            480                 485                 490

CAG GAA GAA AAC CTC TAT GCT GCC GTG AAG GAC ACA CAG TCT GAG GAC      1644
Gln Glu Glu Asn Leu Tyr Ala Ala Val Lys Asp Thr Gln Ser Glu Asp
        495                 500                 505

GGG GTG GAG CTG GAC AGT CAG AGC CCA CAC GAT GAA GAC CCC CAC GCA      1692
Gly Val Glu Leu Asp Ser Gln Ser Pro His Asp Glu Asp Pro His Ala
    510                 515                 520

GTG ACG TAT GCC CCG GTG AAA CAC TCC AGT CCT AGG AGA GAA ATG GCC      1740
Val Thr Tyr Ala Pro Val Lys His Ser Ser Pro Arg Arg Glu Met Ala
525                 530                 535                 540

TCT CCT CCT TCC CCA CTG TCT GGG GAA TTC CTG GAC ACA AAG GAC AGA      1788
Ser Pro Pro Ser Pro Leu Ser Gly Glu Phe Leu Asp Thr Lys Asp Arg
                545                 550                 555

CAG GCA GAA GAG GAC AGA CAG ATG GAC ACT GAG GCT GCT GCA TCT GAA      1836
Gln Ala Glu Glu Asp Arg Gln Met Asp Thr Glu Ala Ala Ala Ser Glu
            560                 565                 570

GCC TCC CAG GAT GTG ACC TAC GCC CAG CTG CAC AGC TTG ACC CTT AGA      1884
Ala Ser Gln Asp Val Thr Tyr Ala Gln Leu His Ser Leu Thr Leu Arg
        575                 580                 585

CGG AAG GCA ACT GAG CCT CCT CCA TCC CAG GAG TTC GAG TCA GTC AGA      1932
Arg Lys Ala Thr Glu Pro Pro Pro Ser Gln Glu Phe Glu Ser Val Arg
    590                 595                 600

TCA GCA TTG  T GAGGCCCCAT CTCTACAAAA AATAAAACCA GTCCGGCGTG GTGGCACAA      1991
Ser Ala Leu
605

(2) INFORMATION FOR SEQ ID NO:6:

(i) SEQUENCE CHARACTERISTICS:
(A) LENGTH: 623 amino acids
(B) TYPE: amino acid
(D) TOPOLOGY: linear

(ii) MOLECULE TYPE: protein

(xi) SEQUENCE DESCRIPTION: SEQ ID NO:6:

Met Thr Pro Ala Leu Thr Ala Leu Leu Cys Leu Gly Leu Ser Leu Gly
-16 -15                 -10                 -5

Pro Arg Thr Arg Val Gln Ala Gly Pro Phe Pro Lys Pro Thr Leu Trp
  1               5                  10                  15

Ala Glu Pro Gly Ser Val Ile Ser Trp Gly Ser Pro Val Thr Ile Trp
            20                  25                  30

Cys Gln Gly Ser Leu Glu Ala Gln Glu Tyr Gln Leu Asp Lys Glu Gly
        35                  40                  45

Ser Pro Glu Pro Leu Asp Arg Asn Asn Pro Leu Glu Pro Lys Asn Lys
    50                  55                  60

Ala Arg Phe Ser Ile Pro Ser Met Thr Gln His His Ala Gly Arg Tyr
65                  70                  75                  80

Arg Cys His Tyr Tyr Ser Ser Ala Gly Trp Ser Glu Pro Ser Asp Pro
                85                  90                  95

Leu Glu Leu Val Met Thr Gly Ala Tyr Ser Lys Pro Thr Leu Ser Ala
            100                 105                 110

Leu Pro Ser Pro Val Val Ala Ser Gly Gly Asn Met Thr Leu Arg Cys
        115                 120                 125

Gly Ser Gln Lys Arg Tyr His His Phe Val Leu Met Lys Glu Gly Glu
    130                 135                 140

His Gln Leu Pro Arg Thr Leu Asp Ser Gln Gln Leu His Ser Gly Gly
145                 150                 155                 160

Phe Gln Ala Leu Phe Pro Val Gly Pro Val Asn Pro Ser His Arg Trp
                165                 170                 175

Arg Phe Thr Cys Tyr Tyr Tyr Tyr Met Asn Thr Pro Arg Val Trp Ser
            180                 185                 190

His Pro Ser Asp Pro Leu Glu Ile Leu Pro Ser Gly Val Ser Arg Lys
        195                 200                 205

Pro Ser Leu Leu Thr Leu Gln Gly Pro Val Leu Ala Pro Gly Gln Ser
    210                 215                 220

Leu Thr Leu Gln Cys Gly Ser Asp Val Gly Tyr Asp Arg Phe Val Leu
225                 230                 235                 240

Tyr Lys Glu Gly Glu Arg Asp Phe Leu Gln Arg Pro Gly Gln Gln Pro
                245                 250                 255

Gln Ala Gly Leu Ser Gln Ala Asn Phe Thr Leu Gly Pro Val Ser Pro
            260                 265                 270

Ser Asn Gly Gly Gln Tyr Arg Cys Tyr Gly Ala His Asn Leu Ser Ser
        275                 280                 285

Glu Trp Ser Ala Pro Ser Asp Pro Leu Asn Ile Leu Met Ala Gly Gln
    290                 295                 300

Ile Tyr Asp Thr Val Ser Leu Ser Ala Gln Pro Gly Pro Thr Val Ala
305                 310                 315                 320

Ser Gly Glu Asn Val Thr Leu Leu Cys Gln Ser Trp Trp Gln Phe Asp
                325                 330                 335

Thr Phe Leu Leu Thr Lys Glu Gly Ala Ala His Pro Pro Leu Arg Leu

340                 345                 350

Arg Ser Met Tyr Gly Ala His Lys Tyr Gln Ala Glu Phe Pro Met Ser
        355                 360                 365

Pro Val Thr Ser Ala His Ala Gly Thr Tyr Arg Cys Tyr Gly Ser Arg
    370                 375                 380

Ser Ser Asn Pro Tyr Leu Leu Ser His Pro Ser Glu Pro Leu Glu Leu
385                 390                 395                 400

Val Val Ser Gly His Ser Gly Gly Ser Ser Leu Pro Pro Thr Gly Pro
                405                 410                 415

Pro Ser Thr Pro Gly Leu Gly Arg Tyr Leu Glu Val Leu Ile Gly Val
            420                 425                 430

Ser Val Ala Phe Val Leu Leu Leu Phe Leu Leu Leu Phe Leu Leu Leu
        435                 440                 445

Arg Arg Gln Arg His Ser Lys His Arg Thr Ser Asp Gln Arg Lys Thr
    450                 455                 460

Asp Phe Gln Arg Pro Ala Gly Ala Ala Glu Thr Glu Pro Lys Asp Arg
465                 470                 475                 480

Gly Leu Leu Arg Arg Ser Ser Pro Ala Ala Asp Val Gln Glu Glu Asn
                485                 490                 495

Leu Tyr Ala Ala Val Lys Asp Thr Gln Ser Glu Asp Gly Val Glu Leu
            500                 505                 510

Asp Ser Gln Ser Pro His Asp Glu Asp Pro His Ala Val Thr Tyr Ala
        515                 520                 525

Pro Val Lys His Ser Ser Pro Arg Arg Glu Met Ala Ser Pro Pro Ser
    530                 535                 540

Pro Leu Ser Gly Glu Phe Leu Asp Thr Lys Asp Arg Gln Ala Glu Glu
545                 550                 555                 560

Asp Arg Gln Met Asp Thr Glu Ala Ala Ala Ser Glu Ala Ser Gln Asp
                565                 570                 575

Val Thr Tyr Ala Gln Leu His Ser Leu Thr Leu Arg Arg Lys Ala Thr
            580                 585                 590

Glu Pro Pro Pro Ser Gln Glu Phe Glu Ser Val Arg Ser Ala Leu
        595                 600                 605

(2) INFORMATION FOR SEQ ID NO:7:

     (i) SEQUENCE CHARACTERISTICS:
          (A) LENGTH: 1550 base pairs
          (B) TYPE: nucleic acid
          (C) STRANDEDNESS: single
          (D) TOPOLOGY: linear

    (ii) MOLECULE TYPE: DNA (genomic)

(ix) FEATURE:
(A) NAME/KEY: CDS
(B) LOCATION: 22..1438

(ix) FEATURE:
(A) NAME/KEY: sig_peptide
(B) LOCATION: 22..67

(ix) FEATURE:
(A) NAME/KEY: mat_peptide
(B) LOCATION: 70..1438

(xi) SEQUENCE DESCRIPTION: SEQ ID NO:7:

GGAATTCCGG GAGGAGACGC C ATG ATC CCC ACC TTC ACG GCT CTG CTC TGC 51
Met Ile Pro Thr Phe Thr Ala Leu Leu Cys
-16 -15 -10

CTC GGG CTG AGT CTG GGC CCC AGT ACC CAC ATG CAG GCA GGG CCC CTC 99
Leu Gly Leu Ser Leu Gly Pro Ser Thr His Met Gln Ala Gly Pro Leu
-5 1 5 10

CCC AAA CCC ACC CTC TGG GCT GAG CCA GGC TCT GTG ATC AGC TGG GGG 147
Pro Lys Pro Thr Leu Trp Ala Glu Pro Gly Ser Val Ile Ser Trp Gly
15 20 25

AAC TCT GTG ACC ATC TGG TGT CAG GGG ACC CTG GAG GCT CGG GAG TAC 195
Asn Ser Val Thr Ile Trp Cys Gln Gly Thr Leu Glu Ala Arg Glu Tyr
30 35 40

CGT CTG GAT AAA GAG GAA AGC CCA GCA CCC TGG GAC AGA CAG AAC CCA 243
Arg Leu Asp Lys Glu Glu Ser Pro Ala Pro Trp Asp Arg Gln Asn Pro
45 50 55

CTG GAG CCC AAG AAC AAG GCC AGA TTC TCC ATC CCA TCC ATG ACA GAG 291
Leu Glu Pro Lys Asn Lys Ala Arg Phe Ser Ile Pro Ser Met Thr Glu
60 65 70

GAC TAT GCA GGG AGA TAC CGC TGT TAC TAT CGC AGC CCT GTA GGC TGG 339
Asp Tyr Ala Gly Arg Tyr Arg Cys Tyr Tyr Arg Ser Pro Val Gly Trp
75 80 85 90

TCA CAG CCC AGT GAC CCC CTG GAG CTG GTG ATG ACA GGA GCC TAC AGT 387
Ser Gln Pro Ser Asp Pro Leu Glu Leu Val Met Thr Gly Ala Tyr Ser
95 100 105

AAA CCC ACC CTT TCA GCC CTG CCG AGT CCT CTT GTG ACC TCA GGA AAG 435
Lys Pro Thr Leu Ser Ala Leu Pro Ser Pro Leu Val Thr Ser Gly Lys
110 115 120

AGC GTG ACC CTG CTG TGT CAG TCA CGG AGC CCA ATG GAC ACT TTT CTT 483
Ser Val Thr Leu Leu Cys Gln Ser Arg Ser Pro Met Asp Thr Phe Leu
125 130 135

CTG ATC AAG GAG CGG GCA GCC CAT CCC CTA CTG CAT CTG AGA TCA GAG 531
Leu Ile Lys Glu Arg Ala Ala His Pro Leu Leu His Leu Arg Ser Glu
140 145 150

CAC GGA GCT CAG CAG CAC CAG GCT GAA TTC CCC ATG AGT CCT GTG ACC 579
His Gly Ala Gln Gln His Gln Ala Glu Phe Pro Met Ser Pro Val Thr

155                 160                 165                 170

TCA GTG CAC GGG GGG ACC TAC AGG TGC TTC AGC TCA CAC GGC TTC TCC     627
Ser Val His Gly Gly Thr Tyr Arg Cys Phe Ser Ser His Gly Phe Ser
                175                 180                 185

CAC TAC CTG CTG TCA CAC CCC AGT GAC CCC CTG GAG CTC ATA GTC TCA     675
His Tyr Leu Leu Ser His Pro Ser Asp Pro Leu Glu Leu Ile Val Ser
            190                 195                 200

GGA TCC TTG GAG GGT CCC AGG CCC TCA CCC ACA AGG TCC GTC TCA ACA     723
Gly Ser Leu Glu Gly Pro Arg Pro Ser Pro Thr Arg Ser Val Ser Thr
        205                 210                 215

GCT GCA GGC CCT GAG GAC CAG CCC CTC ATG CCT ACA GGG TCA GTC CCC     771
Ala Ala Gly Pro Glu Asp Gln Pro Leu Met Pro Thr Gly Ser Val Pro
    220                 225                 230

CAC AGT GGT CTG AGA AGG CAC TGG GAG GTA CTG ATC GGG GTC TTG GTG     819
His Ser Gly Leu Arg Arg His Trp Glu Val Leu Ile Gly Val Leu Val
235                 240                 245                 250

GTC TCC ATC CTG CTT CTC TCC CTC CTC CTC TTC CTC CTC CTC CAA CAC     867
Val Ser Ile Leu Leu Leu Ser Leu Leu Leu Phe Leu Leu Leu Gln His
                255                 260                 265

TGG CGT CAG GGA AAA CAC AGG ACA TTG GCC CAG AGA CAG GCT GAT TTC     915
Trp Arg Gln Gly Lys His Arg Thr Leu Ala Gln Arg Gln Ala Asp Phe
            270                 275                 280

CAA CGT CCT CCA GGG GCT GCC GAG CCA GAG CCC AAG GAC GGG GGC CTA     963
Gln Arg Pro Pro Gly Ala Ala Glu Pro Glu Pro Lys Asp Gly Gly Leu
        285                 290                 295

CAG AGG AGG TCC AGC CCA GCT GCT GAC GTC CAG GGA GAA AAC TTC TGT     1011
Gln Arg Arg Ser Ser Pro Ala Ala Asp Val Gln Gly Glu Asn Phe Cys
    300                 305                 310

GCT GCC GTG AAG GAC ACA CAG CCT GAG GAC GGG GTG GAA ATG GAC ACT     1059
Ala Ala Val Lys Asp Thr Gln Pro Glu Asp Gly Val Glu Met Asp Thr
315                 320                 325                 330

CGG CAG AGC CCA CAC GAT GAA GAC CCC CAG GCA GTG ACG TAT GCC AAG     1107
Arg Gln Ser Pro His Asp Glu Asp Pro Gln Ala Val Thr Tyr Ala Lys
                335                 340                 345

GTG AAA CAC TCC AGA CCT AGG AGA GAA ATG GCC TCT CCT CCC TCC CCA     1155
Val Lys His Ser Arg Pro Arg Arg Glu Met Ala Ser Pro Pro Ser Pro
            350                 355                 360

CTG TCT GGG GAA TTC CTG GAC ACA AAG GAC AGA CAG GCA GAA GAG GAC     1203
Leu Ser Gly Glu Phe Leu Asp Thr Lys Asp Arg Gln Ala Glu Glu Asp
        365                 370                 375

AGA CAG ATG GAC ACT GAG GCT GCT GCA TCT GAA GCC CCC CAG GAT GTG     1251
Arg Gln Met Asp Thr Glu Ala Ala Ala Ser Glu Ala Pro Gln Asp Val
    380                 385                 390

ACT ACG CCC GGC TGC ACA GCT TTA CCC TCA GAC AGA AGG CAA CTG AGC     1299
Thr Thr Pro Gly Cys Thr Ala Leu Pro Ser Asp Arg Arg Gln Leu Ser
395                 400                 405                 410

CTC CTC CAT CCC AGG AAG GGG CCT CTC CAG CTG AGC CCA GTG TCT ATG 1347
Leu Leu His Pro Arg Lys Gly Pro Leu Gln Leu Ser Pro Val Ser Met
415 420 425

CCA CTC TGG CCA TCC ACT AAT CCA GGG GGG ACC CAG ACC CCA CAA GCC 1395
Pro Leu Trp Pro Ser Thr Asn Pro Gly Gly Thr Gln Thr Pro Gln Ala
430 435 440

ATG GAG ACT CAG GAC CCC AGA AGG CAT GGA AGC TGC CTC CAG T 1438
Met Glu Thr Gln Asp Pro Arg Arg His Gly Ser Cys Leu Gln
445 450 455

AGACATCACT GAACCCCAGC CAGCCCAGAC CCCTGACACA GACCACTAGA AGATTCCGGG 1498

AACGTTGGGA GTCACCTGAT TCTGCAAAGA TAAATAATAT CCCTGCATTA TC 1550

(2) INFORMATION FOR SEQ ID NO:8:

(i) SEQUENCE CHARACTERISTICS:
(A) LENGTH: 472 amino acids
(B) TYPE: amino acid
(D) TOPOLOGY: linear

(ii) MOLECULE TYPE: protein

(xi) SEQUENCE DESCRIPTION: SEQ ID NO:8:

Met Ile Pro Thr Phe Thr Ala Leu Leu Cys Leu Gly Leu Ser Leu Gly
-16 -15 -10 -5

Pro Ser Thr His Met Gln Ala Gly Pro Leu Pro Lys Pro Thr Leu Trp
1               5                   10                  15

Ala Glu Pro Gly Ser Val Ile Ser Trp Gly Asn Ser Val Thr Ile Trp
            20                  25                  30

Cys Gln Gly Thr Leu Glu Ala Arg Glu Tyr Arg Leu Asp Lys Glu Glu
        35                  40                  45

Ser Pro Ala Pro Trp Asp Arg Gln Asn Pro Leu Glu Pro Lys Asn Lys
    50                  55                  60

Ala Arg Phe Ser Ile Pro Ser Met Thr Glu Asp Tyr Ala Gly Arg Tyr
65                  70                  75                  80

Arg Cys Tyr Tyr Arg Ser Pro Val Gly Trp Ser Gln Pro Ser Asp Pro
                85                  90                  95

Leu Glu Leu Val Met Thr Gly Ala Tyr Ser Lys Pro Thr Leu Ser Ala
            100                 105                 110

Leu Pro Ser Pro Leu Val Thr Ser Gly Lys Ser Val Thr Leu Leu Cys
        115                 120                 125

Gln Ser Arg Ser Pro Met Asp Thr Phe Leu Leu Ile Lys Glu Arg Ala
    130                 135                 140

Ala His Pro Leu Leu His Leu Arg Ser Glu His Gly Ala Gln Gln His
145                 150                 155                 160

Gln Ala Glu Phe Pro Met Ser Pro Val Thr Ser Val His Gly Gly Thr
                165                 170                 175

Tyr Arg Cys Phe Ser Ser His Gly Phe Ser His Tyr Leu Leu Ser His
            180                 185                 190

Pro Ser Asp Pro Leu Glu Leu Ile Val Ser Gly Ser Leu Glu Gly Pro
        195                 200                 205

Arg Pro Ser Pro Thr Arg Ser Val Ser Thr Ala Ala Gly Pro Glu Asp
    210                 215                 220

Gln Pro Leu Met Pro Thr Gly Ser Val Pro His Ser Gly Leu Arg Arg
225                 230                 235                 240

His Trp Glu Val Leu Ile Gly Val Leu Val Val Ser Ile Leu Leu Leu
                245                 250                 255

Ser Leu Leu Leu Phe Leu Leu Leu Gln His Trp Arg Gln Gly Lys His
            260                 265                 270

Arg Thr Leu Ala Gln Arg Gln Ala Asp Phe Gln Arg Pro Pro Gly Ala
        275                 280                 285

Ala Glu Pro Glu Pro Lys Asp Gly Gly Leu Gln Arg Arg Ser Ser Pro
    290                 295                 300

Ala Ala Asp Val Gln Gly Glu Asn Phe Cys Ala Ala Val Lys Asp Thr
305                 310                 315                 320

Gln Pro Glu Asp Gly Val Glu Met Asp Thr Arg Gln Ser Pro His Asp
                325                 330                 335

Glu Asp Pro Gln Ala Val Thr Tyr Ala Lys Val Lys His Ser Arg Pro
            340                 345                 350

Arg Arg Glu Met Ala Ser Pro Pro Ser Pro Leu Ser Gly Glu Phe Leu
        355                 360                 365

Asp Thr Lys Asp Arg Gln Ala Glu Glu Asp Arg Gln Met Asp Thr Glu
    370                 375                 380

Ala Ala Ala Ser Glu Ala Pro Gln Asp Val Thr Thr Pro Gly Cys Thr
385                 390                 395                 400

Ala Leu Pro Ser Asp Arg Arg Gln Leu Ser Leu Leu His Pro Arg Lys
                405                 410                 415

Gly Pro Leu Gln Leu Ser Pro Val Ser Met Pro Leu Trp Pro Ser Thr
            420                 425                 430

Asn Pro Gly Gly Thr Gln Thr Pro Gln Ala Met Glu Thr Gln Asp Pro
        435                 440                 445

Arg Arg His Gly Ser Cys Leu Gln
    450                 455

(2) INFORMATION FOR SEQ ID NO:9:

     (i) SEQUENCE CHARACTERISTICS:

(A) LENGTH: 1657 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: CDS
(B) LOCATION: 46..1588

(ix) FEATURE:
(A) NAME/KEY: mat_peptide
(B) LOCATION: 94..1588

(ix) FEATURE:
(A) NAME/KEY: sig_peptide
(B) LOCATION: 46..91

(xi) SEQUENCE DESCRIPTION: SEQ ID NO:9:

GCAGGAATTC GGCACGAGCA GAGCAGGGCA GTGGGAGGAG ACGCC ATG ACC CCC 54
Met Thr Pro
-16 -15

ATC CTC ACG GTC CTG ATC TGT CTC GGG CTG AGT CTG GGC CCC AGG ACC 102
Ile Leu Thr Val Leu Ile Cys Leu Gly Leu Ser Leu Gly Pro Arg Thr
-10 -5 1

CAC GTG CAG GCA GGG CAC CTC CCC AAG CCC ACC CTC TGG GCT GAG CCA 150
His Val Gln Ala Gly His Leu Pro Lys Pro Thr Leu Trp Ala Glu Pro
                5                   10                  15

GGC TCT GTG ATC ATC CAG GGA AGT CCT GTG ACC CTC AGG TGT CAG GGG 198
Gly Ser Val Ile Ile Gln Gly Ser Pro Val Thr Leu Arg Cys Gln Gly
20 25 30 35

AGC CTT CAG GCT GAG GAG TAC CAT CTA TAT AGG GAA AAC AAA TCA GCA 246
Ser Leu Gln Ala Glu Glu Tyr His Leu Tyr Arg Glu Asn Lys Ser Ala
40 45 50

TCC TGG GTT AGA CGG ATA CAA GAG CCT GGG AAG AAT GGC CAG TTC CCC 294
Ser Trp Val Arg Arg Ile Gln Glu Pro Gly Lys Asn Gly Gln Phe Pro
55 60 65

ATC CCA TCC ATC ACC TGG GAA CAC GCA GGG CGG TAT CAC TGT CAG TAC 342
Ile Pro Ser Ile Thr Trp Glu His Ala Gly Arg Tyr His Cys Gln Tyr
70 75 80

TAC AGC CAC AAT CAC TCA TCA GAG TAC AGT GAC CCC CTG GAG CTG GTG 390
Tyr Ser His Asn His Ser Ser Glu Tyr Ser Asp Pro Leu Glu Leu Val
                85                  90                  95

GTG ACA GGA GCC TAC AGC AAA CCC ACC CTC TCA GCT CTG CCC AGC CCT 438
Val Thr Gly Ala Tyr Ser Lys Pro Thr Leu Ser Ala Leu Pro Ser Pro
100 105 110 115

GTG GTG ACC TTA GGA GGG AAC GTG ACC CTC CAG TGT GTC TCA CAG GTG 486
Val Val Thr Leu Gly Gly Asn Val Thr Leu Gln Cys Val Ser Gln Val

120 125 130

GCA TTT GAC GGC TTC ATT CTG TGT AAG GAA GGA GAA GAT GAA CAC CCA 534
Ala Phe Asp Gly Phe Ile Leu Cys Lys Glu Gly Glu Asp Glu His Pro
135 140 145

CAA CGC CTG AAC TCC CAT TCC CAT GCC CGT GGG TGG TCC TGG GCC ATC 582
Gln Arg Leu Asn Ser His Ser His Ala Arg Gly Trp Ser Trp Ala Ile
150 155 160

TTC TCC GTG GGC CCC GTG AGC CCG AGT CGC AGG TGG TCG TAC AGG TGC 630
Phe Ser Val Gly Pro Val Ser Pro Ser Arg Arg Trp Ser Tyr Arg Cys
                165                 170                 175

TAT GCT TAT GAC TCG AAC TCT CCC TAT GTG TGG TCT CTA CCC AGT GAT 678
Tyr Ala Tyr Asp Ser Asn Ser Pro Tyr Val Trp Ser Leu Pro Ser Asp
180 185 190 195

CTC CTG GAG CTC CTG GTC CCA GGT GTT TCT AAG AAG CCA TCA CTC TCA 726
Leu Leu Glu Leu Leu Val Pro Gly Val Ser Lys Lys Pro Ser Leu Ser
200 205 210

GTG CAG CCA GGT CCT ATG GTG GCC CCT GGG GAG AGC CTG ACC CTC CAG 774
Val Gln Pro Gly Pro Met Val Ala Pro Gly Glu Ser Leu Thr Leu Gln
215 220 225

TGT GTC TCT GAT GTC GGC TAC GAC AGA TTT GTT CTG TAT AAG GAG GGA 822
Cys Val Ser Asp Val Gly Tyr Asp Arg Phe Val Leu Tyr Lys Glu Gly
230 235 240

GAA CGT GAC TTC CTC CAG CGC CCT GGT TGG CAG CCC CAG GCT GGG CTC 870
Glu Arg Asp Phe Leu Gln Arg Pro Gly Trp Gln Pro Gln Ala Gly Leu
                245                 250                 255

TCC CAG GCC AAC TTC ACC CTG GGC CCT GTG AGC CCC TCC CAC GGG GGC 918
Ser Gln Ala Asn Phe Thr Leu Gly Pro Val Ser Pro Ser His Gly Gly
260 265 270 275

CAG TAC AGA TGC TAC AGT GCA CAC AAC CTC TCC TCC GAG TGG TCG GCC 966
Gln Tyr Arg Cys Tyr Ser Ala His Asn Leu Ser Ser Glu Trp Ser Ala
280 285 290

CCC AGT GAC CCC CTG GAC ATC CTG ATC ACA GGA CAG TTC TAT GAC AGA 1014
Pro Ser Asp Pro Leu Asp Ile Leu Ile Thr Gly Gln Phe Tyr Asp Arg
295 300 305

CCC TCT CTC TCG GTG CAG CCG GTC CCC ACA GTA GCC CCA GGA AAG AAC 1062
Pro Ser Leu Ser Val Gln Pro Val Pro Thr Val Ala Pro Gly Lys Asn
310 315 320

GTG ACC CTG CTG TGT CAG TCA CGG GGG CAG TTC CAC ACT TTC CTT CTG 1110
Val Thr Leu Leu Cys Gln Ser Arg Gly Gln Phe His Thr Phe Leu Leu
                325                 330                 335

ACC AAG GAG GGG GCA GGC CAT CCC CCA CTG CAT CTG AGA TCA GAG CAC 1158
Thr Lys Glu Gly Ala Gly His Pro Pro Leu His Leu Arg Ser Glu His
340 345 350 355

CAA GCT CAG CAG AAC CAG GCT GAA TTC CGC ATG GGT CCT GTG ACC TCA 1206
Gln Ala Gln Gln Asn Gln Ala Glu Phe Arg Met Gly Pro Val Thr Ser
360 365 370

GCC CAC GTG GGG ACC TAC AGA TGC TAC AGC TCA CTC AGC TCC AAC CCC 1254
Ala His Val Gly Thr Tyr Arg Cys Tyr Ser Ser Leu Ser Ser Asn Pro
375 380 385

TAC CTG CTG TCT CTC CCC AGT GAC CCC CTG GAG CTC GTG GTC TCA GCA 1302
Tyr Leu Leu Ser Leu Pro Ser Asp Pro Leu Glu Leu Val Val Ser Ala
390 395 400

TCC CTA GGC CAA CAC CCC CAG GAT TAC ACA GTG GAG AAT CTC ATC CGC 1350
Ser Leu Gly Gln His Pro Gln Asp Tyr Thr Val Glu Asn Leu Ile Arg
                405                 410                 415

ATG GGT GTG GCT GGC TTG GTC CTG GTG GTC CTC GGG ATT CTG CTA TTT 1398
Met Gly Val Ala Gly Leu Val Leu Val Val Leu Gly Ile Leu Leu Phe
420 425 430 435

GAG GCT CAG CAC AGC CAG AGA AGC CTA CAA GAT GCA GCC GGG AGT GAA 1446
Glu Ala Gln His Ser Gln Arg Ser Leu Gln Asp Ala Ala Gly Ser Glu
440 445 450

CAG CAG AGA GGA CAA TGC ATC CTT CAG CGT GGT GGA GCC TCA GGG ACA 1494
Gln Gln Arg Gly Gln Cys Ile Leu Gln Arg Gly Gly Ala Ser Gly Thr
455 460 465

GAT CTG ATG ATC CCA GGA GGC TCT GGA GGA CAA TCT AGG ACC TAC ATT 1542
Asp Leu Met Ile Pro Gly Gly Ser Gly Gly Gln Ser Arg Thr Tyr Ile
470 475 480

ATC TGG ACT GTA TGC TGG TCA TTT CTA GAG ACA GCA ATC AAT ATT T 1588
Ile Trp Thr Val Cys Trp Ser Phe Leu Glu Thr Ala Ile Asn Ile
                485                 490                 495

GAGTGTAAGG AAACTGTCTG GGGTGATTCC TAGAAGATCA TTAAACTGTG GTACATTTTT 1648

TTGTCTATG 1657

(2) INFORMATION FOR SEQ ID NO:10:

(i) SEQUENCE CHARACTERISTICS:
(A) LENGTH: 514 amino acids
(B) TYPE: amino acid
(D) TOPOLOGY: linear

(ii) MOLECULE TYPE: protein

(xi) SEQUENCE DESCRIPTION: SEQ ID NO:10:

Met Thr Pro Ile Leu Thr Val Leu Ile Cys Leu Gly Leu Ser Leu Gly
-16 -15 -10 -5

Pro Arg Thr His Val Gln Ala Gly His Leu Pro Lys Pro Thr Leu Trp
1               5                   10                  15

Ala Glu Pro Gly Ser Val Ile Ile Gln Gly Ser Pro Val Thr Leu Arg
            20                  25                  30

Cys Gln Gly Ser Leu Gln Ala Glu Glu Tyr His Leu Tyr Arg Glu Asn
        35                  40                  45

Lys Ser Ala Ser Trp Val Arg Arg Ile Gln Glu Pro Gly Lys Asn Gly
    50                  55                  60

Gln Phe Pro Ile Pro Ser Ile Thr Trp Glu His Ala Gly Arg Tyr His
65                  70                  75                  80

Cys Gln Tyr Tyr Ser His Asn His Ser Ser Glu Tyr Ser Asp Pro Leu
                85                  90                  95

Glu Leu Val Val Thr Gly Ala Tyr Ser Lys Pro Thr Leu Ser Ala Leu
            100                 105                 110

Pro Ser Pro Val Val Thr Leu Gly Gly Asn Val Thr Leu Gln Cys Val
        115                 120                 125

Ser Gln Val Ala Phe Asp Gly Phe Ile Leu Cys Lys Glu Gly Glu Asp
    130                 135                 140

Glu His Pro Gln Arg Leu Asn Ser His Ser His Ala Arg Gly Trp Ser
145                 150                 155                 160

Trp Ala Ile Phe Ser Val Gly Pro Val Ser Pro Ser Arg Arg Trp Ser
                165                 170                 175

Tyr Arg Cys Tyr Ala Tyr Asp Ser Asn Ser Pro Tyr Val Trp Ser Leu
            180                 185                 190

Pro Ser Asp Leu Leu Glu Leu Leu Val Pro Gly Val Ser Lys Lys Pro
        195                 200                 205

Ser Leu Ser Val Gln Pro Gly Pro Met Val Ala Pro Gly Glu Ser Leu
    210                 215                 220

Thr Leu Gln Cys Val Ser Asp Val Gly Tyr Asp Arg Phe Val Leu Tyr
225                 230                 235                 240

Lys Glu Gly Glu Arg Asp Phe Leu Gln Arg Pro Gly Trp Gln Pro Gln
                245                 250                 255

Ala Gly Leu Ser Gln Ala Asn Phe Thr Leu Gly Pro Val Ser Pro Ser
            260                 265                 270

His Gly Gly Gln Tyr Arg Cys Tyr Ser Ala His Asn Leu Ser Ser Glu
        275                 280                 285

Trp Ser Ala Pro Ser Asp Pro Leu Asp Ile Leu Ile Thr Gly Gln Phe
    290                 295                 300

Tyr Asp Arg Pro Ser Leu Ser Val Gln Pro Val Pro Thr Val Ala Pro
305                 310                 315                 320

Gly Lys Asn Val Thr Leu Leu Cys Gln Ser Arg Gly Gln Phe His Thr
                325                 330                 335

Phe Leu Leu Thr Lys Glu Gly Ala Gly His Pro Pro Leu His Leu Arg
            340                 345                 350

Ser Glu His Gln Ala Gln Gln Asn Gln Ala Glu Phe Arg Met Gly Pro
        355                 360                 365

Val Thr Ser Ala His Val Gly Thr Tyr Arg Cys Tyr Ser Ser Leu Ser

370                 375                 380

Ser Asn Pro Tyr Leu Leu Ser Leu Pro Ser Asp Pro Leu Glu Leu Val
385                 390                 395                 400

Val Ser Ala Ser Leu Gly Gln His Pro Gln Asp Tyr Thr Val Glu Asn
                405                 410                 415

Leu Ile Arg Met Gly Val Ala Gly Leu Val Leu Val Val Leu Gly Ile
            420                 425                 430

Leu Leu Phe Glu Ala Gln His Ser Gln Arg Ser Leu Gln Asp Ala Ala
        435                 440                 445

Gly Ser Glu Gln Gln Arg Gly Gln Cys Ile Leu Gln Arg Gly Gly Ala
    450                 455                 460

Ser Gly Thr Asp Leu Met Ile Pro Gly Gly Ser Gly Gly Gln Ser Arg
465                 470                 475                 480

Thr Tyr Ile Ile Trp Thr Val Cys Trp Ser Phe Leu Glu Thr Ala Ile
                485                 490                 495

Asn Ile

(2) INFORMATION FOR SEQ ID NO:11:

    (i) SEQUENCE CHARACTERISTICS:
        (A) LENGTH: 264 amino acids
        (B) TYPE: amino acid
        (C) STRANDEDNESS: single
        (D) TOPOLOGY: linear

    (ii) MOLECULE TYPE: protein

    (xi) SEQUENCE DESCRIPTION: SEQ ID NO:11:

Met Ala Pro Thr Leu Pro Ala Leu Leu Cys Leu Gly Leu Ser Val Gly
1               5                   10                  15

Leu Arg Thr Gln Val Gln Ala Gly Thr Phe Pro Lys Pro Ile Ile Trp
            20                  25                  30

Ala Glu Pro Ser Ser Val Val Pro Leu Gly Ser Ser Val Thr Ile Leu
        35                  40                  45

Cys Gln Gly Pro Pro Asn Thr Lys Ser Phe Ser Leu Asn Lys Glu Gly
    50                  55                  60

Asp Ser Thr Pro Trp Asn Ile His Pro Ser Leu Glu Pro Trp Asp Lys
65                  70                  75                  80

Ala Asn Phe Phe Ile Ser Asn Val Arg Glu Gln Gln Ala Gly Arg Tyr
                85                  90                  95

His Cys Ser His Phe Ile Gly Val Asn Trp Ser Glu Pro Ser Glu Pro
            100                 105                 110

Leu Asp Leu Leu Val Ala Gly Glu Glu Pro Ala Gly Arg Leu Arg Asp
        115                 120                 125

Arg Pro Ser Leu Ser Val Arg Pro Ser Pro Ser Val Ala Pro Gly Glu
    130                 135                 140

Asn Val Thr Leu Leu Cys Gln Ser Gly Asn Arg Thr Asp Thr Phe Leu
145                 150                 155                 160

Leu Ser Lys Glu Gly Ala Ala His Arg Pro Leu Arg Leu Arg Ser Gln
                165                 170                 175

Asp Gln Asp Gly Trp Tyr Gln Ala Glu Phe Ser Leu Ser Pro Val Thr
            180                 185                 190

Ser Ala His Gly Gly Thr Tyr Arg Cys Tyr Arg Ser Leu Ser Thr Asn
        195                 200                 205

Pro Tyr Leu Leu Ser Gln Pro Ser Glu Pro Leu Ala Leu Leu Val Ala
    210                 215                 220

Asp Tyr Thr Met Gln Asn Leu Ile Arg Met Gly Leu Ala Ala Ser Val
225                 230                 235                 240

Leu Leu Leu Leu Gly Ile Leu Leu Cys Gln Ala Arg His Asp His Gly
                245                 250                 255

Gly Ala Arg Glu Ala Ala Arg Ser
            260

(2) INFORMATION FOR SEQ ID NO:12:

(i) SEQUENCE CHARACTERISTICS:
(A) LENGTH: 186 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:12:

GGTCACGAGC CTNTGTCCTG GCCAGNCACC GAGGGNTCAT CCATCCACAG AGCAGTGCAG 60

TGGGAGGAGC CGNGATGACC CCCATCCTCA AGGTCCTGAT CTGTCTCGGG CCCCTCCCCA 120

AGCCNACCCT CTGGGCTGAG CCAGGCTCTG TGATCANCNA AGGGGAGTCC TGTANCCCTN 180

AGGTGT 186

(2) INFORMATION FOR SEQ ID NO:13:

(i) SEQUENCE CHARACTERISTICS:
(A) LENGTH: 270 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:13:

GGCAGNAGGG NCCCCTCCCC AAGCCCACCC TCTGGGACTG AGCCAGGCTC TGTAATCACC 60

CAAGGNAGTC CTGTAACCCT NAGGTGTNAG GGGAGCCTGG AGACGCAGGA GTACCATCTA 120

TATAGAGAAA AGAAAACAGC ACTCTGGATT ACACGGATCC CACAGGAGCT TGTAAAGAAG 180

GGCCAGTTCC CCATCCTATC CATCACCTGG GAACATGCAG GGCGGTATTC TGTATCTTGG 240

NAGCCACATT NAAGNCCTNT AGGGGCAGTN 270

(2) INFORMATION FOR SEQ ID NO:14:

(i) SEQUENCE CHARACTERISTICS:
(A) LENGTH: 300 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:14:

GGTCCTNAAT CTGTNTCGAA GCCTACAGCA AACCCACCCT CTAAGCTCTG CCCAGCCCTG 60

TGGTGACCTT AGGAGGGAAC GTGAACCCTC CAGTGTGTCT AAAAGGTGGN ATTTGAANGG 120

CTTCATTCTG TGTAAGGTAA GGAGAAGATG AACACCCACA ACGCCTGANC TCCCATTCCN 180

ATGCCCGTGG GTGGTCCTGG GCCATCTTCT CCGTGGGCCC CGTGAGCCCG AGTGGCAGTG 240

GGTCGTACAG GTGCTATGNT TGNTAATTNG GAANTGTGCC CTATGANNTG GGTGTTNTAA 300

(2) INFORMATION FOR SEQ ID NO:15:

(i) SEQUENCE CHARACTERISTICS:
(A) LENGTH: 253 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:15:

GGCACGAGCC TACAGGTGCT ACGGCTCACT CAGCTCCAAC CCCTACCTGC TGACTCACCC 60

CAGTNACCCC CTGGAGCTCG TGGTCTCAGG AGCAGCTGAG ACCCTCAGCC CACCACAAAA 120

CAAGTCCGAC TCCAAGGCTG GTGAGTGAGG AGATGCTTGC CGTGATGACG CTGGGCACAG 180

AGGGTCAGGT CCTNTCAAGA GGAGCTGGGT GTCCTNGGTG GACATTTNAA GAATTATATT 240

NATTGCANCT TGA 253

(2) INFORMATION FOR SEQ ID NO:16:

(i) SEQUENCE CHARACTERISTICS:
(A) LENGTH: 197 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:16:

TAATTCGGCA GAGCGGGCGG GGACAATAAG GGAATAAAAG CTGGCGAGCN CGGCACCCCA 60

CCAGAGTCTG CTTCCACGTT GTGGNAGCTT TGTTCTCTTG CTCTACACAA TAAATCTTGC 120

TGCTGCTAAA AAAAAAAAAA AAAAAAAAAA AAAAAATTTN GGGGGTCCNA AAAAAAAAAG 180

AAAGGGAAAG GNTTTTT 197

(2) INFORMATION FOR SEQ ID NO:17:

(i) SEQUENCE CHARACTERISTICS:
(A) LENGTH: 495 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:17:

AATTCGGCAG AGAGTNGTTC GGCCCCCAGC GACCCCCTGA ACATCCTGAT GGCAGGACAG 60

ATCTATGACA CCGTCTCCCT GTCAGCACAG CCGGGCCCCA CAGTGGCCTC AGGAGAGAAC 120

GTGAACCCTG NCTGTGTCAG TNCATGGTGG TCAGTTTGAC ACTTTCCTTC TGACCAAAGA 180

AGGGGCAGCC CATCCCCCAC TGCGTCTGAG ATCAATGTAC GGAGCTCATA AGTACCAGGC 240

TGAAATTCCC CATGAGTCCT GTGAACCTCA GCCCACGCGG GGNACCTACA GGTGCTAACG 300

GNTCACGNAG TTCCAACCCC CACCTGNTGT NTTCACCCCA GTNGGCNCCC TGGGAGCTCG 360

TTGGTTTTCA GGACAATTTT GGGGGNTTNC ANNTTCCNAN GCCAAAAGGN CNGTCTTTCA 420

NAANTGGTTT TGGGNAGATA ACTTGGNGGT TTTNAATTGG GGTTTTGGTT GGGCTTTGGG 480

CNTGTTGTTT TGCCT 495

(2) INFORMATION FOR SEQ ID NO:18:

(i) SEQUENCE CHARACTERISTICS:
(A) LENGTH: 397 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:18:

AATTCGGCAG AGNCAGCACT GAGGGCTCAT CCCTCTGCAG AGCGCGGGGT CACCGGTAAG 60

GAGACGCCAT GACGCCCGCC CTCACAGCCC TGCTCTGCCT TGGGCTGAGT CTGGGCCCCA 120

GGAACCCGCG TGGCAGGCAG GGCCCTTCCC CAAACCCACC CTCTGGGGCT GAGCCAGGCT 180

CTGTGAATCA GCTGGGGGGA GCCCCGTGAA CCATCTGGTG TCAGGGGGAG CCTGGAGGNC 240

CAGGAGTACC AACTGGATAA AGAAGGGAAG CCCAGAGCCC TTGGGACAGA AATAACCCAC 300

TGGGAACCCA AGGAACAAGG GCCAGATTTT TGCATNNCCA TTNCATGGAT ACAGNAACCT 360

TGNNAGGGGA GGATTACCGG TTGGNCAATT ATTAACA 397

(2) INFORMATION FOR SEQ ID NO:19:

(i) SEQUENCE CHARACTERISTICS:
(A) LENGTH: 445 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:19:

AATTCGGCAN AGCCACAAGG TCCGTNTCAA CAGCTGGCCC TGAGGACCAG CCCCTTATGC 60

CTACAGGGTC AGTCCCNCAC AGTGGTCTGA GAAGGCACTG NGAGGTACTG ATCGGGGTCT 120

TGGTGGTCTN CATCCTGCTT CTTTCCCTCC TCCTCTTCCT CCTCCTCCAA CACTGGCGTC 180

AGGGAAAACA CAGGACATTG GCCCAGAGAC AGGCTGATTT CCAACGTCCT CCAGGGGCTG 240

CCGAGCCAGA GCCCAAGGAC GGGGGCCTAC AGAGGAGGTN CAGCCCAGTT CTTGACGTTC 300

CAGGGAGAAA AATTTTTTGN TTNCGTNAAG GACAAAAAAG CTTNGGGACG GGGTTGGAAT 360

TGNCAATTGG GANNGCCCAN AAGTTTAAGA NCCCCNGGNA TTTANGNTTT NCAAAGTNTA 420

AAAATTTCAN ACTTTGGGGG GAATT 445

(2) INFORMATION FOR SEQ ID NO:20:

(i) SEQUENCE CHARACTERISTICS:
(A) LENGTH: 260 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:20:

AATTCGGCAC GAGGTACTGG GAGGTACTGA TCGGGGTCTT GGTGGTCTCC ANCCTGCTTC 60

TNTCCNTCCT CCTCTTCCTC CTCCTCCAAC ACTGGCGTCA GGGAAAACAC AGGACATTGG 120

CCCAGAGACA GGNTGATTTN CAACGTCCTC CAGGGGCTGC CGAGCCAGAG CCCAAGGACG 180

GGGGCCTACA GAGGAGGTCC AGCCCAGNTG CTGACGTNCA GGGAGAAAAC TTNTGTGCTN 240

CCGTGAAGAA CACACAGNCT 260

(2) INFORMATION FOR SEQ ID NO:21:

(i) SEQUENCE CHARACTERISTICS:
(A) LENGTH: 255 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:21:

CGGAAAGCCC AGCACCCTTG ANNAGANAGA ACCCACTGGA GCCCAAGAAC AAGGCCAGAT 60

TCTCGATCCC ATCCATGGAC AGAGGACTAT GCAGGGAGAT ACCGCTGTTA ACTATCGCAG 120

CCCTGTAGGC TGGGTGCACA CCNAGGTGAA CCCCCTGNTG NCTGGGAGAT GGTCAGGAGC 180

CTAGAGTAAA CCCACCCTTT AAGGCCCTGC CGAGTCCTCT TGTANCCNCA GTGAAAGAGC 240

GTGACCCTGC TGTNT 255

(2) INFORMATION FOR SEQ ID NO:22:

(i) SEQUENCE CHARACTERISTICS:
(A) LENGTH: 497 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:22:

AATTCCGGCC TCTCCTCCCT CCCCACTGTC TGGGGAATTC CTGGACACAA AGGACAGACA 60

GGCAGAAGAG GACAGACAGA TGGACACTGA GGCTGCTGCA TCTAAAGCCC CCCAGGATGT 120

GAACCTACGC CCAGCTGCAC AGTTTACCCT CAGACAGAAG GCAACTGAGC CTCCTCCATC 180

CCAGGAAGGG GCCTCTNCAG CTGAGCCCAG TGTCTATGCC ACTCTGGCCA TCCACTTAAT 240

CCAGGGGGGA CCCAGACCCC ACAAGCCATG GAGATTCAGG ACCNNAGAAG GCATGGAAGN 300

TGCCTTCCAG TAGACATCAN TGAACCCCAG NCAGNCCAGA ACCTTNANAA AGACCATTAG 360

AAGTTTTNGG GGAAGTTTGG GGGTGAATTG NTTTTTGGAA AGGTTAAATA ATTNTNNCTG 420

GNATTTTTNA AATTAAAGTT GGGAGACTTT TTAATTTNNA ATGGGGTTTA TTGNTTNAAA 480

AAANNNGNNN NGTNGNN 497

(2) INFORMATION FOR SEQ ID NO:23:

(i) SEQUENCE CHARACTERISTICS:
(A) LENGTH: 512 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:23:

AATTCGGCAC GACCTTTCCT CCTCCTCTCC CTCCTCCTCC TCCGTCAGCN TCACAGCAAA 60

CACAGGACAT CTGACCAGAG AAAGACTGAT TTCCAGCGTC CTGCAGGGGC TGCGGAGACA 120

GAGCCCAAGG ACAGGGGCCT GCTGAGGAGG TCCAGCCCAG CTGCTGACGT CCAGGAAGAA 180

AACCTCTGTA AGAGGAAGAG AGGGGACAAA TGGGGGTGCT GGAGAGACAG GAGTCCCAAA 240

ATTTCAGTAG CAACAGGGAG GGGCTGGGAA GGGTCTGGGG CTCCGTGGAA GATGGTCTTN 300

CCCCACACTG TNGGGACCTC CCTGCATTCG GTGGCCCCTT TGGGAGCAGG GCAGGGGGCC 360

AGCAGGATTN AGAGGTTTTA GAGAACCAGG NGANGANCCC TTTGTTTTGN CCCAGNAGTT 420

GTTGTTTTTN AGGGACAAAA ATTTTTNGGN NAGGTTGGAG TTTGNNNATT NAGAGCCCNA 480

AGGTTNNGGN CCCCNGGNAT TNNGTTTTCC CC 512

(2) INFORMATION FOR SEQ ID NO:24:

(i) SEQUENCE CHARACTERISTICS:
(A) LENGTH: 300 base pairs

(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:24:

GGTCCTNAAT CTGTNTCGAA GCCTACAGCA AACCCACCCT CTAAGCTCTG CCCAGCCCTG 60

TGGTGACCTT AGGAGGGAAC GTGAACCCTC CAGTGTGTCT AAAAGGTGGN ATTTGAANGG 120

CTTCATTCTG TGTAAGGTAA GGAGAAGATG AACACCCACA ACGCCTGANC TCCCATTCCN 180

ATGCCCGTGG GTGGTCCTGG GCCATCTTCT CCGTGGGCCC CGTGAGCCCG AGTGGCAGTG 240

GGTCGTACAG GTGCTATGNT TGNTAATTNG GAANTGTGCC CTATGANNTG GGTGTTNTAA 300

(2) INFORMATION FOR SEQ ID NO:25:

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:25:

GACTCATGAC TGAGCCAGGC TCTGTGATCA CCC 33

(2) INFORMATION FOR SEQ ID NO:26:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:26:

GACAGATCTC TCACCAGCCT TGGAGTC 27

(2) INFORMATION FOR SEQ ID NO:27:

(i) SEQUENCE CHARACTERISTICS:
(A) LENGTH: 31 base pairs
(B) TYPE: nucleic acid

(C) STRANDEDNESS: single
(D) TOPOLOGY: linear

(ii) MOLECULE TYPE: DNA (genomic)

(xi) SEQUENCE DESCRIPTION: SEQ ID NO:27:

GACAGATCTG AGCCAGGCTC TGTGATCACC C 31

(2) INFORMATION FOR SEQ ID NO:28:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:28:

GACTCTAGAG TCCACCCAGG ACACCCAGC 29

(2) INFORMATION FOR SEQ ID NO:29:

(i) SEQUENCE CHARACTERISTICS:
(A) LENGTH: 136 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:29:

CTAGCCAGAT CTGCCACCAT GAACTCCTTC TCCACAAGCG CCTTCGGTCC AGTTGCCTTC 60

TCCCTGGGGC TGCTCCTGGT GTTGCCTGCT GCCTTCCCTG CCCCAGTTGT GAGAGAGCCA 120

GGCTCTGTGA TCACCC 136

(2) INFORMATION FOR SEQ ID NO:30:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:30:

CAGCTCGAGC TCACCAGCCT TGGAGTC 27

(2) INFORMATION FOR SEQ ID NO:31:

(i) SEQUENCE CHARACTERISTICS:
(A) LENGTH: 136 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:31:

CTAGCCGGAT CCGCCACCAT GAACTCCTTC TCCACAAGCG CCTTCGGTCC AGTTGCCTTC 60

TCCCTGGGGC TGCTCCTGGT GTTGCCTGCT GCCTTCCCTG CCCCAGTTGT GAGAGAGCCA 120

GGCTCTGTGA TCACCC 136

(2) INFORMATION FOR SEQ ID NO:32:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:32:

GCTCTAGAGT CCACCCAGGA CACCCAGC 28